# EUROPEAN PATENT APPLICATION

(11) **EP 3 444 347 A1**
(43) Date of publication of application: **20.02.2019**
(21) Application number: 17186885.4
(22) Date of filing: 18.08.2017
(51) Int. Cl.: C12N 15/113, C12N 15/63, C12N 9/22, C07K 14/01

(54) **USE OF ANTI-CRISPR POLYPEPTIDES FOR SPECIFIC ACTIVATION OF CAS NUCLEASES**

(71) Applicant: Universität Heidelberg, 69117 Heidelberg (DE); Deutsches Krebsforschungszentrum, 69120 Heidelberg (DE)
(72) Inventor: NIOPEK, Dominik, 69121 Heidelberg (DE); EILS, Roland, 69198 Schriesheim (DE); GRIMM, Dirk, 69117 Heidelberg (DE); HOFFMANN, Mareike Daniela, 69221 Dossenheim (DE); DOMENGER, Claire, 69214 Eppelheim (DE); FAKHIRI, Julia, 69120 Heidelberg (DE); ASCHENBRENNER, Sabine, 64646 Heppenheim (DE); SCHMELAS, Carolin, 69221 Dossenheim (DE)
(74) Representative: Herzog, Fiesser & Partner Patentanwälte PartG mbB

(57) **Abstract**

The present invention relates to a polynucleotide comprising (i) a sequence encoding an anti-CRISPR (Acr) polypeptide and (ii) an miRNA target sequence and to vectors and host cells related thereto. The present invention further relates to a kit comprising (I) a first polynucleotide comprising (i) a sequence encoding an anti-CRISPR (Acr) polypeptide and (ii) an RNA-interference (RNAi) target sequence; and a Cas nuclease or a second polynucleotide comprising an expressible gene encoding a Cas nuclease; (II) a first polynucleotide comprising (i) a sequence encoding an anti-CRISPR (Acr) polypeptide and (ii) an RNA-interference (RNAi) target sequence, said first polynucleotide further comprising an expressible gene encoding a first fragment of a Cas nuclease, and a second polynucleotide comprising an expressible gene encoding a second fragment of a Cas nuclease, wherein said first and second fragment of said Cas nuclease together reconstitute an active Cas nuclease; or (III) a first polynucleotide comprising (i) a sequence encoding an anti-CRISPR (Acr) polypeptide, (ii) an RNA-interference (RNAi) target sequence, and (iii) a sequence encoding a Cas nuclease; and a second polynucleotide comprising an expressible gene encoding an inhibitory RNA, preferably a siRNA or a miRNA hybridizing to said RNAi target sequence, more preferably a siRNA hybridizing to said RNAi target sequence. Further, the present invention relates to methods and uses related to the aforesaid means.

## Description

The present invention relates to a polynucleotide comprising (i) a sequence encoding an anti-CRISPR (Acr) polypeptide and (ii) an miRNA target sequence and to vectors and host cells related thereto. The present invention further relates to a kit comprising (I) a first polynucleotide comprising (i) a sequence encoding an anti-CRISPR (Acr) polypeptide and (ii) an RNA-interference (RNAi) target sequence; and a Cas nuclease or a second polynucleotide comprising an expressible gene encoding a Cas nuclease; (II) a first polynucleotide comprising (i) a sequence encoding an anti-CRISPR (Acr) polypeptide and (ii) an RNA-interference (RNAi) target sequence, said first polynucleotide further comprising an expressible gene encoding a first fragment of a Cas nuclease, and a second polynucleotide comprising an expressible gene encoding a second fragment of a Cas nuclease, wherein said first and second fragment of said Cas nuclease together reconstitute an active Cas nuclease; or (III) a first polynucleotide comprising (i) a sequence encoding an anti-CRISPR (Acr) polypeptide, (ii) an RNA-interference (RNAi) target sequence, and (iii) a sequence encoding a Cas nuclease; and a second polynucleotide comprising an expressible gene encoding an inhibitory RNA, preferably a siRNA or a miRNA hybridizing to said RNAi target sequence, more preferably a siRNA hybridizing to said RNAi target sequence. Further, the present invention relates to methods and uses related to the aforesaid means.

CRISPR (Clustered, Regularly Interspaced Short Palindromic Repeats) systems in bacteria and archaea mediate specific degradation of foreign, invading nucleic acids (Barrangou et al., 2007; Bhaya et al., 2011; Terns and Terns, 2011; Wiedenheft et al., 2012). They comprise a CRISPR-associated (Cas) nuclease which can be programmed by short guideRNAs (gRNAs) to induce double-strand breaks at specific, sequence-complementary DNA loci (Jinek et al., 2012). Diverse CRISPR-Cas systems have been adopted for genome engineering in mammalian cells and animals, most prominently the CRISPR-Cas9 system from Streptococcus pyogenes (SpyCas9) (Cong et al., 2013; Jinek et al., 2013; Mali et al., 2013). CRISPR-Cas9 systems have also been successfully applied for genome editing in embryonic stem cells (Wang et al., 2013) as well as in animals. For instance, transgenic mice were reported that stably express SpyCas9 and thus enable in vivo gene knockout screens (Platt et al., 2014). Alternatively, transient and efficient in vivo delivery of the Cas protein and gRNA components via e.g. hydrodynamic plasmid DNA injection (Yin et al., 2014) or Adeno-associated viral (AAV) vectors (Senis et al., 2014; Ran et al., 2015) has also been achieved. Not surprisingly, the potential of CRISPR-Cas-based human gene therapy is considered to be enormous and motivates an ever increasing number of preclinical studies for treatment of genetic diseases (Schmidt and Grimm, 2015; Dai et al., 2016; Xue et al., 2016).

However, a number of challenges still hamper the straightforward application of CRISPR-Cas systems for basic or applied research especially in multi-cellular systems. One is the insufficient specificity of the Cas nuclease and hence the risk of unintended editing of non-target loci (Fu et al., 2013; Hsu et al., 2013; Cho et al., 2014; Wang et al., 2015; Zhang et al., 2015). This issue was attempted to be resolved by employing engineered Cas variants with an improved on-target specificity (Kleinstiver et al., 2016; Slaymaker et al., 2016) or by timely activating/de-activating the Cas nuclease. The latter can be achieved either by using Cas variants permitting control with exogenous triggers such as chemicals (Zetsche et al., 2015b; Maji et al., 2017) or light (Hemphill et al., 2015; Nihongaki et al., 2015a; Nihongaki et al., 2015b; Polstein and Gersbach, 2015), or by using synthetic gene circuits controlling CRISPR-Cas activation (Kiani et al., 2014; Petris et al., 2017)

A second, major hurdle is the need for highly specific delivery of CRISPR-Cas into and/or its activation (solely) within the desired target tissue or cell, ideally even after systemic administration of the CRISPR-Cas components. Target tissues of (therapeutic) interest could be e.g. the skeletal muscles in case of Duchenne muscular dystrophy treatment, T-helper cells in the case of HIV therapy, the brain in case of Huntington's or Alzheimer's disease, the liver, the pancreas, a tumor or any other possible organ/tissue within the human body. An important consideration for tissue-specific CRISPR-Cas activation is the mode of delivery. AAV vectors are often considered as the prime choice for delivery of CRISPR-Cas into cells, animals or patients (Senis et al., 2014; Schmidt and Grimm, 2015). These are small, single-stranded and non-pathogenic DNA viruses belonging to the family of Parvoviridae. Besides their potency and safety for ex or in vivo gene delivery, AAVs are beneficial over other (viral) delivery systems due to the possibility to modulate tissue targeting as well as transduction efficacy after systemic vector administration in animals or humans. This can be achieved by "pseudotyping" AAV vectors, i.e. packaging the transgene-carrying genome into different natural or engineered AAV capsids (reviewed in Kay, 2011).

Still, targeting specificity remains a major concern for most CRISPR applications, as none of the existing delivery systems, including pseudotyped/engineered AAV vectors, are absolutely stringent. Also, specific delivery into one out of several (histologically) closely related tissues (e.g. a specific type of neurons; a tumor, but not the surrounding healthy tissue; certain muscle types, e.g. skeletal muscles but not heart) is particularly challenging and close to impossible using existing delivery strategies. Hence, the risk of undesired CRISPR-Cas activation in off-target tissues and, consequently, of undesired side-effects during an in vivo CRISPR-Cas application including gene therapy in humans remains a major concern. Additional layers for controlling CRISPR-Cas activity in a tissue specific manner are, therefore, highly desired and urgently needed.

Endogenous microRNA (miRNA) signatures can be used to efficiently discriminate between different cell types and tissues. MicroRNAs are small, non-coding RNAs that play key roles in the regulation of mammalian gene expression. More than 1000 miRNAs have been described in humans (cf. e.g. www.mirbase.org), some of which (co-)occur mainly or exclusively in selected cell types or tissues, or which are dysregulated under specific conditions including diseases such as cancer. Several tissue-specific transgene expression strategies based on endogenous miRNA signatures exist (Xie et al., 2011; Geisler and Fechner, 2016). They commonly employ miRNA binding sites integrated typically into the 3' UTR (untranslated region) of a transgene. RNA interference (RNAi) mediated knockdown of transgene expression then occurs specifically within the tissue/cell type expressing the corresponding miRNA or miRNA set. This results in the negative regulation of transgene expression by endogenous miRNAs and thus transgene "exclusion" from unintended (off-target) tissues. A positive regulation of transgene expression by endogenous miRNAs ("miR-ON") has also been achieved (Amendola et al., 2013). In this case, a transcriptional repressor inhibiting the promoter driving a transgene is put under miRNA regulation, again by embedding miRNA binding sites into its 3' UTR. MicroRNA-induced knockdown of the repressor then releases transgene expression specifically within the tissue expressing the said miRNA (WO 2007/000668 A2). Moreover, two recent studies showed successful reduction of CRISPR-SpyCas9 activity in off-target cells by integrating miRNA target sites into the 3' UTR of the delivered Cas9 gene (Senis et al., 2014; Hirosawa et al., 2017). The latter study also created a tissue specific Cas-ON switch. To this end, the translational repressor L7Ae was put under miRNA control and Cas9 was expressed from an mRNA harboring an L7Ae binding motif (K-turn). However, the Cas-ON switch in the reported configuration is highly leaky and therefore unlikely to be of major benefit for ex vivo or in vivo studies as well as therapeutic applications.

There is, thus, a need in the art for improved means and methods for providing Cas nuclease activity in a cell- and/or tissue-specific manner. This problem is solved by the means and methods disclosed herein.

Accordingly, the present invention relates to a polynucleotide comprising (i) a sequence encoding an anti-CRISPR (Acr) polypeptide and (ii) an RNA-interference (RNAi) target sequence.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. It will be understood that any component defined herein as being included may preferably be explicitly excluded from the claimed invention by way of proviso or negative limitation.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s). In the context of nucleic acid sequences, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

The term "polynucleotide", as used herein, refers to a linear or circular nucleic acid molecule. The term encompasses single as well as partially or completely double-stranded polynucleotides. Preferably, the polynucleotide is RNA or DNA, including cDNA. Moreover, comprised are also chemically modified polynucleotides including naturally occurring modified polynucleotides such as glycosylated or methylated polynucleotides or artificially modified derivatives such as biotinylated polynucleotides. The polynucleotide of the present invention shall be provided, preferably, either as an isolated polynucleotide (i.e. isolated from its natural context) or in genetically modified form.

The polynucleotide of the invention, preferably, comprises at least one heterologous sequence, i.e. comprises sequences from at least two different species. Preferably, said sequences from two different species are the sequence encoding an Acr polypeptide and the RNAi target sequence. Also preferably, the polynucleotide comprises at least one heterologous sequence relative to a mammalian, preferably human, cell, i.e. comprises at least one nucleic acid sequence not known to occur or not occurring in a mammalian, preferably human, cell. Preferably, said heterologous sequence relative to a mammalian cell is at least the sequence encoding an Acr polypeptide. Also preferably, the polynucleotide comprises at least one sequence homologous to a sequence comprised in a mammalian cell; said homologous sequence, preferably, is the RNAi target sequence, which is, more preferably, an miRNA target sequence as specified elsewhere herein.

The polynucleotide of the present invention has the activities of (i) encoding an Acr polypeptide and (ii) comprising an RNA-interference (RNAi) target sequence, both as specified elsewhere herein; thus, as referred to herein, the polynucleotide has the activities of (i) causing production of a functional Acr polypeptide in a permissive host cell and (ii) of being degraded and/or translationally inhibited (in case the polynucleotide is an RNA) or of causing degradation and/or translational inhibition of an RNA expressed from said polynucleotide (in case the polynucleotide is e.g. a DNA) in the presence of the factors required for RNAi as specified herein below. Preferably, the RNAi target sequence is not comprised in the sequence encoding an Acr polypeptide; thus, preferably, the polynucleotide comprises said elements in the order 5'- RNAi target sequence(s) - sequence encoding an Acr polypeptide -3', or, 5'- RNAi target sequence(s) - sequence encoding an Acr polypeptide - RNAi target sequence(s) -3', or 5'- sequence encoding an Acr polypeptide - RNAi target sequence(s) -3'; more preferably the order is 5'- sequence encoding an Acr polypeptide - RNAi target sequence(s) -3'. Preferably, the polynucleotide comprises the nucleic acid sequence of SEQ ID NO:21, preferably encoding a polypeptide comprising the amino acid sequence of SEQ ID NO:20, and comprises an RNAi target sequence, preferably a miRNA target sequence, preferably selected from the list consisting of SEQ ID NOs: 40 to 43.

As used herein, the term polynucleotide, preferably, includes variants of the specifically indicated polynucleotides. More preferably, the term polynucleotide relates to the specific polynucleotides indicated. The term "polynucleotide variant", as used herein, relates to a variant of a polynucleotide related to herein comprising a nucleic acid sequence characterized in that the sequence can be derived from the aforementioned specific nucleic acid sequence by at least one nucleotide substitution, addition and/or deletion, wherein the polynucleotide variant shall have the activities as specified for the specific polynucleotide. Thus, it is to be understood that a polynucleotide variant as referred to in accordance with the present invention shall have a nucleic acid sequence which differs due to at least one nucleotide substitution, deletion and/or addition. Preferably, said polynucleotide variant comprises an ortholog, a paralog or another homolog of the specific polynucleotide or of a functional subsequence thereof, e.g. of the sequence encoding an Acr polypeptide. Also preferably, said polynucleotide variant comprises a naturally occurring allele of the specific polynucleotide or of a functional subsequence thereof, in particular of the sequence encoding an Acr polypeptide. In the context of polynucleotide variants, the term "functional subsequence", as used herein, relates to a part sequence of the polynucleotide of the present invention mediating at least one activity thereof; thus, preferably, the term functional subsequence relates to (i) a part sequence causing production of a functional Acr polypeptide in a permissible host cell, or (ii) a part sequence being degraded and/or translationally inhibited (in case the part sequence is comprised in an RNA) or of causing degradation and/or translational inhibition of an RNA expressed from said polynucleotide (in case the polynucleotide is e.g. a DNA) in the presence of the factors required for RNA-mediated degradation. Polynucleotide variants also encompass polynucleotides comprising a nucleic acid sequence which is capable of hybridizing to the aforementioned specific polynucleotides or functional subsequences thereof, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6x sodium chloride/sodium citrate (= SSC) at approximately 45°C, followed by one or more wash steps in 0.2x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1x to 5x SSC (pH 7.2). If organic solvent is present in the abovementioned buffer, for example 50% formamide, the temperature under standard conditions is approximately 42°C. The hybridization conditions for DNA:DNA hybrids are preferably for example 0.1x SSC and 20°C to 45°C, preferably between 30°C and 45°C. The hybridization conditions for DNA:RNA hybrids are preferably, for example, 0.1x SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer- based amplification of DNA, i.e. using degenerated primers against conserved domains of a polypeptide of the present invention. Conserved domains of a polypeptide may be identified by a sequence comparison of the nucleic acid sequence of the polynucleotide or the amino acid sequence of the polypeptide of the present invention with sequences of other organisms. As a template, DNA or cDNA from bacteria, fungi, plants or, preferably, from animals may be used. Further, variants include polynucleotides comprising nucleic acid sequences which are at least 70%, at least 75%, at least 80%, at least 85%, at least 90%, at least 95%, at least 98% or at least 99% identical to the specifically indicated nucleic acid sequences or functional subsequence thereof. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences specifically indicated. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit [Needleman and Wunsch (J. Mol. Biol. 48; 443-453 (1970)) and Smith and Waterman (Adv. Appl. Math. 2; 482-489 (1981))], which are part of the GCG software packet (Genetics Computer Group, 575 Science Drive, Madison, Wisconsin, USA 53711 (1991)), are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the specifically indicated nucleic acid sequences, said polynucleotide retaining the indicated activity or activities, is also encompassed as a variant polynucleotide of the present invention. A fragment as meant herein, preferably, comprises at least 100, preferably at least 200, more preferably at least 250 consecutive nucleotides of any one of the specific nucleic acid sequences or encodes an amino acid sequence comprising at least 50, preferably at least 60, more preferably at least 75 consecutive amino acids of any one of the specific amino acid sequences.

The polynucleotides of the present invention either consist, essentially consist of, or comprise the aforementioned nucleic acid sequences. Thus, they may contain further nucleic acid sequences as well. Specifically, the polynucleotides of the present invention may encode fusion proteins wherein one partner of the fusion protein is an Acr polypeptide being encoded by a nucleic acid sequence recited herein. Such fusion proteins may comprise as additional part polypeptides for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and are described elsewhere herein. Preferably, the polynucleotide encodes an Acr polypeptide fused to a nuclear localization sequence (NLS). Preferably, the polynucleotide does not comprise a nucleic acid sequence encoding a Cas nuclease; more preferably, the polynucleotide further comprises a nucleic acid sequence encoding at least a fragment of a Cas nuclease, preferably as specified elsewhere herein.

Preferably, the polynucleotide is an RNA. More preferably, the polynucleotide is a DNA comprising a nucleic acid sequence expressible as a continuous RNA comprising said sequence encoding an Acr polypeptide and said RNAi target sequence. Preferably, in case the polynucleotide is DNA, the polynucleotide is operatively linked to expression control sequences allowing expression in prokaryotic or eukaryotic, preferably in eukaryotic host cells or isolated fractions thereof. Expression of said polynucleotide comprises transcription of the polynucleotide, preferably into a translatable mRNA. Regulatory elements ensuring expression in eukaryotic cells, preferably mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Examples for regulatory elements permitting expression in eukaryotic host cells are the AOX1 or GAL1 promoter in yeast or the SMVP-, U6-, H1-, 7SK-, CMV- EFS-, SV40-, or RSV-promoter (Rous sarcoma virus), CMV-enhancer, SV40-enhancer or a globin intron in mammalian and other animal cells. Moreover, inducible or cell type-specific expression control sequences may be comprised in a polynucleotide of the present invention. Inducible expression control sequences may comprise tet or lac operator sequences or sequences inducible by heat shock or other environmental factors. Suitable expression control sequences are well known in the art. Besides elements which are responsible for the initiation of transcription, such regulatory elements may also comprise transcription termination signals, such as the SV40-poly-A site or the tk-poly-A site, downstream of the polynucleotide.

The "clustered regularly interspaced short palindromic repeats" or "CRISPR" systems are known to the skilled person, as described herein above. As used herein, the term "gRNA" includes a crRNA/tracrRNA hybrid and a crRNA-tracrRNA fusion RNA of a Cas CRISPR system, as well as a guide RNA of a CPF1 CRISPR system. More preferably, the term gRNA relates to a crRNA-tracrRNA fusion RNA of a Cas CRISPR system, as well as a guide RNA of a CPF1 CRISPR system. Most preferably, term gRNA relates to a crRNA-tracrRNA fusion RNA of a Cas CRISPR system. Preferably, the gRNA comprises at least 15, preferably at least 18, more preferably at least 20 nucleotides complementary to the target sequence. As used herein, the term "complementary", if not otherwise noted, relates to at least 90%, more preferably at least 95%, still more preferably 99% complementarity. Most preferably complementarity relates to 100% complementarity over the aforementioned number of nucleotides. Moreover, preferably, the gRNA of the present invention further comprises a nucleotide sequence mediating binding of a Cpfl endonuclease or comprises tracrRNA sequence, preferably of a Cas CRISPR system, more preferably of a Cas9 CRISPR system. Preferably, the gRNA comprises a structure 5'-targeting sequence-activation sequence-3', more preferably, the gRNA comprises a structure 5'-targeting sequence-linker loop-activation sequence-3', wherein said linker loop comprises a stem-loop comprising of from 10 to 30, more preferably of from 15 to 25 base pairs.

The terms "CRISPR-associated endonuclease" and "Cas nuclease", as used herein, both equally relate to an endonuclease, preferably an endo-DNase, recognizing a gRNA as specified herein, which is, in principle, known in the art. Preferably, the Cas nuclease is a type II CRISPR endonuclease. Preferably, the Cas nuclease is a CRISPR endonuclease from Prevotella and Francisella endonuclease, i.e. a Cpfl endonuclease. More preferably, the CRISPR endonuclease is a Cas9 endonuclease. Preferably, the Cas9 nuclease is a Cas9 endonuclease from Staphylococcus aureus or is a Cas9 endonuclease from Streptococcus pyogenes, more preferably is a Cas9 endonuclease from Streptococcus pyogenes. Preferably, the Cas nuclease has an amino acid sequence as shown in SEQ ID NO:36, preferably encoded by a nucleic acid sequence as shown in SEQ ID NO:37. The term "fragment of a Cas nuclease", as used herein, relates to a polypeptide fragment of a Cas nuclease which by itself is not catalytically active as a nuclease, however can be reconstituted to form a catalytically active nuclease by contacting said fragment with a second fragment which by itself is not catalytically active as well. Thus, a fragment of a Cas nuclease, as referred to herein, is a reconstitutable fragment. An example of a combination of fragments of a Cas nuclease corresponding to the above definition is shown herein in the examples. Preferably, reconstitution of Cas activity is accomplished by fusion of two non-identical fragments of a Cas polypeptide to auxiliary peptide sequences mediating binding between the two fragments. More preferably, as shown in the examples, reconstitution of Cas activity is accomplished by fusion of two non-identical fragments to auxiliary peptide sequences mediating trans-protein splicing between the two fragments, e.g. by fusing the two Cas fragments to split intein sequences, preferably amino acid sequences comprisisng the sequences of SEQ ID NOs:24 and 32, preferanly encoded by nucleic acid sequences comprising the sequences of SEQ ID NOs: 25 and 33, respectively). Accordingly, a fragment of a Cas nuclease, preferably has the amino acid sequence of SEQ ID NO:22, preferably encoded by a polynucleotide comprising SEQ ID NO:23 and/or or SEQ ID NO:30, preferably encoded by a polynucleotide comprising SEQ ID NO:31.

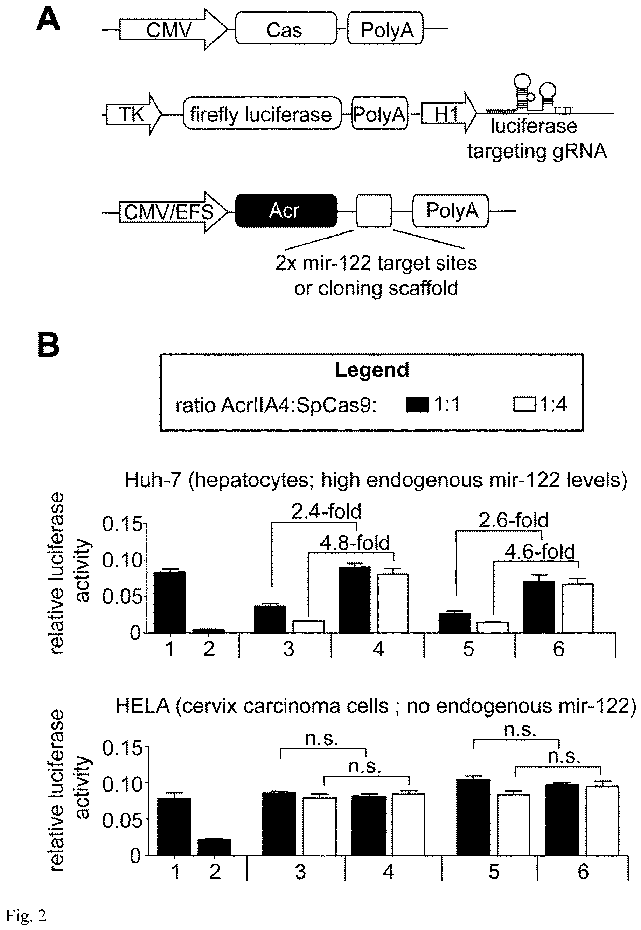

The terms "anti-CRISPR polypeptide" and "Acr polypeptide" are known to the skilled person and relate equally to a polypeptide having the activity of inhibiting at least one Cas nuclease, preferably a Cas9 nuclease. Acr polypeptides are known in the art e.g. from Pawluk et al. (2016), Cell 167(7): 1829, and from Rauch et al. (2017), Cell 168(1-2): 150. The inhibitory activity of a polypeptide to inhibit a Cas nuclease can be determined by determining the activity of said Cas nuclease in the presence of the suspected Acr polypeptide, preferably as specified herein in the Example of Fig. 2. Preferably, a polypeptide is identified as an Acr polypeptide if the activity of at least one Cas nuclease is inhibited by at least 50% in an assay as specified above. More preferably, a polypeptide is identified as an Acr polypeptide if the activity of at least one Cas9 nuclease, more preferably of Cas9 endonuclease from Streptococcus pyogenes, is inhibited by at least 50% in an assay as specified above. However, as will be understood by the skilled person, it is also within the capabilities of the skilled person to establish whether a polypeptide of interest is an Acr polypeptide for another Cas nuclease of interest. As will also be understood, preferably, the Acr polypeptide of the present invention is selected such that it inhibits the Cas nuclease intended to be used. Thus, e.g. in case the Cas9 endonuclease from Streptococcus pyogenes is intended to be used for genetic modification and shall be inhibited in at least a part of cells contacted therewith, an Acr polypeptide of a Listeria monocytogenes prophage may be used. Thus, preferably, the Acr polypeptide is an Acr polypeptide of a Listeria monocytogenes prophage, more preferably is an AcrIIA2 or AcrIIA4 polypeptide, most preferably an AcrIIA4 polypeptide. Preferably, the Acr polypeptide comprises, preferably consists of, an amino acid sequence as shown in SEQ ID NO:20, more preferably encoded by a nucleic sequence comprising, preferably consisting of, the sequence as shown in SEQ ID NO:21.

The term "RNA-interference" (RNAi) is known to the skilled person to relate to an endogenous biological process of most higher cells, including in particular mammalian cells and higher plant cells, in which double-stranded RNA (dsRNA) molecules inhibit expression of genes comprising a sequence complementary to a double-stranded RNA, mediated by the RNA-induced silencing complex (RISC). Without wishing to be bound by theory, RNAi is generally used to silence expression of a gene of interest by targeting mRNA. Briefly, the process of RNAi in the cell is initiated by dsRNAs which are cleaved by a ribonuclease, thus producing interfering RNA. The interfering RNA binds to another intracellular enzyme complex which is thereby activated to target whatever mRNA molecules are sufficiently complementary to the interfering RNA sequence. The function of the complex is to target the homologous mRNA molecule through base pairing interactions between one of the interfering RNA strands and the target mRNA. The effect of this binding largely depends on the degree of similarity between the interfering RNA and the target: in case there is imperfect complementarity (at least one base mismatch), the effect mainly is translational inhibition, i.e. prevention of translation of the target mRNA; in case there is perfect complementarity (no mismatch) the effect mainly is degradation of the target mRNA: The mRNA is then cleaved approximately 12 nucleotides from the 3' terminus of the siRNA and degraded. In this manner, specific mRNAs can be targeted and translationally inhibited and/or degraded, thereby resulting in a loss of protein expression from the targeted mRNA. As is also known to the skilled person, RNAi can be caused by an exogenous (heterologous) dsRNA molecule, which is cleaved to provide a silencing RNA (siRNA), or may be initiated by an endogenous dsRNA molecule, cleaved to provide a micro-RNA (miRNA). Both pathways converge at the RISC, which, depending on the degree of homology between the dsRNA and its target, may induce translational repression and/or degradation of the targeted RNA. As is understood by the skilled person, the exogenous dsRNA causing RNAi may be provided to the cell as such, e.g. by transfection of a dsRNA, which may be a short hairpin RNA (shRNA), or may be provided by expressing an RNA forming such dsRNA. Thus, unless otherwise noted, the term siRNA includes shRNA.

Accordingly, the term "RNAi target sequence", as used herein, relates to a nucleic acid sequence specifically hybridizing to a dsRNA inducing RNA interference (interfering RNA). Thus, the RNAi target sequence is a sequence essentially complementary to at least one RNAi inducing molecule (interfering RNA). Preferably, the RNAi target sequence is a miRNA target sequence or a siRNA target sequence, preferably is a miRNA target sequence. Thus, preferably, the present invention relates to a polynucleotide comprising (i) a sequence encoding an anti-CRISPR (Acr) polypeptide and (ii) a miRNA target sequence.

Preferably, the polynucleotide of the present invention comprises at least two RNAi target sequences. As will be understood, the two RNAi target sequences may be essentially identical, or may be different. Thus, in the first case, the at least two RNAi target sequences are essentially complementary to the same interfering RNA, preferably are essentially complementary to the same nucleic acid sequence of an interfering RNA. More preferably, in such case, the at least two RNAi target sequences are 100% complementary to the same miRNA, preferably are 100% complementary to the same nucleic acid sequence of an interfering RNA. In the second case, the at least two RNAi target sequences are not essentially identical, more preferably are less than 80%, even more preferably less than 70%, most preferably less than 60% identical to each other. Thus, preferably, in case the at least two RNAi target sequences are different, they are essentially, preferably 100%, complementary to two different interfering RNAs. As will be understood by the skilled person, the polynucleotide of the present invention may also comprise more than two RNAi target sequences, e.g. three essentially identical RNAi target sequences, or two essentially identical target sequences plus one RNAi target sequence non-identical to the other two, or an analogous constellation.

As will be also understood by the skilled person, the polynucleotide of the invention may also comprise RNAi target sequences of different types, e.g. one siRNA target site and one miRNA target site; or three RNAi target sites of which two are miRNA target sites (which may be essentially identical, or not) and one siRNA target site. Preferably, the polynucleotide comprises at least one miRNA target site, more preferably at least two miRNA target sites, even more preferably at least two essentially identical miRNA target sites. According to the invention, inclusion of miRNA target sites is, preferably, used to ensure Cas nuclease activity in one or more specific tissue(s) and/or cell type(s) and inclusion of siRNA target sites is used to permit external induction of Cas nuclease activity.

The term "interfering RNA", as used herein, relates to an RNA molecule inducing RNA interference; as detailed elsewhere herein, the interfering RNA is an at least partially double-stranded RNA molecule; correspondingly, the term interfering single-stranded RNA (ss-iRNA) relates to a single-stranded RNA derived from an interfering RNA by the cellular silencing components and inducing RNA interference. The term interfering RNA preferably refers to RNA having a duplex structure comprising two complementary and anti-parallel nucleic acid strands. Not all nucleotides of a double-stranded portion of an interfering RNA necessarily exhibit complete Watson-Crick base pairs; the two RNA strands may be substantially complementary. The RNA strands forming the double-stranded portion of the interfering RNA may have the same or a different number of nucleotides, with the maximum number of base pairs being the number of nucleotides in the shorter strand of the RNA. Preferably, the double-stranded region of the interfering RNA is no more than 200, more preferably less than 100, even more preferably less than 50, most preferably between 19 and 23, nucleotides in length. The interfering RNA is subsequently degraded by a ribonuclease enzyme into a short interfering RNA (siRNA) or a microRNA (miRNA). The complementary regions of the interfering RNA or ss-iRNA allow sufficient hybridization to the target RNA and thus mediate RNAi. In mammalian cells, ss-iRNAs are approximately 20-25 nucleotides in length. The ss-iRNA sequence needs to be of sufficient length to bring the ss-iRNA and target RNA together through complementary base-pairing interactions. The length of the ss-iRNA is preferably of sufficient length to stably interact with the target RNA; specifically 10-30 nucleotides; more specifically is any integer between 10 and 30 nucleotides, most preferably 10, 11, 12, 13, 14, 15, 16, 17, 18, 19, 20, 21, 22, 23, 24, 25, 26, 27, 28, 29, or 30. By "stably interact" is meant interaction of the ss-iRNA with the target nucleic acid, preferably by forming hydrogen bonds with complementary nucleotides in the target under physiological conditions. Preferably, complementarity between the ss-iRNA and the RNA target is at least 80%, more preferably at least 90%, even more preferably at least 95%, still more preferably at least 98%. Most preferably, complementarity between the ss-iRNA and the RNA target is 100%. Preferably, said complementarity is calculated over the stretch of at least 20 nucleotides providing the highest complementarity value, more preferably is calculated over the whole length of the ss-iRNA. Methods relating to the use of RNAi to silence genes in organisms, including C. elegans, Drosophila, plants, and mammals, are known in the art (see, e.g., WO 0129058; WO 09932619; and Elbashir (2001), Nature 411: 494-498).

Preferably, the interfering RNA is a siRNA. In a narrow sense, the term siRNA relates to the short, single-stranded RNA molecule cleaved from a dsRNA precursor and incorporated into the protein complex mediating gene silencing; however, in a broader sense, the term is used both in the aforementioned sense and for the precursor dsRNA from which the single-stranded siRNA is cleaved; the latter, broader definition is the definition as used herein; for differentiation, the siRNA in the narrow sense is referred to as single-stranded siRNA. Thus, the term "siRNA", as used herein, refers to an exogenous dsRNA which is at least in part essentially complementary to an RNAi target sequence of the invention and which diminishes or abolishes gene expression by RNA interference (RNAi). The term "exogenous RNA", as used herein, relates to an RNA originating from the exterior of a cell; for the avoidance of doubt, the term includes RNA introduced into a cell as such, but also includes RNA for which a different polynucleotide, in particular a DNA, was introduced to a cell to cause expression of said RNA. Structures of appropriate siRNAs and methods for providing them are well-known in the art. Preferably, the siRNA is an RNA comprising inverted repeats, preferably intervened by a "loop" sequence, wherein said inverted repeat sequences comprise a sequence essentially complementary to an RNAi target sequence, preferably having a length and complementarity as specified herein above.

More preferably, the interfering RNA is a microRNA (miRNA). In analogy to the above, the term miRNA relates to the short, single-stranded RNA molecule cleaved from a miRNA precursor and incorporated into the protein complex mediating gene silencing; however, in a broader sense, the term is used both in the aforementioned sense and for the precursor miRNA from which the single-stranded miRNA is cleaved; the latter, broader definition is the definition as used herein; for differentiation, the miRNA in the narrow sense is referred to as single-stranded miRNA. Thus, the term "miRNA", as used herein, refers to an endogenous RNA which is at least in part essentially complementary to an RNAi target sequence of the invention and which diminishes or abolishes gene expression by RNA interference (RNAi). The term "endogenous RNA", as used herein, relates to an RNA originating from the cell itself; preferably, the miRNA is a nucleic acid transcribed from the genome of the cell. Preferably, the miRNA is a miRNA expressed in at most five tissues and/or cell types, more preferably at most four tissues and/or cell types, even more preferably at most three tissues and/or cell types, still more preferably at most two tissues and/or cell types; most preferably, expression of the miRNA is tissue- and/or cell type-specific. Specificity of expression in tissue and cell type are known for most miRNAs and can be derived from public databases. Moreover, methods for determining tissue and/or cell type specificity of a miRNA are known in the art, e.g. from Landgraf et al. ((2007), Cell 129(7):1401, Guo et al. (2014), Scientific Reports 4, doi:10.1038/srep05150; Babak et al. (2004), RNA 10:1813; Liu et al. (2011), J Biomed Sci Eng 4(19): 666; Ludwig et al. (2016), NAR 44(8):3865; and Baker et al. (2017) JASN, doi: 10.1681/ASN.2016121280). Preferably, the miRNA is a mammalian, more preferably a human miRNA; e.g., the database miRBase (www.mirbase.org), release 21, lists 296 high-confidence human miRNAs. More preferably, the miRNA is selected from human miRNAs miR-1 (SEQ ID NO:44), miR-206 (SEQ ID NO:45), miR-208a (SEQ ID NO:46), miR-122 (SEQ ID NO:47), and miR-142 (SEQ ID NO:48).

Advantageously, it was found in the work underlying the present invention that the activity of a Cas nuclease can be inhibited in a cell by expressing an Acr gene, but this inhibition can be relieved in a cell type-specific manner if a miRNA-dependent degradation signal is incorporated in the mRNA encoding the Acr. As will be understood, by appropriately selecting the miRNA target sequence, e.g. a miRNA expressed in more than one cell type, or by including target sequences for more than one miRNA, release of inhibition in more than one cell type and/or tissue is possible. Moreover, by providing a polynucleotide comprising only a siRNA target site by means of a cell type-specific delivery means, and by further providing a siRNA by a second delivery means, double specificity of Cas nuclease activity can be achieved; i.e. in case the first delivery means is specific for a first receptor on the surface of a cell, and the second delivery means is specific for a second receptor, activation of Cas nuclease will only occur in cells having both receptors. Of advantage is further that inhibition of the Cas nuclease takes place post-translationally. Hence, Cas activity can be efficiently suppressed at any point in time as transcription and translation of the small Acr (87 amino acids in case of AcrIIA4) is faster and more efficient as compared to the long Cas (>1,000 amino acids). Further, in contrast to existing miR-ON/Cas-ON strategies, the system of the invention is independent of specific regulatory elements required within the Cas transgene itself such as repressor-dependent promoter variants or repressor-targetable, regulatory elements within UTRs (untranslated regions) of the transgene.

The definitions made above apply mutatis mutandis to the following. Additional definitions and explanations made further below also apply for all embodiments described in this specification mutatis mutandis.

The present invention further relates to a vector comprising the polynucleotide according to the present invention.

As used herein, the term "vector" relates to a polynucleotide comprising structural determinants required for delivering into and/or stably maintaining and/or propagating the polynucleotide in a cell, said structural determinants optionally including the elements of an outer shell of a self-propagating entity, e.g. a virus. The term, preferably, encompasses phage, plasmid, and viral vectors as well as artificial chromosomes, such as bacterial or yeast artificial chromosomes. Thus, the vector may be or comprise RNA or DNA. Moreover, the term also relates to targeting constructs which allow for random or site-directed integration of the targeting construct into genomic DNA. Such target constructs, preferably, comprise DNA of sufficient length for either homologous or heterologous recombination as described in detail below. The vector encompassing the polynucleotide of the present invention, preferably, further comprises selectable markers for propagation and/or selection in a host. The vector may be delivered into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerenes. Alternatively, a plasmid vector may be introduced by heat shock or electroporation techniques. Should the vector be a virus, it may be packaged in vitro using an appropriate packaging cell line prior to application to host cells. Retroviral vectors may be replication-competent or replication-defective. In the latter case, viral propagation generally will occur only in complementing host/cells. Preferably, the vector is an adeno-associated virus, preferably a non-replication-competent adeno-associated virus. Targeted delivery, i.e. delivery of a polynucleotide or vector into one or more cell population(s) or tissue(s) with high specificity may be achieved by viral vectors, which may have a natural tropism for cell(s) and/or tissue(s) of interest or may be retargeted thereto; however, also non-viral targeting methods are known to the skilled person, e.g. from Harris et al. (2010), Biomaterials 31(5): 998.

Preferably, in the vector of the invention the polynucleotide is operatively linked to expression control sequences as specified herein above. In this context, suitable expression vectors are known in the art such as Okayama-Berg cDNA expression vector pcDV1 (Pharmacia), pBluescript (Stratagene), pCDM8, pRc/CMV, pcDNA1, pcDNA3 (ThermoFisher) or pSPORT1 (Invitrogen). Analogous expression control vectors are also known for RNA vectors such as retroviruses. Preferably, the vector is an expression vector and a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

The present invention also relates to a host cell comprising the polynucleotide according to the present invention and/or the vector according to the present invention.

As used herein, the term "host cell" relates to any cell capable of receiving, and optionally maintaining and/or propagating, the polynucleotide and/or the vector of the present invention. Preferably, the cell is a bacterial cell, more preferably a cell of a common laboratory bacterial strain known in the art, most preferably an Escherichia strain, in particular an E. coli strain. Also preferably, the host cell is a eukaryotic cell, preferably a plant or yeast cell, e.g. a cell of a strain of baker's yeast, or is an animal cell. More preferably, the host cell is an insect cell or a mammalian cell, in particular a mouse or rat cell. Even more preferably, the host cell is a mammalian cell, most preferably is a human cell. The term "permissive host cell", as used herein, relates to a host cell as specified herein comprising all components required for RNA interference to occur being general for the pathway; i.e. in a permissive host cell RNA interference is expected to occur in case a target RNA and an interfering RNA are provided. Preferably, the permissive host cell is a plant or yeast cell, e.g. a cell of a strain of baker's yeast, or is an animal cell. More preferably, the permissive host cell is an insect cell or a mammalian cell, in particular a mouse or rat cell. Even more preferably, the permissive host cell is a mammalian cell, most preferably is a human cell.

The present invention further relates to the polynucleotide according to the present invention and/or the vector according to the present invention and/or the host cell according to the present invention for use in medicine.

The present invention further relates to the polynucleotide according to the present invention and/or the vector according to the present invention and/or the host cell according to the present invention for use in treatment and/or prevention of genetic disease, neurodegenerative disease, cancer, and/or infectious disease.

The means and methods of the present invention are, in principle, usable in treatment and/or prevention of each and every disease for which genetic modification of a cell, preferably a specific type of cell, is considered beneficial. Such is the case in particular in genetic disease, neurodegenerative disease, cancer, and infectious disease. As used herein, the term "genetic modification", preferably, includes modification of any kind of nucleic acid comprised in a host cell at a given time, including nuclear DNA, organelle DNA (mitochondrial DNA or plastid DNA), but also nucleic acid from an infectious agent, either as free nucleic acid or covalently connected to the DNA of the host cell. Preferably, genetic modification is modification of nucleic acid, preferably DNA, present in the nucleus of a host cell.

The term "treatment" refers to an amelioration of the diseases or disorders referred to herein or the symptoms accompanied therewith to a significant extent. Said treating as used herein also includes an entire restoration of the health with respect to the diseases or disorders referred to herein. It is to be understood that treating as used in accordance with the present invention may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 10%, at least 20%, at least 50%, at least 60%, at least 70%, at least 80%, or at least 90% of the subjects of a given cohort or population.

The term "preventing" refers to retaining health with respect to the diseases or disorders referred to herein for a certain period of time in a subject. It will be understood that the said period of time is dependent on a variety of individual factors of the subject and the specific preventive treatment. It is to be understood that prevention may not be effective in all subjects treated with the compound according to the present invention. However, the term requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification.

The term "genetic disease", as used herein, relates to a disease causally linked to one or more modifications, preferably mutations in the genome of an individual. Thus, preferably, the genetic disease is causally linked to one or more epigenetic changes, more preferably is causally linked to one or more genetic mutations. As will be understood, symptoms of a genetic disease often are caused by expression of a mutated gene and/or lack of expression of a gene providing normal function of the gene product in one or more specific tissue(s) and/or cell type(s). Thus, it may be preferable to genetically modify by Cas activity only those cells in which the mutation contributes to disease. Preferably, the genetic disease is Duchenne muscular dystrophy, Huntington's disease, Hemophilia A/B, cystic fibrosis, myotubular myopathy, a glycogen storage disorder, or sickle cell anemia, the causes and symptoms of which are known to the skilled person from textbooks of medicine.

The term "neurodegenerative disease" relates to a disease caused by progressive loss of structure and/or function of neurons in the peripheral and/or central nervous system of an individual. Preferably, the neurodegenerative disease is a neurodegenerative disease of motoneurons and/or a neurodegenerative disease of the central nervous system. Preferably, the neurodegenerative disease is Alzheimer's disease, amyotrophic lateral sclerosis (ALS), Parkinson's disease, or a spinocerebellar ataxia, preferably spinocerebellar ataxia type 1 (SCA1). As will be understood, many neurodegenerative diseases are genetic diseases.

The term "cancer" is, in principle, understood by the skilled person and relates to a disease of an animal, including man, characterized by uncontrolled growth by a group of body cells ("cancer cells"). This uncontrolled growth may be accompanied by intrusion into and destruction of surrounding tissue and possibly spread of cancer cells to other locations in the body. Preferably, also included by the term cancer is a relapse. Thus, preferably, the cancer is a non-solid cancer, e.g. a leukemia, or is a tumor of a solid cancer, a metastasis, or a relapse thereof. As is known to the skilled person, cancer cells accumulate mutations in particular in oncogenes or in tumor-suppressor genes, which may be amenable to correction by genetic modification. Moreover, the means and methods of the present invention may be used to induce cell death, e.g. via apoptosis, specifically in cancer cells. Preferably, treating cancer is reducing tumor and/or cancer cell burden in a subject. As will be understood by the skilled person, effectiveness of treatment of e.g. cancer is dependent on a variety of factors including, e.g. cancer stage and cancer type.

The term "infectious disease" is, in principle, understood by the skilled person. Preferably, the term as used herein relates to an infectious disease in which the replicative cycle of the infectious agent comprises at least one stage in which the genome of the infectious agent is present in a permissive host cell. Thus the infectious disease, preferably, is a viral infection, preferably is immunodeficiency virus infection, herpes virus infection, papillomavirus infection, or hepatitis B virus infection.

The present invention further relates to a kit comprising
(I) a first polynucleotide comprising (i) a sequence encoding an anti-CRISPR (Acr) polypeptide and (ii) an RNA-interference (RNAi) target sequence; and a Cas nuclease or a second polynucleotide comprising an expressible gene encoding a Cas nuclease;
(II) a first polynucleotide comprising (i) a sequence encoding an anti-CRISPR (Acr) polypeptide and (ii) an RNA-interference (RNAi) target sequence, said first polynucleotide further comprising an expressible gene encoding a first fragment of a Cas nuclease; and a second polynucleotide comprising an expressible gene encoding a second fragment of a Cas nuclease, wherein said first and second fragment of said Cas nuclease together reconstitute an active Cas nuclease; or
(III) a first polynucleotide comprising (i) a sequence encoding an anti-CRISPR (Acr) polypeptide, (ii) an RNA-interference (RNAi) target sequence, and (iii) a sequence encoding a Cas nuclease; and a second polynucleotide comprising an expressible gene encoding an inhibitory RNA, preferably a siRNA or a miRNA hybridizing to said RNAi target sequence, more preferably a siRNA hybridizing to said RNAi target sequence.

The term "kit", as used herein, refers to a collection of the aforementioned compounds, means or reagents of the present invention which may or may not be packaged together. The components of the kit may be comprised by separate vials (i.e. as a kit of separate parts) or provided in a single vial. Moreover, it is to be understood that the kit of the present invention, preferably, is to be used for practicing the methods referred to herein above. It is, in an embodiment, envisaged that all components are provided in a ready-to-use manner for practicing the methods referred to above. Further, the kit, in an embodiment, contains instructions for carrying out said methods. The instructions can be provided by a user's manual in paper or electronic form. In addition, the manual may comprise instructions for interpreting the results obtained when carrying out the aforementioned methods using the kit of the present invention. Preferably, the first polynucleotide comprised in the kit is the polynucleotide of the present invention. Also preferably, expression of a nucleic acid sequence encoding a Cas nuclease or fragment thereof optionally comprised by a polynucleotide of the kit is driven by a cell type-specific promoter. Also preferably, the kit further comprises a polynucleotide encoding at least one guide RNA (gRNA). Preferably, the polynucleotide encoding at least one gRNA is the first or the second polynucleotide, more preferably is the second polynucleotide. As will be understood from the above, the description of the kit comprising polynucleotides, preferably, relates to a kit comprising corresponding vectors mutatis mutandis.

Preferably, the kit further comprises at least one delivery means for the polynucleotides it comprises, the term "delivery means" relating to any means suitable to cause a polynucleotide of the kit to enter the interior of a host cell, preferably a permissive host cell. Suitable delivery means are known in the art and include in particular transfection reagents, packaging means, and the like. Preferably, the polynucleotides of the present invention are pre-packaged in a delivery means, e.g. in viral particles. As specified herein above, the skilled person is aware of delivery means providing different specificities for cellular receptors. Accordingly, preferably, the first polynucleotide of the kit is packaged in a first delivery means targeting a first receptor of a permissive host cell; and said second polynucleotide is packaged in a second delivery means targeting a second receptor of said permissive host cell. Preferably, the first and second delivery means are non-identical and differ at least in the cellular receptor which they preferentially target. Also preferably, the first receptor and the second receptor are non-identical and are both present in an accessible manner, preferably on the surface of a target cell.

In a first embodiment, the kit comprises a first polynucleotide comprising (i) a sequence encoding an anti-CRISPR (Acr) polypeptide and (ii) an RNA-interference (RNAi) target sequence; and a second polynucleotide comprising an expressible gene encoding a Cas nuclease. Accordingly, by co-delivering the first and second polynucleotide into a host cell, Cas activity is inhibited, however, can be activated if the cell expresses a miRNA corresponding to the RNAi target sequence of the first polynucleotide or by additionally delivering a siRNA or shRNA corresponding to the RNAi target sequence of the first polynucleotide. Thus, preferably, in the first embodiment, the kit is for cell type- or tissue-specific Cas activation, or for inducible Cas activation.

In a second embodiment, the kit comprises a first polynucleotide comprising (i) a sequence encoding an anti-CRISPR (Acr) polypeptide and (ii) an RNA-interference (RNAi) target sequence, said first polynucleotide further comprising an expressible gene encoding a first fragment of a Cas nuclease, and a second polynucleotide comprising an expressible gene encoding a second fragment of a Cas nuclease, wherein said first and second fragment of said Cas nuclease together reconstitute an active Cas nuclease. As will be understood, the fragments of a Cas nuclease expressed from the polynucleotides of the kit are non-active, however, together generate an active Cas nuclease, as specified herein above. Accordingly, by co-delivering the first and second polynucleotide into a host cell, Cas activity is reconstituted, however is inhibited by the Acr polypeptide; thus, Cas activity can be activated as detailed above for the first embodiment of the kit. As will be understood, in case the first and the second polypeptide are delivered by delivery means targeting two non-identical cellular receptors, specificity of delivery of Cas nuclease activity may be further increased.

In a third embodiment, the kit comprises a first polynucleotide comprising (i) a sequence encoding an anti-CRISPR (Acr) polypeptide, (ii) an RNA-interference (RNAi) target sequence, and (iii) a sequence encoding a Cas nuclease; and a second polynucleotide comprising an expressible gene encoding an inhibitory RNA, preferably a siRNA or shRNA or a miRNA hybridizing to said RNAi target sequence, more preferably a siRNA hybridizing to said RNAi target sequence. As will be understood, in case the first and the second polypeptide are delivered by delivery means targeting two non-identical cellular receptors, Cas nuclease activation may be restricted to cells carrying both the first and the second receptor.

The present invention further relates to a method for genetically modifying a host cell comprising
a1) contacting said host cell with a polynucleotide according to the present invention; with the vector according to the present invention; and/or with the host cell according to the present invention; and further contacting said host cell with a Cas nuclease activity and a gRNA; or
a2) contacting said host cell with the components comprised in a kit according to the present invention and a gRNA; and,
b) thereby, genetically modifying said host cell.

The method for genetically modifying a host cell of the present invention, preferably, is an in vitro method. In an embodiment, the method may, however, also be applied in vivo. Moreover, the method may comprise steps in addition to those explicitly mentioned above. For example, further steps may relate, e.g., to providing host cells, preferably permissive host cells, for step a1) and/or a2), or incubating said cells for an appropriate time in step b). Moreover, one or more of said steps may be performed by automated equipment.

The term "genetically modifying" is, in principle, understood by the skilled person and relates to introducing at least one modification into a nucleic acid of a host cell, wherein said modification causes altered expression of at least one gene. Thus, genetically modifying, preferably, includes introducing at least one mutation, as well as introducing at least one epigenetic modification, as disclosed e.g. in Jurkowski et al., (2015), Clin Epigenetics 7(1):18. Preferably, genetically modifying is introducing at least one mutation into a nucleic acid, preferably DNA, more preferably the genome of the host cell. Preferably, said mutation is deletion of a part of the genome by a Cas nuclease activity and/or introducing a nucleic acid sequence comprising at least one mutation compared to the original sequence into the genome of said cell. Thus, more preferably, genetically modifying is exchanging a gene or a part thereof for a modified version thereof. Most preferably, genetically modifying is exchanging a non-functional copy of a gene for a functional copy thereof.

The term "contacting", as used in the context of the methods of the present invention, is understood by the skilled person. Preferably, the term relates to bringing at least one polynucleotide, vector, and/or host cell of the present invention in physical contact with a host cell, preferably a permissive host cell e.g. allowing the sample and the compound(s) to interact. Preferably, contacting includes delivery of at least one polynucleotide of the present invention into the interior of a host cell, preferably via a delivery means.

The present invention also relates to a method for treating genetic disease, neurodegenerative disease, cancer, and/or infectious disease in a subject suffering therefrom, said method comprising
a1) contacting said host cell with a polynucleotide according to the present invention; with the vector according to the present invention; and/or with the host cell according to the present invention; and further contacting said host cell with a Cas nuclease activity and a gRNA; or
a2) contacting said host cell with the components comprised in a kit according to the present invention and a gRNA; and,
b) thereby, treating genetic disease, neurodegenerative disease, cancer, and/or infectious disease in said subject.

The method for treating a subject of the present invention, preferably, is an in vivo method. In an embodiment, the method may, however, also be applied in vitro. Moreover, the method may comprise steps in addition to those explicitly mentioned above. For example, further steps may relate, e.g., to providing a sample of host cells, preferably permissive host cells, for step a1) and/or a2), or incubating said cells for an appropriate time in step b). Moreover, one or more of said steps may be performed by automated equipment.

According to the present invention, the polynucleotide, the vector, the host cell, and/or the components of the kit are, preferably, administered to a sample of the subject comprising permissive host cells, e.g. a blood sample, and said sample or cells derived thereof are re-administered to said subject after they were genetically modified. More preferably, the polynucleotide, the vector, the host cell, and/or the components of the kit are administered to the subject directly, e.g. by intravenous injection or topical application.

Thus, preferably, the polynucleotide, the vector, the host cell, and/or the components of the kit of the present invention are provided as a pharmaceutical composition. The term "pharmaceutical composition", as used herein, comprises the compounds of the present invention and optionally one or more pharmaceutically acceptable carrier. The compounds of the present invention can be formulated as pharmaceutically acceptable salts. Acceptable salts comprise acetate, methylester, HCl, sulfate, chloride and the like. The pharmaceutical compositions are, preferably, administered topically or systemically. Suitable routes of administration conventionally used for drug administration are oral, intravenous, or parenteral administration as well as inhalation. However, depending on the nature and mode of action of a compound, the pharmaceutical compositions may be administered by other routes as well.
For example, polynucleotide compounds may be administered in a gene therapy approach by using viral vectors or viruses or liposomes, as specified herein above. Moreover, the compounds can be administered in combination with other drugs either in a common pharmaceutical composition or as separated pharmaceutical compositions wherein said separated pharmaceutical compositions may be provided in form of a kit of parts. The compounds are, preferably, administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

The carrier(s) must be acceptable in the sense of being compatible with the other ingredients of the formulation and being not deleterious to the recipient thereof. The pharmaceutical carrier employed may be, for example, either a solid, a gel or a liquid. Exemplary of solid carriers are lactose, terra alba, sucrose, talc, gelatin, agar, pectin, acacia, magnesium stearate, stearic acid and the like. Exemplary of liquid carriers are phosphate-buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

The diluent(s) is/are selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like.

A therapeutically effective dose refers to an amount of the compounds to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. A typical dose can be, for example, in the range of 1 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. However, depending on the subject and the mode of administration, the quantity of substance administration may vary over a wide range to provide from about 0.01 mg per kg body mass to about 10 mg per kg body mass. In case a viral vector, in particular adeno-associated viral vector is administered, preferred doses are from 5 x 10¹¹, to 2 x 10¹³ viral particles or viral genomes / kg body weight; as will be understood, these exemplary doses may be modified depending, in addition to the factors described above, on additional factors like type of virus, target organ, and the like.

The pharmaceutical compositions and formulations referred to herein are administered at least once in order to treat or ameliorate or prevent a disease or condition recited in this specification. However, the said pharmaceutical compositions may be administered more than one time, for example from one to four times daily up to a non-limited number of days.

Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adopted to the mode of administration, i.e. in the forms of tablets, capsules, suppositories, solutions, suspensions or the like. Dosage recommendations shall be indicated in the prescribers or users instructions in order to anticipate dose adjustments depending on the considered recipient.

Furthermore, the present invention relates to the use of the polynucleotide according to the present invention; the vector according to the present invention; the host cell according to the present invention; and/or the kit according to the present invention for genetically modifying a host cell.

Also, the present invention relates to the use of a polynucleotide according to the present invention; the vector according to the present invention; the host cell according to the present invention; and/or the kit according to the present invention; the manufacture of a medicament for treating genetic disease, neurodegenerative disease, cancer, and/or infectious disease.

In view of the above, the following embodiments are particularly envisaged:
1. A polynucleotide comprising (i) a sequence encoding an anti-CRISPR (Acr) polypeptide and (ii) a RNA-interference (RNAi) target sequence.
2. The polynucleotide of embodiment 1, wherein said Acr polypeptide is an AcrIIA4 polypeptide.
3. The polynucleotide of embodiment 1 or 2, wherein said sequence encoding an Acr polypeptide comprises
   a) the nucleic acid sequence of SEQ ID NO:21;
   b) a nucleic acid sequence being at least 70% identical to the sequence of SEQ ID NO:21;
   c) a nucleic acid sequence encoding a polypeptide comprising the amino acid sequence of SEQ ID NO:20; or
   d) a nucleic acid sequence encoding a polypeptide comprising an amino acid sequence being at least 70% identical to the sequence of SEQ ID NO:20.
4. The polynucleotide of any one of embodiments 1 to 3, wherein said RNAi target sequence is a miRNA target sequence or a siRNA target sequence, preferably is a miRNA target sequence.
5. The polynucleotide of any one of embodiments 1 to 4, wherein said polynucleotide comprises at least two RNAi target sequences, preferably at least two non-identical RNAi target sequences, more preferably at least two RNAi target sequences being less than 80%, even more preferably less than 70%, most preferably less than 60% identical to each other.
6. The polynucleotide of any one of embodiments 1 to 5, wherein said polynucleotide comprises at least two RNAi target sequences, preferably at least two miRNA target sequences essentially complementary to the same miRNA, more preferably at least two target sequences being essentially complementary to the same nucleic acid sequence of a miRNA.
7. The polynucleotide of any one of embodiments 1 to 6, wherein said miRNA is a tissue-specific and/or cell type-specific miRNA.
8. The polynucleotide of any one of embodiments 1 to 7, wherein said miRNA is selected from human miRNAs miR-1, miR-206, miR-208a, miR-122, and miR-142.
9. The polynucleotide of any one of embodiments 1 to 8, wherein said polynucleotide further comprises at least one promoter causing transcription of said expressible nucleic acid sequence.
10. The polynucleotide of any one of embodiments 1 to 9, wherein said promoter is a constitutive promoter.
11. The polynucleotide of any one of embodiments 1 to 10, wherein said polynucleotide comprises the nucleic acid sequence of SEQ ID NO:21 and at least one of SEQ ID NOs: 40 to 43.
12. The polynucleotide of any one of embodiments 1 to 11, wherein said sequence encoding an Acr polypeptide is located upstream of said RNAi target sequence, preferably of all RNAi target sequences comprised in said expressible nucleic acid sequence.
13. The polynucleotide of any one of embodiments 1 to 12, wherein said polynucleotide is an RNA.
14. The polynucleotide of any one of embodiments 1 to 13, wherein said polynucleotide is a DNA comprising a nucleic acid sequence expressible as a continuous RNA comprising said sequence encoding an Acr polypeptide and said RNAi target sequence.
15. The polynucleotide of any one of embodiments 1 to 14, wherein said polynucleotide does not comprise a nucleic acid sequence encoding a CRISPR-associated (Cas) nuclease.
16. The polynucleotide of any one of embodiments 1 to 14, wherein said polynucleotide further comprises a nucleic acid sequence encoding at least a fragment of a CRISPR-associated (Cas) nuclease.
17. A vector comprising the polynucleotide according to any one of embodiments 1 to 16.
18. The vector of embodiment 17, wherein said vector is an adeno-associated virus vector.
19. A host cell comprising the polynucleotide according to any one of embodiments 1 to 16 and/or the vector according to embodiment 17 or 18.
20. A polynucleotide according to any one of embodiments 1 to 16 and/or the vector according to embodiment 17 or 18 and/or the host cell according to embodiment 19 for use in medicine.
21. A polynucleotide according to any one of embodiments 1 to 16 and/or the vector according to embodiment 17 or 18 and/or the host cell according to embodiment 19 for use in treatment of genetic disease, neurodegenerative disease, cancer, and/or infectious disease.
22. The polynucleotide, vector, and/or host cell for use of embodiment 21, wherein said genetic disease is Duchenne muscular dystrophy, Huntington's disease, Hemophilia A/B, cystic fibrosis, myotubular myopathy, a glycogen storage disorder, or sickle cell anemia; wherein said neurodegenerative disease is Alzheimer's disease, amyotrophic lateral sclerosis (ALS), Parkinson's disease, or spinocerebellar ataxia type 1 (SCA1); wherein said cancer is hepatocellular carcinoma, pancreatic cancer, osteosarcoma, leukemia or colorectal cancer; and/or wherein said infectious disease is human immunodeficiency virus infection, herpes virus infection, papillomavirus infection, or hepatitis B virus infection.
23. A kit comprising
   (I) a first polynucleotide comprising (i) a sequence encoding an anti-CRISPR (Acr) polypeptide and (ii) an RNA-interference (RNAi) target sequence; and a Cas nuclease or a second polynucleotide comprising an expressible gene encoding a Cas nuclease;
   (II) a first polynucleotide comprising (i) a sequence encoding an anti-CRISPR (Acr) polypeptide and (ii) an RNA-interference (RNAi) target sequence, said first polynucleotide further comprising an expressible gene encoding a first fragment of a Cas nuclease, and a second polynucleotide comprising an expressible gene encoding a second fragment of a Cas nuclease, wherein said first and second fragment of said Cas nuclease together reconstitute an active Cas nuclease; or
   (III) a first polynucleotide comprising (i) a sequence encoding an anti-CRISPR (Acr) polypeptide, (ii) an RNA-interference (RNAi) target sequence, and (iii) a sequence encoding a Cas nuclease; and a second polynucleotide comprising an expressible gene encoding an inhibitory RNA, preferably a siRNA, shRNA or a miRNA hybridizing to said RNA-mediated degradation target sequence, more preferably a siRNA or shRNA hybridizing to said RNA-mediated degradation target sequence.
24. The kit of embodiment 23, wherein expression of said Cas nuclease or fragment thereof is driven by a cell type-specific or tissue-specific promoter.
25. The kit of embodiment 23 or 24, wherein the Acr polypeptide encoded by the first polypeptide is a Listeria monocytogenes AcrIIA4 and wherein said Cas nuclease or fragment thereof is a Streptococcus pyogenes Cas9 nuclease.
26. The kit of any one of embodiments 23 to 25, wherein said kit comprises a polynucleotide encoding at least one guide RNA (gRNA), preferably wherein said polynucleotide encoding at least one gRNA is the first or the second polynucleotide, more preferably is the second polynucleotide.
27. The kit of any one of embodiments 23 to 26, wherein said first polynucleotide is packaged in a first delivery means targeting a first receptor of a permissive host cell and wherein said second polynucleotide is packaged in a second delivery means targeting a second receptor of said permissive host cell.
28. The kit of embodiment 27, wherein said first and second receptor are non-identical.
29. The kit of embodiment 27 or 28, wherein said first and second delivery means are non-identical.
30. The kit of any one of embodiments 23 to 29, wherein said first polynucleotide is a polynucleotide according to any one of embodiments 1 to 16.
31. A method for genetically modifying a host cell comprising
   a1) contacting said host cell with a polynucleotide according to any one of embodiments 1 to 16; with the vector according to embodiment 17 or 18; and/or with the host cell according to embodiment 19; and further contacting said host cell with a Cas nuclease activity and a gRNA; or
   a2) contacting said host cell with the components comprised in a kit according to any one of embodiments 23 to 30; and,
   b) thereby, genetically modifying said host cell.
32. A method for treating genetic disease, neurodegenerative disease, cancer, and/or infectious disease in a subject suffering therefrom, said method comprising
   a1) contacting said host cell with a polynucleotide according to any one of embodiments 1 to 16; with the vector according to embodiment 17 or 18; and/or with the host cell according to embodiment 19; and further contacting said host cell with a Cas nuclease activity and a gRNA; or
   a2) contacting said host cell with the components comprised in a kit according to any one of embodiments 23 to 30; and,
   b) thereby, treating genetic disease, neurodegenerative disease, cancer, and/or infectious disease in said subject.
33. Use of the polynucleotide according to any one of embodiments 1 to 16; the vector according to embodiment 17 or 18; host cell according to embodiment 19; and/or the kit according to any one of embodiments 23 to 30; for genetically modifying a host cell.
34. Use of the polynucleotide according to any one of embodiments 1 to 16; the vector according to embodiment 17 or 18; the host cell according to embodiment 19; and/or the kit according to any one of embodiments 23 to 30 for the manufacture of a medicament for treating genetic disease, neurodegenerative disease, cancer, and/or infectious disease.

All references cited in this specification are herewith incorporated by reference with respect to their entire disclosure content and the disclosure content specifically mentioned in this specification.

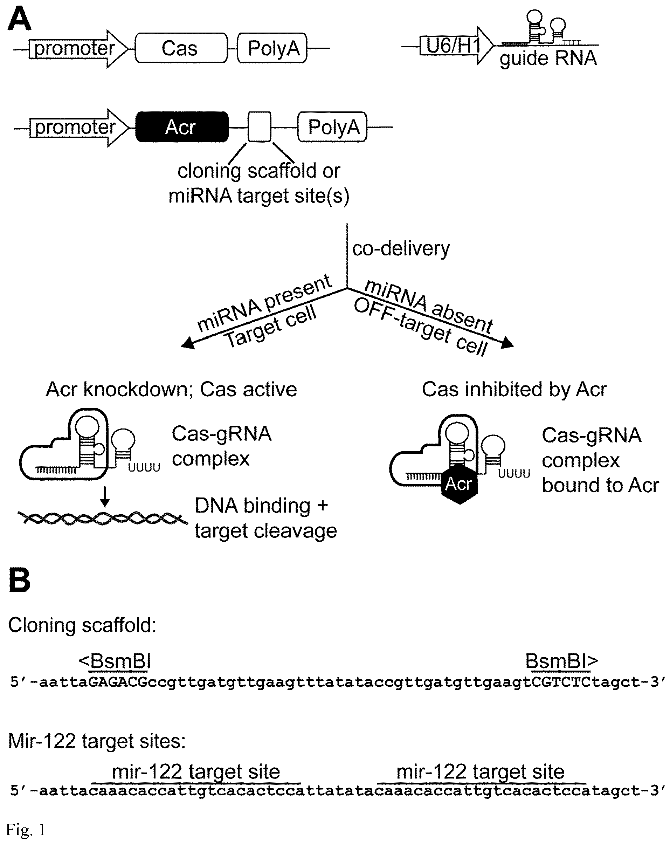

Figure Legends
Fig. 1: Cell- and tissue-specific activation of CRISPR-Cas9 using Anti-CRISPR proteins regulated by endogenous miRNAs. (A) Binding sites for miRNA(s) highly abundant in the target cell/tissue of interest, but not in relevant off-target cells/tissues are embedded into the 3' UTR of the acr transgene. Upon co-delivery of Acr and the CRISPR-Cas components (Cas and guideRNA; vector schemes, top part), knockdown of Acr and concurrent release of Cas activity occurs only within the intended target tissue (bottom part). (B) Any miRNA binding site can be easily introduced into our Acr expression vectors (cloning scaffold, SEQ ID NO:1) via BsmBI restriction sites. In all experiments, Acr constructs bearing the miR-122 target sites (lower part, SEQ ID NO:2) were compared to vectors bearing the "empty" scaffold identical in length (upper part). miR-122 target site has SEQ ID NO:40.
Fig. 2: Hepatocyte-specific activation of Cas9-mediated reporter cleavage. (A) Schematics of used constructs. The AcrIIA4 vector either bears 2x miR-122 binding sites within its 3' UTR or not (as control). The luciferase reporter also carries a constitutive *Renilla* expression cassette for normalization purposes (not shown in schematic). (B) Huh-7 cells (high endogenous miR-122 levels) or HELA cells (control, no endogenous miR-122) were triple-transfected with the constructs in A. The ratio of transfected AcrIIA4 to Cas9 constructs is indicated. Forty-eight h post-transfection firefly and *Renilla* luciferase activity were measured by dual-luciferase assay. Firefly luciferase photon counts were normalized to *Renilla* photon counts. 1, reporter only (negative control); 2, reporter + Cas9 (positive control); 3, CMV promoter-driven AcrIIA4 with 2x miR-122 binding sites; 4, CMV promoter-driven AcrIIA4, no miR-122 binding sites ("scaffold"); 5, EFS promoter-driven AcrIIA4 with 2x miR-122 binding sites; 6, EFS promoter-driven AcrIIA4, no miR-122 binding sites ("scaffold"); 3-6 also all contain reporter + Cas9; n.s., not significant. Error bars indicate the standard error of the mean, 3 replicates.

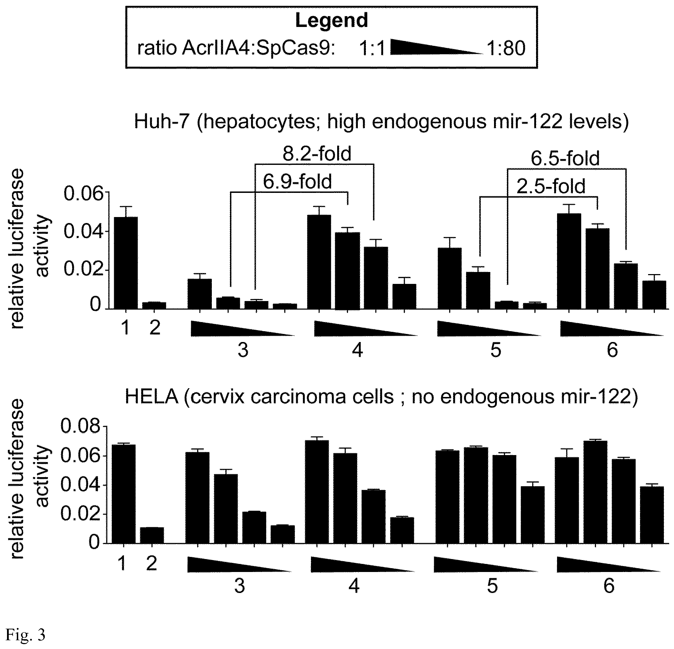

Fig. 3: Cas9 inhibition and release is dependent on the supplied Acr dose. Experiment was performed in Huh-7 cells and as in Figure 2B, but the ratio of AcrIIA4 to Cas9 construct during transfection was varied as indicated. 1, reporter only (negative control); 2, reporter + Cas9 (positive control); 3, CMV promoter-driven AcrIIA4 with 2x miR-122 binding sites; 4, CMV promoter-driven AcrIIA4, no miR-122 binding sites ("scaffold"); 5, EF1α promoter-driven AcrIIA4 with 2x miR-122 binding sites; 6, EF1α promoter-driven AcrIIA4, no miR-122 binding sites ("scaffold"); 3-6 also contain reporter + Cas9 (Figure 2A). Error bars indicate the standard error of the mean, 3 replicates.

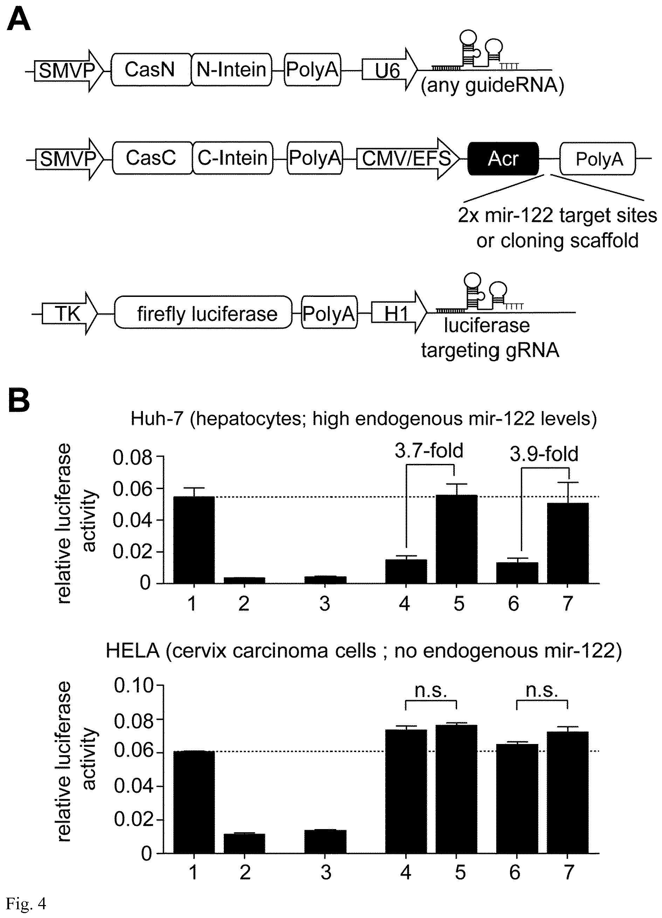

Fig. 4: Coupling of Cas9 and AcrIIA4 delivery results in tight, miR-dependent Cas regulation.
   (A) Experimental overview and schematics of used constructs. SpyCas9 was split into two separate fragments (CasN and CasC), fused to a split-intein (N-intein and C-intein, respectively). Co-delivery of both Cas-intein parts results in re-assembly of an active, full-length Cas9. AcrIIA4 is located on the CasC vector and either bears 2x miR-122 binding sites within its 3' UTR or not (scaffold). The luciferase reporter-encoding vector also carries a constitutive *Renilla* expression cassette for normalization purposes (not shown in schematic).
   (B) Huh-7 cells (high endogenous miR-122 levels) or HELA cells (control, no endogenous miR-122) were triple-transfected with the constructs in A in equimolar amounts. Forty-eight h post-transfection firefly and *Renilla* luciferase activity were measured by dual-luciferase assay. Firefly luciferase photon counts were normalized to *Renilla* photon counts. 1, reporter only (negative control); 2, reporter + full-length Cas9 (positive control); 3, reporter + Cas9N + Cas9C (without Acr cassette; positive control); 4-6 Cas9N + Cas9C (specified below) + reporter; 4, CMV promoter-driven AcrIIA4 carrying 2x miR-122 binding sites; 5, CMV promoter-driven AcrIIA4, no miR-122 binding sites ("scaffold"); 6, EFS promoter-driven AcrIIA4 with 2x miR-122 binding sites; 7, EFS promoter-driven AcrIIA4, no miR-122 binding sites ("scaffold"). n.s., not significant. The dashed line indicates reporter activity in absence of Cas9. Error bars indicate the standard error of the mean, 3 replicates.

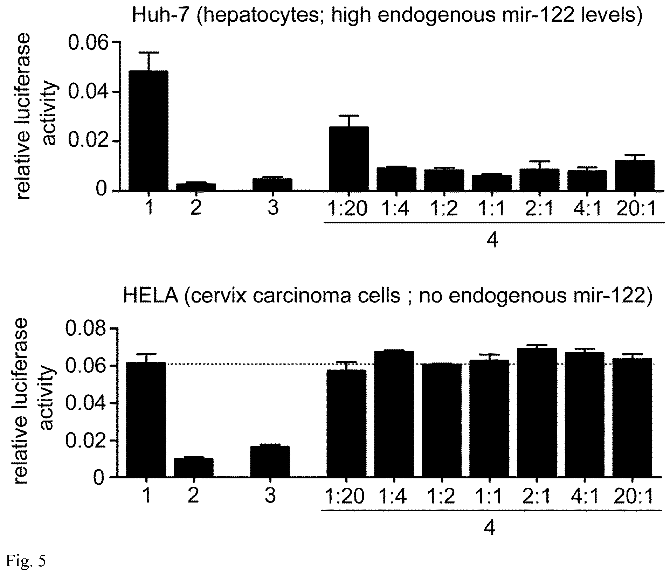

Fig. 5: Cas9 inhibition in off-target cells is independent of the CasN:CasC vector ratio. Huh-7 cells (high endogenous miR-122 levels) or HELA cells (control, no endogenous miR-122) were co-transfected with the CasN-N-intein and CasC-C-intein construct (bearing the CMV promoter-driven acr with 2x miR-122 target sites) as well as a corresponding luciferase cleavage reporter. Forty-eight h post-transfection firefly and *Renilla* luciferase activity were measured by dual-luciferase assay. Firefly luciferase photon counts were normalized to *Renilla* photon counts. 1, reporter only (negative control); 2, reporter + full-length Cas9 (positive control); 3, reporter + Cas9N + Cas9C (without Acr cassette; positive control); 4, Cas9N + Cas9C co-transfected at the indicated ratios. The dashed line indicates reporter activity in absence of Cas9. Error bars indicate the standard error of the mean, 3 replicates.

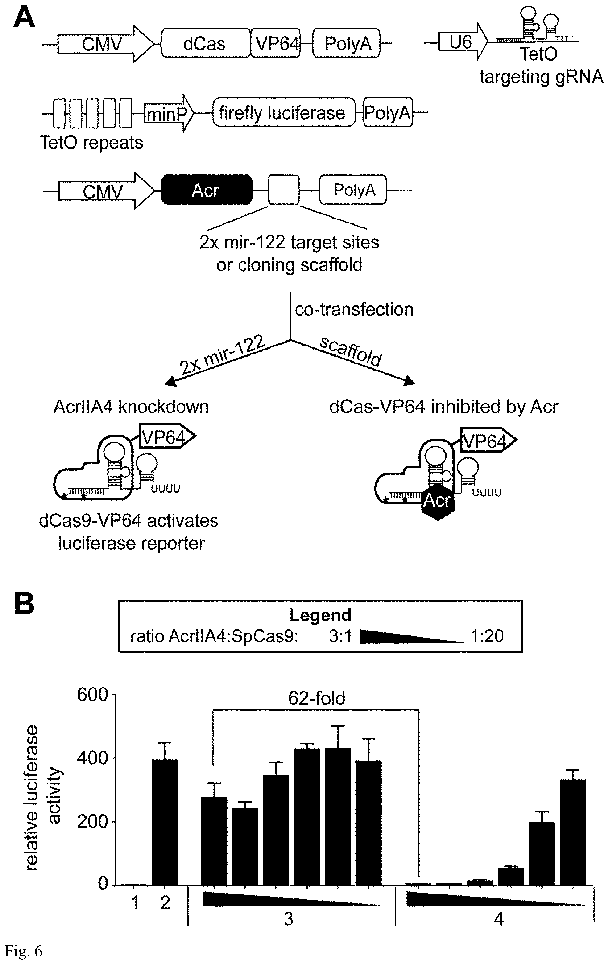

Fig. 6: MiR-122 dependent induction of gene expression by dCas9-VP64. (A) Experimental overview and schematics of used constructs. The AcrIIA4 vector either carries 2x miR-122 binding sites within its 3' UTR or not (as control). (B) Huh-7 cells (high endogenous miR-122 levels) were co-transfected with the constructs in A as well as a constitutive *Renilla* luciferase expression vector (not shown in the vector schematic in A). The ratio of transfected AcrIIA4 to Cas9 constructs is indicated. Forty-eight h post-transfection firefly and *Renilla* luciferase activity were measured by dual-luciferase assay. Firefly luciferase photon counts were normalized to *Renilla* photon counts. 1, reporter only (negative control); 2, reporter + dCas9-VP64 (positive control); 3, CMV promoter-driven AcrIIA4 with 2x miR-122 binding sites; 4, CMV promoter-driven AcrIIA4, no miR-122 binding sites ("scaffold"); 3-4 also contain reporter + dCas9-VP64; error bars indicate the standard error of the mean, 3 replicates.

The following Examples shall merely illustrate the invention. They shall not be construed, whatsoever, to limit the scope of the invention.

### Example 1: Methods

### 1.1 Plasmids

pAAV-SMVP-Cas9N (Addgene #80930) and pAAV-SMVP-Cas9C (Addgene #80931) were gifts from George Church. Further vectors used herein have SEQ ID NOs: 3 to 19 (cf. Nihongaki et al. (2015), Chem Biol. 22(2):169-74; Konerman et al. (2015), Nature 517(7536):583-8).

### 1.2 Cell Culture

Human cervix adenocarcinoma cells (HELA) and the human liver carcinoma cells (Huh-7) were maintained at 37 °C and 5 % CO₂ in a humidified tissue culture incubator. Cells were cultured in 75 cm² flasks (STARLAB) (Table 1) and passaged every 2 to 3 days.

**Table 1: Media Compositions**

| **Cell line** | **Media Composition** |
|---|---|
| HELA | 1x DMEM (Gibco) with 4,500 mg/L-glucose, 10 % FBS, 0.01 % PS (Gibco), 2 mM L-glutamine (Gibco) |
| Huh-7 | 1x DMEM (Gibco) with 4,500 mg/L-glucose, 10 % FBS, 0.01 % PS (Gibco), 2 mM L-glutamine (Gibco), 1 mM non-essential amino acids (Gibco) |

### 1.3 Cell seeding and transient transfection

For experiments, cells were seeded into transparent, tissue culture-treated 96-well plates (STARLAB) (6,000 cells per well and in 100 µl media). Transfection was performed 12 to 16 hours after seeding. Lipofectamine™ 3000 (Invitrogen) was used for all transfections according to the manufacturer's protocol.

For experiments involving the catalytically active Cas9, 20 ng Cas9 expression vector and 20 ng luciferase cleavage reporter were co-transfected (amounts are per well). AcrIIA4 expression vectors with or without miR-122 target sites were added in a ratio AcrIIA4:Cas9 = 1:1 to 1:80 during transfection as indicated in the figure legends.

For experiments involving the Split Cas9 constructs, 20 ng of each, the luciferase cleavage reporter, the CasN-N-intein and the CasC-C-intein constructs were co-transfected, if not indicated otherwise in the figure legend.

For experiments involving dCas9-VP64, 20 ng of each, the dCas9-VP64 vector, the TetO guideRNA vector as well as the TetO-dependent firefly luciferase reporter were co-transfected alongside 1 ng of pRL-TK (Promega; encodes *Renilla* luciferase). AcrIIA4 expression vectors with or without miR-122 target sites were added in a ratio AcrIIA4:dCas9-VP64 = 3:1 to 1:20 during transfection as indicated in the figure legend.

The total amount of DNA transfected per well was 100 ng or, in case of dCas9-VP64 experiments, 101 ng. DNA was topped up with inert stuffer DNA if required. Six hours post-transfection the medium was exchanged.

### 1.4 Luciferase Assay

Forty-eight hours post-transfection firefly and *Renilla* luciferase activity was determined using the Dual Luciferase® Reporter Assay System (Promega) according to the manufacturer's recommendations. In brief, media was aspirated and cells were washed with 100 µl PBS. 30 µl of the supplied lysis buffer was added to each well and plates were shaken for 30 minutes at 450 rpm and at room temperature. 10 µl of lysate was then transferred to a white 96-well plate (greiner bio-one) and luminescence was measured using a GloMax® 96 Microplate Luminometer (Promega) equipped with automated injectors. First, the firefly luciferase signal was measured by adding 35 µl Luciferase Assay Reagent II. Second, the reaction was quenched, and the *Renilla* luciferase reaction was simultaneously initiated by adding 35 µl of the Stop & Glo® reagent. Signal integration time was 10 s preceded by a 2 s delay. For data analysis, firefly values were divided by *Renilla* values. Data are means plus/minus standard error of the mean, 3 experimental replicates.

### Example 2: Results

We generated a set of modular AcrIIA4 expression constructs with variable AcrIIA4-driving promoters (cytomegalovirus promoter, CMV; elongation factor alpha promoter, EF-1α; EF-1α shortened promoter, EFS) as well as a modular 3' UTR for simple insertion of any miRNA target site(s) (Figure 1A). We then introduced a concatemer of two target sites for miR-122 into our AcrIIA4 constructs, a miRNA specifically expressed in hepatocytes, but not in any other cell type (Figure 1B).

When co-delivered into hepatocytes together with SpyCas9 and a corresponding gRNA, one would expect a potent knockdown of AcrIIA4 and hence a release in Cas9 activity (Figure 1A). In any other cell type, i.e. in the absence of miR-122, AcrIIA4 should remain highly expressed and therefore efficiently repress Cas9 activity. To test our hypothesis, we co-transfected Huh-7, a hepatocellular carcinoma cell line expressing high amounts of miR-122, as well as HELA cells (cervix carcinoma cell line not expressing miR-122, as control) with different AcrIIA4-encoding vectors, an SpyCas9 expression vector, and a luciferase-based cleavage reporter to monitor Cas9 catalytic activity (Figure 2A).

The AcrIIA4-encoding vectors differed in the promoter driving AcrIIA4 expression (CMV or EFS), as well as the presence or absence of two miR-122 binding sites within the AcrIIA4 gene 3' UTR. As expected, AcrIIA4 vectors not bearing miR-122 binding sites potently inhibited Cas9 activity in both, Huh-7 as well as HELA cells (Figure 2B). In contrast, for the AcrIIA4 constructs carrying the miR-122 target sites, we observed a strong release in Cas9 activity (i.e. potent luciferase knockdown) exclusively in Huh-7, but not in HELA cells, indicating successful, miR-122-dependent activation of Cas9 (Figure 2B).

Next, we repeated the experiment in Huh-7 cells, this time titrating the amount of AcrIIA4 and thereby altering the ratio of AcrIIA4:Cas9 constructs transfected between 1:1 and 1:80. As AcrIIA4-driving promoters, we used either CMV or EF1α (non-shortened). Cas9 inhibition by AcrIIA4 was dose-dependent (Figure 3). At intermediate Acr:Cas ratios, a potent release in Cas9 activity (up to 8-fold increase in reporter knockdown) was observed exclusively in Huh-7 and in presence of the miR-122 binding sites within the acr 3' UTR. In contrast, a very high AcrIIA4:Cas9 transfection ratio resulted in efficient Cas9 inhibition, even in the presence of the miR-122 binding sites in the acr gene, probably due to a saturation of endogenous RNAi components (e.g. Argonaute-2 (Ago-2), the catalytic subunit of the RNA-induced silencing complex (RISC)) or sequestration of the majority of endogenous miR-122. On the other hand, a low AcrIIA4:Cas9 ratio led to noticeable Cas9 activity even in the absence of the miR-122 binding sites on the AcrIIA4 vector. This is expected, as AcrIIA4 acts as a competitive inhibitor and therefore needs to be provided in excess as compared to its Cas9 target to efficiently function (Dong et al., 2017; Shin et al., 2017; Yang and Patel, 2017).

In the aforementioned experiments Acr and Cas9 expression cassettes were located on different vectors (Figure 2A). This may be suboptimal, as the Acr:Cas9 vector ratio upon delivery and hence the corresponding Acr:Cas9 expression ratios within the target cells will likely strongly vary. This may result in an undesired leakiness due to a very low Acr:Cas9 ratio occurring with a certain probability (refer to Figure 3). To address this issue, we devised a strategy directly coupling Cas9 activity to the amount of *acr* transgene present. Our strategy grounds on a SpyCas9-AAV vector design reported by the group of George Church at Harvard University (Chew et al., 2016). In their design, the SpyCas9 was split into two separate pieces (CasN and CasC), fused to *Rhodothermus marinus* derived split-intein sequences. The CasN-N-Intein and CasC-C-Intein constructs are completely inactive by themselves. However, upon co-delivery, intein-mediated protein trans-splicing results in reconstitution of an active, full-length Cas9.

We adopted this design and inserted a U6-promoter driven guide-RNA expression cassette into the reported CasN-N-Intein AAV vector (Addgene #80930) and an AcrIIA4 expression cassette (with or without 2x miR-122 binding sites) into the corresponding CasC-C-Intein AAV vector (Addgene #80931) (Figure 4A). Efficient re-assembly of SpyCas9 should now only be possible, if a cell receives sufficient amounts of both the CasN as well as the Acr-encoding CasC vectors, thereby tightly linking Cas9 activity to AcrIIA4-mediated Cas regulation. To validate our strategy, we again co-transfected Huh-7 or HELA cells with these split-SpyCas9 vectors alongside our firefly luciferase cleavage reporter indicating Cas9 catalytic activity. As expected, we observed a potent, miR-122 dependent rescue in Cas9 catalytic activity (Figure 4B). Importantly, no considerable leakiness (Cas9 activity) was observed in absence of the miR-122 binding sites, suggesting tight regulation of Cas9 by Acr. Next, we repeated this experiment, this time titrating the CasN-N-Intein and CasC-C-Intein vector co-encoding the CMV promoter-driven acr with 2x miR-122 target site against each other in ratios of 1:20 - 20:1. We observed a potent luciferase reporter knockdown, i.e. strong miR-122 dependent rescue in Cas9 catalytic activity, for CasN:CasC ratios between 1:4 and 4:1 (Figure 5). Higher or lower CasN:Cas9 ratios resulted in noticeably reduced Cas9 activity, likely due to the low expression of one out of the two required split Cas9 fragments. Remarkably, in HELA cells, Cas9 remained inactive for all tested CasN:CasC ratios, once again indicating successful coupling of AcrIIA4 expression and Cas9 re-assembly in off-target cells (Figure 5).

It has been shown that AcrIIA4 efficiently inhibits a catalytically dead (d)SpyCas9 in bacterial cells (Rauch et al., 2017), suggesting that AcrIIA4 could be used for cell type- or tissue-specific control of virtually any dCas9-based tool, e.g. dCas9-based transcriptional regulators or epigenetic modifiers (Maeder et al., 2013; Hilton et al., 2015). Concurrently, by using endogenous miRNA signatures for controlling AcrIIA4 expression, one might be able to activate any dCas9-based tool in a cell type- or tissue-specific manner. To validate this idea, we again titrated the amount of AcrIIA4 vector (with 2x miR-122 binding sites or empty scaffold) co-transfected with a dCas9-VP64 transactivator, a tetracycline (tet) operator targeting gRNA expression construct, as well as a corresponding firefly luciferase reporter driven from a minimal promoter preceded by tet operator repeats (Figure 6A).

We observed pronounced luciferase reporter activation only when using dCas9-VP64 in combination with the miR-122 target site-bearing AcrIIA4 construct, but not when using the scaffold AcrIIA4 control vector, indicating an efficient miR-122-mediated rescue of dCas9-VP64 reporter transactivation (Figure 6B). Inhibition in the absence of miR-122 target sites and release of dCas9-VP64 activity in the presence of the miRNA target sites was again dose-dependent. At very low AcrIIA4:dCas9-VP64 transfection ratios the luciferase reporter was active independent of the presence or absence of miR-122 traget sites in the acr 3' UTR. At high AcrIIA4:dCas9-VP64 ratios, the reporter activation upon miR-122-mediated AcrIIA4 knockdown was still potent, but incomplete. This is in line with our experiments employing the catalytically active Cas9 (compare Figure 3 and 6B), and, once again, shows that the AcrIIA4:Cas9 expression ratio is a critical parameter to consider when employing our CasON approach.

### Cited Literature

Amendola et al. (2013). Mol Ther 21, 934-946.
Babak et al. (2004). RNA 10, 1813.
Baker et al. (2017). JASN, doi: 10.1681/ASN.2016121280.
Barrangou et al. (2007). Science 315, 1709-1712.
Bhaya et al. (2011). Annu Rev Genet 45, 273-297.
Chew et al. (2016). Nat Methods 13, 868-874.
Cho et al. (2014). Genome Res 24, 132-141.
Cong et al. (2013). Science 339, 819-823.
Dai et al. (2016). Mol Ther Nucleic Acids 5, e349.
Dong et al. (2017). Nature 546, 436-439.
Elbashir (2001), Nature 411, 494-498.
Fu et al. (2013). Nat Biotechnol 31, 822-826.
Geisler and Fechner (2016). World J Exp Med 6, 37-54.
Guo et al. (2014). Scientific Reports 4, doi:10.1038/srep05150.
Harris et al. (2010). Biomaterials 31(5), 998.
Hemphill et al. (2015). J Am Chem Soc 137, 5642-5645.
Hilton et al. (2015). Nat Biotechnol 33, 510-517.
Hirosawa et al. (2017). Nucleic Acids Res. 13, e118.
Hsu et al. (2013). Nat Biotechnol 31, 827-832.
Jinek et al. (2012). Science 337, 816-821.
Jinek et al. (2013). Elife 2, e00471.
Jurkowski et al. (2015). Clin Epigenetics 7(1), 18.
Kay (2011). Nat Rev Genet 12, 316-328.
Kiani et al. (2014). Nat Methods 11, 723-726.
Kleinstiver et al. (2016). Nature 529, 490.
Konerman et al. (2015). Nature 517, 583-588.
Landgraf et al. (2007). Cell 129, 1401.
Liu et al. (2011). J Biomed Sci Eng 4(19), 666.
Ludwig et al. (2016). NAR 44(8), 3865.
Maeder et al. (2013). Nat Methods 10, 977-979.
Maji et al. (2017). Nat Chem Biol 13, 9-11.
Mali et al. (2013). Science 339, 823-826.
Nihongaki et al. (2015a). Nat Biotechnol 33, 755-760.
Nihongaki et al. (2015b). Chemistry & Biology 22, 169-174.
Pawluk et al. (2016). Cell 167, 1829.
Petris et al. (2017). Nat Communications 8, 15334.
Platt et al. (2014). Cell 159, 440-455.
Polstein and Gersbach (2015). Nat Chem Biol 11, 198-200.
Ran et al. (2015). Nature 520, 186-191.
Rauch et al. (2017). Cell 168, 150-158 e110.
Schmidt and Grimm (2015). Biotechnol J 10, 258-272.
Senis et al. (2014). Biotechnol J 9, 1402-1412.
Shin et al. (2017). Sci Adv 3, e1701620.
Slaymaker et al. (2016). Science 351, 84-88.
Terns and Terns (2011). Curr Opin Microbiol 14, 321-327.
Truong et al. (2015). Nucleic Acids Res 43, 6450-6458.
Wang et al. (2013). Cell 153, 910-918.
Wang et al. (2015). Nat Biotechnol 33, 175-178.
Wiedenheft et al. (2012). Nature 482, 331-338.
WO 0129058.
WO 09932619.
Xie et al. (2011). Science 333, 1307-1311.
Xue et al. (2016). Gene Ther 23, 557-559.
Yang and Patel (2017). Mol Cell 67, 117-127
Yin et al. (2014). Nat Biotechnol 32, 551-553.
Zetsche et al. (2015a). Cell 163, 759-771.
Zetsche et al. (2017). Nat Biotechnol 35, 31-34.
Zetsche et al. (2015b). Nat Biotechnol 33, 139-142.
Zhang et al. (2015). Mol Ther Nucleic Acids 4, e264.

## Claims

1. A polynucleotide comprising (i) a sequence encoding an anti-CRISPR (Acr) polypeptide and (ii) a miRNA target sequence.

2. The polynucleotide of claim 1, wherein said miRNA is a tissue-specific and/or cell type-specific miRNA.

3. The polynucleotide of claim 1 or 2, wherein said polynucleotide comprises at least two miRNA target sequences essentially complementary to the same miRNA, more preferably at least two target sequences being essentially complementary to the same nucleic acid sequence of a miRNA.

4. The polynucleotide of any one of claims 1 to 3, wherein said polynucleotide comprises at least two non-identical miRNA target sequences, more preferably at least two miRNA target sequences being less than 80%, even more preferably less than 70%, most preferably less than 60% identical to each other.

5. The polynucleotide of any one of claims 1 to 4, wherein said sequence encoding an Acr polypeptide is located upstream of said miRNA target sequence, preferably of all miRNA target sequences comprised in said expressible nucleic acid sequence

6. The polynucleotide of any one of claims 1 to 5, wherein said Acr polypeptide is an AcrIIA4 polypeptide.

7. The polynucleotide of any one of claims 1 to 6, wherein said sequence encoding an Acr polypeptide comprises
a) the nucleic acid sequence of SEQ ID NO:21;
b) a nucleic acid sequence being at least 70% identical to the sequence of SEQ ID NO:21;
c) a nucleic acid sequence encoding a polypeptide comprising the amino acid sequence of SEQ ID NO:20; or
d) a nucleic acid sequence encoding a polypeptide comprising an amino acid sequence being at least 70% identical to the sequence of SEQ ID NO:20.

8. The polynucleotide of any one of claims 1 to 7, wherein said polynucleotide further comprises a nucleic acid sequence encoding at least a fragment of a CRISPR-associated (Cas) nuclease.

9. A vector comprising the polynucleotide according to any one of claims 1 to 8.

10. A host cell comprising the polynucleotide according to any one of claims 1 to 8 and/or the vector according to claim 9.

11. A polynucleotide according to any one of claims 1 to 8 and/or the vector according to claim 9 and/or the host cell according to claim 10 for use in medicine, preferably for use in treatment of genetic disease, neurodegenerative disease, cancer, and/or infectious disease.

12. A kit comprising
(I) a first polynucleotide comprising (i) a sequence encoding an anti-CRISPR (Acr) polypeptide and (ii) an RNA-interference (RNAi) target sequence; and a Cas nuclease or a second polynucleotide comprising an expressible gene encoding a Cas nuclease;
(II) a first polynucleotide comprising (i) a sequence encoding an anti-CRISPR (Acr) polypeptide and (ii) an RNA-interference (RNAi) target sequence, said first polynucleotide further comprising an expressible gene encoding a first fragment of a Cas nuclease, and a second polynucleotide comprising an expressible gene encoding a second fragment of a Cas nuclease, wherein said first and second fragment of said Cas nuclease together reconstitute an active Cas nuclease; or
(III) a first polynucleotide comprising (i) a sequence encoding an anti-CRISPR (Acr) polypeptide, (ii) an RNA-interference (RNAi) target sequence, and (iii) a sequence encoding a Cas nuclease; and a second polynucleotide comprising an expressible gene encoding an inhibitory RNA, preferably a siRNA or shRNA or a miRNA hybridizing to said RNAi target sequence, more preferably a siRNA hybridizing to said RNAi target sequence.

13. The kit of claim 12, wherein said kit comprises a polynucleotide encoding at least one guide RNA (gRNA), preferably wherein said polynucleotide encoding at least one gRNA is the first or the second polynucleotide, more preferably is the second polynucleotide.

14. A method for genetically modifying a host cell comprising
a1) contacting said host cell with a polynucleotide according to any one of claims 1 to 8; with the vector according to claim 9; and/or with the host cell according to claim 10; and further contacting said host cell with a Cas nuclease activity and a gRNA;
or
a2) contacting said host cell with the components comprised in a kit according to claim 12 or 13;
and,
b) thereby, genetically modifying said host cell.

15. Use of the polynucleotide according to any one of claims 1 to 8; the vector according to claim 9; the host cell according to claim 10; and/or the kit according to claim 12 or 13; for genetically modifying a host cell.


SEQUENCE LISTING

<110>  Universität Heidelberg
       Deutsches Krebsforschungszentrum

<120>  Use of anti-CRISPR polypeptides for specific activation of Cas
       nucleases

<130>  UH14769EP

<160>  48

<170>  PatentIn version 3.3

<210>  1
<211>  72
<212>  DNA
<213>  Artificial

<220>
<223>  cloning scafold in Acr mRNA, partial sequence in Fig. 1B,

<400>  1
tgcagaatta gagacgccgt tgatgttgaa gtttatatac cgttgatgtt gaagtcgtct      60

ctagcttaag tt                                                          72


<210>  2
<211>  72
<212>  DNA
<213>  Artificial

<220>
<223>  Acr mRNA, partial sequence with miR122 target sequences, Fig. 1 B

<400>  2
tgcagaatta caaacaccat tgtcacactc cattatatac aaacaccatt gtcacactcc      60

atagcttaag tt                                                          72


<210>  3
<211>  7601
<212>  DNA
<213>  Artificial

<220>
<223>  Cas9 Luciferase Cleavage Reporter Plasmid

<400>  3
ctaaattgta agcgttaata ttttgttaaa attcgcgtta aatttttgtt aaatcagctc      60

attttttaac caataggccg aaatcggcaa aatcccttat aaatcaaaag aatagaccga      120

gatagggttg agtgttgttc cagtttggaa caagagtcca ctattaaaga acgtggactc     180

caacgtcaaa gggcgaaaaa ccgtctatca gggcgatggc ccactacgtg aaccatcacc     240

ctaatcaagt tttttggggt cgaggtgccg taaagcacta aatcggaacc ctaaagggag     300

cccccgattt agagcttgac ggggaaagcc ggcgaacgtg gcgagaaagg aagggaagaa     360

agcgaaagga gcgggcgcta gggcgctggc aagtgtagcg gtcacgctgc gcgtaaccac 420
cacacccgcc gcgcttaatg cgccgctaca gggcgcgtcc cattcgccat tcaggctgcg 480
caactgttgg gaagggcgat cggtgcgggc ctcttcgcta ttacgccagc tggccactcc 540
ctctctgcgc gctcgctcgc tcactgaggc cgggcgacca aaggtcgccc gacgcccggg 600
ctttgcccgg gcggcctcag tgagcgagcg agcgcgcaga gagggagtgg ccaactccat 660
cactaggggt tccttgtagt taatgattaa cccgccatgc tacttatcta cgtagccatg 720
ctctagagca agatctgcgc agcaccatgg cctgaaataa cctctgaaag aggaacttgg 780
ttaggtacct tctgaggcgg aaagaaccag ctgtggaatg tgtgtcagtt agggtgtgga 840
aagtccccag gctccccagc aggcagaagt atgcaaagca tgcatctcaa ttagtcagca 900
accaggtgtg gaaagtcccc aggctcccca gcaggcagaa gtatgcaaag catgcatctc 960
aattagtcag caaccatagt cccgccccta actccgccca tcccgcccct aactccgccc 1020
agttccgccc attctccgcc ccatggctga ctaatttttt ttatttatgc agaggccgag 1080
gccgcctcgg cctctgagct attccagaag tagtgaggag gcttttttgg aggcctaggc 1140
ttttgcaaaa agcttgattc ttctgacaca acagtctcga acttaagctg cagaagttgg 1200
tcgtgaggca ctgggcaggt aagtatcaag gttacaagac aggtttaagg agaccaatag 1260
aaactgggct tgtcgagaca gagaagactc ttgcgtttct gataggcacc tattggtctt 1320
actgacatcc actttgcctt tctctccaca ggtgtccact cccagttcaa ttacagctct 1380
taaggctaga gtacttaata cgactcacta taggctagcc accatggctt ccaaggtgta 1440
cgaccccgag caacgcaaac gcatgatcac tgggcctcag tggtgggctc gctgcaagca 1500
aatgaacgtg ctggactcct tcatcaacta ctatgattcc gagaagcacg ccgagaacgc 1560
cgtgattttt ctgcatggta acgctgcctc cagctacctg tggaggcacg tcgtgcctca 1620
catcgagccc gtggctagat gcatcatccc tgatctgatc ggaatgggta agtccggcaa 1680
gagcgggaat ggctcatatc gcctcctgga tcactacaag tacctcaccg cttggttcga 1740
gctgctgaac cttccaaaga aaatcatctt tgtgggccac gactgggggg cttgtctggc 1800
ctttcactac tcctacgagc accaagacaa gatcaaggcc atcgtccatg ctgagagtgt 1860
cgtggacgtg atcgagtcct gggacgagtg gcctgacatc gaggaggata tcgccctgat 1920
caagagcgaa gagggcgaga aaatggtgct tgagaataac ttcttcgtcg agaccatgct 1980
cccaagcaag atcatgcgga aactggagcc tgaggagttc gctgcctacc tggagccatt 2040
caaggagaag ggcgaggtta gacggcctac cctctcctgg cctcgcgaga tccctctcgt 2100
taagggaggc aagcccgacg tcgtccagat tgtccgcaac tacaacgcct accttcgggc 2160
cagcgacgat ctgcctaaga tgttcatcga gtccgaccct gggttctttt ccaacgctat 2220
tgtcgaggga gctaagaagt tccctaacac cgagttcgtg aaggtgaagg gcctccactt 2280

cagccaggag gacgctccag atgaaatggg taagtacatc aagagcttcg tggagcgcgt 2340

gctgaagaac gagcagtaat tctaggcgat cgctcgagcc cgggaattcg tttaaaccta 2400

gagcggccgc tggccgcaat aaaatatctt tattttcatt acatctgtgt gttggttttt 2460

tgtgtgagga tctaaatgag tcttcggacc tcgcgggggc cgcttaagcg gtggttaggg 2520

tttgtctgac gcggggggag ggggaaggaa cgaaacactc tcattcggag gcggctcggg 2580

gtttggtctt ggtggccacg ggcacgcaga agagcgccgc gatcctctta agcacccccc 2640

cgccctccgt ggaggcgggg gtttggtcgg cgggtggtaa ctggcgggcc gctgactcgg 2700

gcgggtcgcg cgccccagag tgtgaccttt tcggtctgct cgcagacccc cgggcggcgc 2760

cgccgcggcg gcgacgggct cgctgggtcc taggctccat ggggaccgta tacgtggaca 2820

ggctctggag catccgcacg actgcggtga tattaccgga gaccttctgc gggacgagcc 2880

gggtcacgcg gctgacgcgg agcgtccgtt gggcgacaaa caccaggacg gggcacaggt 2940

acactatctt gtcacccgga ggcgcgaggg actgcaggag cttcagggag tggcgcagct 3000

gcttcatccc cgtggcccgt tgctcgcgtt tgctggcggt gtccccggaa gaaatatatt 3060

tgcatgtctt tagttctatg atgacacaaa ccccgcccag cgtcttgtca ttggcgaatt 3120

cgaacacgca gatgcagtcg gggcggcgcg gtcccaggtc cacttcgcat attaaggtga 3180

cgcgtgtggc ctcgaacacc gagcgaccct gcagcgaccc gcttaaaagc ttggcattcc 3240

ggtactgttg gtaaagccac catggccgat gctaagaaca ttaagaaggg ccctgctccc 3300

ttctaccctc tggaggatgg caccgctggc gagcagctgc acaaggccat gaagaggtat 3360

gccctggtgc ctggcaccat tgccttcacc gatgcccaca ttgaggtgga catcacctat 3420

gccgagtact tcgagatgtc tgtgcgcctg gccgaggcca tgaagaggta cggcctgaac 3480

accaaccacc gcatcgtggt gtgctctgag aactctctgc agttcttcat gccagtgctg 3540

ggcgccctgt tcatcggagt ggccgtggcc cctgctaacg acatttacaa cgagcgcgag 3600

ctgctgaaca gcatgggcat ttctcagcct accgtggtgt tcgtgtctaa gaagggcctg 3660

cagaagatcc tgaacgtgca gaagaagctg cctatcatcc agaagatcat catcatggac 3720

tctaagaccg actaccaggg cttccagagc atgtacacat tcgtgacatc tcatctgcct 3780

cctggcttca acgagtacga cttcgtgcca gagtctttcg acagggacaa aaccattgcc 3840

ctgatcatga acagctctgg gtctaccggc ctgcctaagg gcgtggccct gcctcatcgc 3900

accgcctgtg tgcgcttctc tcacgcccgc gaccctattt tcggcaacca gatcatcccc 3960

gacaccgcta ttctgagcgt ggtgccattc caccacggct tcggcatgtt caccaccctg 4020

ggctacctga tttgcggctt tcgggtggtg ctgatgtacc gcttcgagga ggagctgttc 4080

ctgcgcagcc tgcaagacta caaaattcag tctgccctgc tggtgccaac cctgttcagc 4140

ttcttcgcta agagcaccct gatcgacaag tacgacctgt ctaacctgca cgagattgcc 4200

tctggcggcg ccccactgtc taaggaggtg ggcgaagccg tggccaagcg ctttcatctg 4260

ccaggcatcc gccagggcta cggcctgacc gagacaacca gcgccattct gattacccca 4320

gagggcgacg acaagcctgg cgccgtgggc aaggtggtgc cattcttcga ggccaaggtg 4380

gtggacctgg acaccggcaa gaccctggga gtgaaccagc gcggcgagct gtgtgtgcgc 4440

ggccctatga ttatgtccgg ctacgtgaat aaccctgagg ccacaaacgc cctgatcgac 4500

aaggacggct ggctgcactc tggcgacatt gcctactggg acgaggacga gcacttcttc 4560

atcgtggacc gcctgaagtc tctgatcaag tacaagggct accaggtggc cccagccgag 4620

ctggagtcta tcctgctgca gcaccctaac attttcgacg ccggagtggc cggcctgccc 4680

gacgacgatg ccggcgagct gcctgccgcc gtcgtcgtgc tggaacacgg caagaccatg 4740

accgagaagg agatcgtgga ctatgtggcc agccaggtga caaccgccaa gaagctgcgc 4800

ggcggagtgg tgttcgtgga cgaggtgccc aagggcctga ccggcaagct ggacgcccgc 4860

aagatccgcg agatcctgat caaggctaag aaaggcggca agatcgccgt gtaataattc 4920

tagagtcggg gcggccggcc gcttcgagca gacatgataa gatacattga tgagtttgga 4980

caaaccacaa ctagaatgca gtgaaaaaaa tgctttattt gtgaaatttg tgatgctatt 5040

gctttatttg taaccattat aagctgcaat aaacaagtta acaacaacaa ttgcattcat 5100

tttatgtttc aggttcaggg ggaggtgtgg gaggtttttt aaagcaagta aaacctctac 5160

aaatgtggta aaatcgatac tagtgcaaaa aaagcaccga ctcggtgcca ctttttcaag 5220

ttgataacgg actagcctta ttttaacttg ctatgctgtt tccagcatag ctcttaaacg 5280

gtagtcggtc ttagagtccg gtgtaccgtg gtctcataca gaacttataa gattcccaaa 5340

tccaaagaca tttcacgttt atggtgattt cccagaacac atagcgacat gcaaatatgg 5400

gcatcgataa actagttgct ctagagcatg gctacgtaga taagtagcat ggcgggttaa 5460

tcattaacta caaggaaccc ctagtgatgg agttggccac tccctctctg cgcgctcgct 5520

cgctcactga ggccgggcga ccaaaggtcg cccgacgccc gggctttgcc cgggcggcct 5580

cagtgagcga gcgagcgcgc agagagggag tggccagctg cattaatgaa tcggccaacg 5640

cgcggggaga ggcggtttgc gtattgggcg ctcttccgct tcctcgctca ctgactcgct 5700

gcgctcggtc gttcggctgc ggcgagcggt atcagctcac tcaaaggcgg taatacggtt 5760

atccacagaa tcaggggata acgcaggaaa gaacatgtga gcaaaaggcc agcaaaaggc 5820

caggaaccgt aaaaaggccg cgttgctggc gtttttccat aggctccgcc cccctgacga 5880

gcatcacaaa aatcgacgct caagtcagag gtggcgaaac ccgacaggac tataaagata 5940

ccaggcgttt ccccctggaa gctccctcgt gcgctctcct gttccgaccc tgccgcttac 6000

cggatacctg tccgcctttc tcccttcggg aagcgtggcg ctttctcata gctcacgctg 6060

taggtatctc agttcggtgt aggtcgttcg ctccaagctg ggctgtgtgc acgaaccccc 6120

cgttcagccc gaccgctgcg ccttatccgg taactatcgt cttgagtcca acccggtaag 6180

acacgactta tcgccactgg cagcagccac tggtaacagg attagcagag cgaggtatgt 6240

aggcggtgct acagagttct tgaagtggtg gcctaactac ggctacacta gaaggacagt 6300

atttggtatc tgcgctctgc tgaagccagt taccttcgga aaaagagttg gtagctcttg 6360

atccggcaaa caaaccaccg ctggtagcgg tggttttttt gtttgcaagc agcagattac 6420

gcgcagaaaa aaaggatctc aagaagatcc tttgatcttt tctacggggt ctgacgctca 6480

gtggaacgaa aactcacgtt aagggatttt ggtcatgaga ttatcaaaaa ggatcttcac 6540

ctagatcctt ttaaattaaa aatgaagttt taaatcaatc taaagtatat atgagtaaac 6600

ttggtctgac agttaccaat gcttaatcag tgaggcacct atctcagcga tctgtctatt 6660

tcgttcatcc atagttgcct gactccccgt cgtgtagata actacgatac gggagggctt 6720

accatctggc cccagtgctg caatgatacc gcgagaccca cgctcaccgg ctccagattt 6780

atcagcaata aaccagccag ccggaagggc cgagcgcaga agtggtcctg caactttatc 6840

cgcctccatc cagtctatta attgttgccg ggaagctaga gtaagtagtt cgccagttaa 6900

tagtttgcgc aacgttgttg ccattgctac aggcatcgtg gtgtcacgct cgtcgtttgg 6960

tatggcttca ttcagctccg gttcccaacg atcaaggcga gttacatgat cccccatgtt 7020

gtgcaaaaaa gcggttagct ccttcggtcc tccgatcgtt gtcagaagta agttggccgc 7080

agtgttatca ctcatggtta tggcagcact gcataattct cttactgtca tgccatccgt 7140

aagatgcttt tctgtgactg gtgagtactc aaccaagtca ttctgagaat agtgtatgcg 7200

gcgaccgagt tgctcttgcc cggcgtcaat acgggataat accgcgccac atagcagaac 7260

tttaaaagtg ctcatcattg gaaaacgttc ttcggggcga aaactctcaa ggatcttacc 7320

gctgttgaga tccagttcga tgtaacccac tcgtgcaccc aactgatctt cagcatcttt 7380

tactttcacc agcgtttctg ggtgagcaaa aacaggaagg caaaatgccg caaaaaaggg 7440

aataagggcg acacggaaat gttgaatact catactcttc ctttttcaat attattgaag 7500

catttatcag ggttattgtc tcatgagcgg atacatattt gaatgtattt agaaaaataa 7560

acaaataggg gttccgcgca catttccccg aaaagtgcca c 7601

<210> 4
<211> 4848
<212> DNA
<213> Artificial

<220>
<223> dCas9-VP64 transactivation luciferase reporter plasmid

<400> 4

gacgatatcc ctcgatcgag tttaccactc cctatcagtg atagagaaaa gtgaaagtcg 60

agtttaccac tccctatcag tgatagagaa aagtgaaagt cgagtttacc actccctatc 120

agtgatagag aaaagtgaaa gtcgagttta ccactcccta tcagtgatag agaaaagtga 180

aagtcgagtt taccactccc tatcagtgat agagaaaagt gaaagtcgag tttaccactc 240

cctatcagtg atagagaaaa gtgaaagtcg agtttaccac tccctatcag tgatagagaa 300

aagtgaaagt cgagtttacc actccctatc agtgatagag aaaagtgaaa gtcgagttta 360

ccactcccta tcagtgatag agaaaagtga aagtcgagtt taccactccc tatcagtgat 420

agagaaaagt gaaagtcgag tttaccactc cctatcagtg atagagaaaa gtgaaagtcg 480

agtttaccac tccctatcag tgatagagaa aagtgaaagt cgagtttacc actccctatc 540

agtgatagag aaaagtgaaa gtcgagctcg gtacgctatg gcatgcatgt gtcgcgacct 600

gcaggccctg aagttcatct gcaccaccgg caagctgccc gtgccctggc ccaccctcgt 660

gaccaccctg acctggggcg tgcagtgctt cgcccgctac cccgaccaca tgaagcagca 720

cgacttcttc aagtccgcca tgcccgaagg ctacgtccag gagcgcacca tcttcttcaa 780

ggacgacggc aactacaaga cccgcgccga ggtgaagttc gagggcgaca ccctggtgaa 840

ccgcatcgag ctgaagggca tcgacttcaa ggaggacggc aacatcctgg ggcacaagct 900

ggagtacaac gccatcagcg acaacgtcta tatcaccgcc gacaagcaga agaacggcat 960

caaggccaac ttcaagagct agccctatat aagcagagct cgtttagtga accgtcagat 1020

cgcctggaga cgccatccac gctgttttga cctccataga agacaccggg accgatccag 1080

cctccgcggc cccggtaccg aattcgccac catggaagat gccaaaaaca ttaagaaggg 1140

cccagcgcca ttctacccac tcgaagacgg gaccgccggc gagcagctgc acaaagccat 1200

gaagcgctac gccctggtgc ccggcaccat cgcctttacc gacgcacata tcgaggtgga 1260

cattacctac gccgagtact tcgagatgag cgttcggctg gcagaagcta tgaagcgcta 1320

tgggctgaat acaaaccatc ggatcgtggt gtgcagcgag aatagcttgc agttcttcat 1380

gcccgtgttg ggtgccctgt tcatcggtgt ggctgtggcc ccagctaacg acatctacaa 1440

cgagcgcgag ctgctgaaca gcatgggcat cagccagccc accgtcgtat tcgtgagcaa 1500

gaaagggctg caaaagatcc tcaacgtgca aaagaagcta ccgatcatac aaaagatcat 1560

catcatggat agcaagaccg actaccaggg cttccaaagc atgtacacct tcgtgacttc 1620

ccatttgcca cccggcttca acgagtacga cttcgtgccc gagagcttcg accgggacaa 1680

aaccatcgcc ctgatcatga acagtagtgg cagtaccgga ttgcccaagg gcgtagccct 1740

accgcaccgc accgcttgtg tccgattcag tcatgcccgc gaccccatct tcggcaacca 1800

gatcatcccc gacaccgcta tcctcagcgt ggtgccattt caccacggct tcggcatgtt 1860

caccacgctg ggctacttga tctgcggctt tcgggtcgtg ctcatgtacc gcttcgagga 1920

ggagctattc ttgcgcagct tgcaagacta taagattcaa tctgccctgc tggtgcccac 1980

actatttagc ttcttcgcta agagcactct catcgacaag tacgacctaa gcaacttgca 2040

cgagatcgcc agcggcgggg cgccgctcag caaggaggta ggtgaggccg tggccaaacg 2100

cttccaccta ccaggcatcc gccagggcta cggcctgaca gaaacaacca gcgccattct 2160

gatcaccccc gaaggggacg acaagcctgg cgcagtaggc aaggtggtgc ccttcttcga 2220

ggctaaggtg gtggacttgg acaccggtaa gacactgggt gtgaaccagc gcggcgagct 2280

gtgcgtccgt ggccccatga tcatgagcgg ctacgttaac aaccccgagg ctacaaacgc 2340

tctcatcgac aaggacggct ggctgcacag cggcgacatc gcctactggg acgaggacga 2400

gcacttcttc atcgtggacc ggctgaagag cctgatcaaa tacaagggct accaggtagc 2460

cccagccgaa ctggagagca tcctgctgca acaccccaac atcttcgacg ccggggtcgc 2520

cggcctgccc gacgacgatg ccggcgagct gcccgccgca gtcgtcgtgc tggaacacgg 2580

taaaaccatg accgagaagg agatcgtgga ctatgtggcc agccaggtta caaccgccaa 2640

gaagctgcgc ggtggtgttg tgttcgtgga cgaggtgcct aaaggactga ccggcaagtt 2700

ggacgcccgc aagatccgcg agattctcat taaggccaag aagtaaagat cttattaaag 2760

cagaacttgt ttattgcagc ttataatggt tacaaataaa gcaatagcat cacaaatttc 2820

acaaataaag catttttttc actgcattct agttgtggtt tgtccaaact catcaatgta 2880

tcttatcatg tctggtcgac tctagaatct tccgcttcct cgctcactga ctcgctgcgc 2940

tcggtcgttc ggctgcggcg agcggtatca gctcactcaa aggcggtaat acggttatcc 3000

acagaatcag gggataacgc aggaaagaac atgtgagcaa aaggccagca aaaggccagg 3060

aaccgtaaaa aggccgcgtt gctggcgttt ttccataggc tccgcccccc tgacgagcat 3120

cacaaaaatc gacgctcaag tcagaggtgg cgaaacccga caggactata aagataccag 3180

gcgtttcccc ctggaagctc cctcgtgcgc tctcctgttc cgaccctgcc gcttaccgga 3240

tacctgtccg cctttctccc ttcgggaagc gtggcgcttt ctcatagctc acgctgtagg 3300

tatctcagtt cggtgtaggt cgttcgctcc aagctgggct gtgtgcacga accccccgtt 3360

cagcccgacc gctgcgcctt atccggtaac tatcgtcttg agtccaaccc ggtaagacac 3420

gacttatcgc cactggcagc agccactggt aacaggatta gcagagcgag gtatgtaggc 3480

ggtgctacag agttcttgaa gtggtggcct aactacggct acactagaag gacagtattt 3540

ggtatctgcg ctctgctgaa gccagttacc ttcggaaaaa gagttggtag ctcttgatcc 3600

ggcaaacaaa ccaccgctgg tagcggtggt ttttttgttt gcaagcagca gattacgcgc 3660

agaaaaaaag gatctcaaga agatcctttg atcttttcta cggggtctga cgctcagtgg 3720

aacgaaaact cacgttaagg gattttggtc atgagattat caaaaaggat cttcacctag 3780

atccttttaa attaaaaatg aagttttaaa tcaatctaaa gtatatatga gtaaacttgg 3840

tctgacagtt accaatgctt aatcagtgag gcacctatct cagcgatctg tctatttcgt 3900

tcatccatag ttgcctgact ccccgtcgtg tagataacta cgatacggga gggcttacca 3960

tctggcccca gtgctgcaat gataccgcga gacccacgct caccggctcc agatttatca 4020

gcaataaacc agccagccgg aagggccgag cgcagaagtg gtcctgcaac tttatccgcc 4080

tccatccagt ctattaattg ttgccgggaa gctagagtaa gtagttcgcc agttaatagt 4140

ttgcgcaacg ttgttgccat tgctacaggc atcgtggtgt cacgctcgtc gtttggtatg 4200

gcttcattca gctccggttc ccaacgatca aggcgagtta catgatcccc catgttgtgc 4260

aaaaaagcgg ttagctcctt cggtcctccg atcgttgtca gaagtaagtt ggccgcagtg 4320

ttatcactca tggttatggc agcactgcat aattctctta ctgtcatgcc atccgtaaga 4380

tgcttttctg tgactggtga gtactcaacc aagtcattct gagaatagtg tatgcggcga 4440

ccgagttgct cttgcccggc gtcaatacgg gataataccg cgccacatag cagaacttta 4500

aaagtgctca tcattggaaa acgttcttcg gggcgaaaac tctcaaggat cttaccgctg 4560

ttgagatcca gttcgatgta acccactcgt gcacccaact gatcttcagc atcttttact 4620

ttcaccagcg tttctgggtg agcaaaaaca ggaaggcaaa atgccgcaaa aaagggaata 4680

agggcgacac ggaaatgttg aatactcata ctcttccttt ttcaatatta ttgaagcatt 4740

tatcagggtt attgtctcat gagcggatac atatttgaat gtatttagaa aaataaacaa 4800

ataggggttc cgcgcacatt tccccgaaaa gtgccacctg acgtcgtc 4848

<210> 5
<211> 3226
<212> DNA
<213> Artificial

<220>
<223> TetO-targeting gRNA für dCas9-VP64 reporter plasmid

<400> 5
ctaaattgta agcgttaata ttttgttaaa attcgcgtta aatttttgtt aaatcagctc 60

attttttaac caataggccg aaatcggcaa aatcccttat aaatcaaaag aatagaccga 120

gatagggttg agtgttgttc cagtttggaa caagagtcca ctattaaaga acgtggactc 180

caacgtcaaa gggcgaaaaa ccgtctatca gggcgatggc ccactacgtg aaccatcacc 240

ctaatcaagt tttttggggt cgaggtgccg taaagcacta aatcggaacc ctaaagggag 300

cccccgattt agagcttgac ggggaaagcc ggcgaacgtg gcgagaaagg aagggaagaa 360

agcgaaagga gcgggcgcta gggcgctggc aagtgtagcg gtcacgctgc gcgtaaccac 420

cacacccgcc gcgcttaatg cgccgctaca gggcgcgtcc cattcgccat tcaggctgcg 480

caactgttgg gaagggcgat cggtgcgggc ctcttcgcta ttacgccagc tggcgaaagg 540

gggatgtgct gcaaggcgat taagttgggt aacgccaggg ttttcccagt cacgacgttg 600

taaaacgacg gccagtgagc gcgcgtaata cgactcacta tagggcgaat tgggtaccga 660

gggcctattt cccatgattc cttcatattt gcatatacga tacaaggctg ttagagagat 720

aattggaatt aatttgactg taaacacaaa gatattagta caaaatacgt gacgtagaaa 780

gtaataattt cttgggtagt ttgcagtttt aaaattatgt tttaaaatgg actatcatat 840

gcttatcgta acttgaaagt atttcgattt cttggcttta tatatcttgt ggaaaggacg 900

aaacaccgtc tctatcactg atagggaggt tttagagcta gaaatagcaa gttaaaataa 960

ggctagtccg ttatcaactt gaaaaagtgg caccgagtcg gtgctttttt tccgcggtgg 1020

agctccagct tttgttccct ttagtgaggg ttaattgcgc gcttggcgta atcatggtca 1080

tagctgtttc ctgtgtgaaa ttgttatccg ctcacaattc cacacaacat acgagccgga 1140

agcataaagt gtaaagcctg gggtgcctaa tgagtgagct aactcacatt aattgcgttg 1200

cgctcactgc ccgctttcca gtcgggaaac ctgtcgtgcc agctgcatta atgaatcggc 1260

caacgcgcgg ggagaggcgg tttgcgtatt gggcgctctt ccgcttcctc gctcactgac 1320

tcgctgcgct cggtcgttcg gctgcggcga gcggtatcag ctcactcaaa ggcggtaata 1380

cggttatcca cagaatcagg ggataacgca ggaaagaaca tgtgagcaaa aggccagcaa 1440

aaggccagga accgtaaaaa ggccgcgttg ctggcgtttt tccataggct ccgcccccct 1500

gacgagcatc acaaaaatcg acgctcaagt cagaggtggc gaaacccgac aggactataa 1560

agataccagg cgtttccccc tggaagctcc ctcgtgcgct ctcctgttcc gaccctgccg 1620

cttaccggat acctgtccgc ctttctccct tcgggaagcg tggcgctttc tcatagctca 1680

cgctgtaggt atctcagttc ggtgtaggtc gttcgctcca agctgggctg tgtgcacgaa 1740

ccccccgttc agcccgaccg ctgcgcctta tccggtaact atcgtcttga gtccaacccg 1800

gtaagacacg acttatcgcc actggcagca gccactggta acaggattag cagagcgagg 1860

tatgtaggcg gtgctacaga gttcttgaag tggtggccta actacggcta cactagaagg 1920

acagtatttg gtatctgcgc tctgctgaag ccagttacct tcggaaaaag agttggtagc 1980

tcttgatccg gcaaacaaac caccgctggt agcggtggtt tttttgtttg caagcagcag 2040

attacgcgca gaaaaaaagg atctcaagaa gatcctttga tcttttctac ggggtctgac 2100

gctcagtgga acgaaaactc acgttaaggg attttggtca tgagattatc aaaaaggatc 2160

ttcacctaga tccttttaaa ttaaaaatga agttttaaat caatctaaag tatatatgag 2220

taaacttggt ctgacagtta ccaatgctta atcagtgagg cacctatctc agcgatctgt 2280

ctatttcgtt catccatagt tgcctgactc cccgtcgtgt agataactac gatacgggag 2340

ggcttaccat ctggccccag tgctgcaatg ataccgcgag acccacgctc accggctcca 2400

gatttatcag caataaacca gccagccgga agggccgagc gcagaagtgg tcctgcaact 2460

ttatccgcct ccatccagtc tattaattgt tgccgggaag ctagagtaag tagttcgcca 2520

gttaatagtt tgcgcaacgt tgttgccatt gctacaggca tcgtggtgtc acgctcgtcg 2580

tttggtatgg cttcattcag ctccggttcc caacgatcaa ggcgagttac atgatccccc 2640

atgttgtgca aaaaagcggt tagctccttc ggtcctccga tcgttgtcag aagtaagttg 2700

gccgcagtgt tatcactcat ggttatggca gcactgcata attctcttac tgtcatgcca 2760

tccgtaagat gcttttctgt gactggtgag tactcaacca agtcattctg agaatagtgt 2820

atgcggcgac cgagttgctc ttgcccggcg tcaatacggg ataataccgc gccacatagc 2880

agaactttaa aagtgctcat cattggaaaa cgttcttcgg ggcgaaaact ctcaaggatc 2940

ttaccgctgt tgagatccag ttcgatgtaa cccactcgtg cacccaactg atcttcagca 3000

tcttttactt tcaccagcgt ttctgggtga gcaaaaacag gaaggcaaaa tgccgcaaaa 3060

aagggaataa gggcgacacg gaaatgttga atactcatac tcttcctttt tcaatattat 3120

tgaagcattt atcagggtta ttgtctcatg agcggataca tatttgaatg tatttagaaa 3180

aataaacaaa taggggttcc gcgcacattt ccccgaaaag tgccac 3226

<210> 6
<211> 14547
<212> DNA
<213> Artificial

<220>
<223> dCas9-VP64-2A-GFP

<400> 6
gtcgacggat cgggagatct cccgatcccc tatggtgcac tctcagtaca atctgctctg 60

atgccgcata gttaagccag tatctgctcc ctgcttgtgt gttggaggtc gctgagtagt 120

gcgcgagcaa aatttaagct acaacaaggc aaggcttgac cgacaattgc atgaagaatc 180

tgcttagggt taggcgtttt gcgctgcttc gcgatgtacg ggccagatat acgcgttgac 240

attgattatt gactagttat taatagtaat caattacggg gtcattagtt catagcccat 300

atatggagtt ccgcgttaca taacttacgg taaatggccc gcctggctga ccgcccaacg 360

acccccgccc attgacgtca ataatgacgt atgttcccat agtaacgcca atagggactt 420

tccattgacg tcaatgggtg gagtatttac ggtaaactgc ccacttggca gtacatcaag 480

tgtatcatat gccaagtacg ccccctattg acgtcaatga cggtaaatgg cccgcctggc 540

attatgccca gtacatgacc ttatgggact ttcctacttg gcagtacatc tacgtattag 600

tcatcgctat taccatggtg atgcggtttt ggcagtacat caatgggcgt ggatagcggt 660

ttgactcacg gggatttcca agtctccacc ccattgacgt caatgggagt ttgttttggc 720

accaaaatca acgggacttt ccaaaatgtc gtaacaactc cgccccattg acgcaaatgg 780

gcggtaggcg tgtacggtgg gaggtctata taagcagcgc gttttgcctg tactgggtct 840

ctctggttag accagatctg agcctgggag ctctctggct aactagggaa cccactgctt 900

aagcctcaat aaagcttgcc ttgagtgctt caagtagtgt gtgcccgtct gttgtgtgac 960

tctggtaact agagatccct cagacccttt tagtcagtgt ggaaaatctc tagcagtggc 1020

gcccgaacag ggacttgaaa gcgaaaggga aaccagagga gctctctcga cgcaggactc 1080

ggcttgctga agcgcgcacg gcaagaggcg aggggcggcg actggtgagt acgccaaaaa 1140

ttttgactag cggaggctag aaggagagag atgggtgcga gagcgtcagt attaagcggg 1200

ggagaattag atcgcgatgg gaaaaaattc ggttaaggcc agggggaaag aaaaaatata 1260

aattaaaaca tatagtatgg gcaagcaggg agctagaacg attcgcagtt aatcctggcc 1320

tgttagaaac atcagaaggc tgtagacaaa tactgggaca gctacaacca tcccttcaga 1380

caggatcaga agaacttaga tcattatata atacagtagc aaccctctat tgtgtgcatc 1440

aaaggataga gataaaagac accaaggaag ctttagacaa gatagaggaa gagcaaaaca 1500

aaagtaagac caccgcacag caagcggccg ctgatcttca gacctggagg aggagatatg 1560

agggacaatt ggagaagtga attatataaa tataaagtag taaaaattga accattagga 1620

gtagcaccca ccaaggcaaa gagaagagtg gtgcagagag aaaaaagagc agtgggaata 1680

ggagctttgt tccttgggtt cttgggagca gcaggaagca ctatgggcgc agcgtcaatg 1740

acgctgacgg tacaggccag acaattattg tctggtatag tgcagcagca gaacaatttg 1800

ctgagggcta ttgaggcgca acagcatctg ttgcaactca cagtctgggg catcaagcag 1860

ctccaggcaa gaatcctggc tgtggaaaga tacctaaagg atcaacagct cctggggatt 1920

tggggttgct ctggaaaact catttgcacc actgctgtgc cttggaatgc tagttggagt 1980

aataaatctc tggaacagat ttggaatcac acgacctgga tggagtggga cagagaaatt 2040

aacaattaca caagcttaat acactcctta attgaagaat cgcaaaacca gcaagaaaag 2100

aatgaacaag aattattgga attagataaa tgggcaagtt tgtggaattg gtttaacata 2160

acaaattggc tgtggtatat aaaattattc ataatgatag taggaggctt ggtaggttta 2220

agaatagttt ttgctgtact ttctatagtg aatagagtta ggcagggata ttcaccatta 2280

tcgtttcaga cccacctccc aaccccgagg ggacccgaca ggcccgaagg aatagaagaa 2340

gaaggtggag agagagacag agacagatcc attcgattag tgaacggatc ggcactgcgt 2400

gcgccaattc tgcagacaaa tggcagtatt catccacaat tttaaaagaa aaggggggat 2460

tggggggtac agtgcagggg aaagaatagt agacataata gcaacagaca tacaaactaa 2520

agaattacaa aaacaaatta caaaaattca aaattttcgg gtttattaca gggacagcag 2580

agatccagtt tggttaatta atgcaaagat ggataaagtt ttaaacagag aggaatcttt 2640

gcagctaatg gaccttctag gtcttgaaag gagtgggaat tggctccggt gcccgtcagt 2700

gggcagagcg cacatcgccc acagtccccg agaagttggg gggaggggtc ggcaattgaa 2760

ccggtgccta gagaaggtgg cgcggggtaa actgggaaag tgatgtcgtg tactggctcc 2820

gcctttttcc cgagggtggg ggagaaccgt atataagtgc agtagtcgcc gtgaacgttc 2880

tttttcgcaa cgggtttgcc gccagaacac aggtaagtgc cgtgtgtggt tcccgcgggc 2940

ctggcctctt tacgggttat ggcccttgcg tgccttgaat tacttccacc tggctgcagt 3000

acgtgattct tgatcccgag cttcgggttg gaagtgggtg ggagagttcg aggccttgcg 3060

cttaaggagc cccttcgcct cgtgcttgag ttgaggcctg gcctgggcgc tggggccgcc 3120

gcgtgcgaat ctggtggcac cttcgcgcct gtctcgctgc tttcgataag tctctagcca 3180

tttaaaattt ttgatgacct gctgcgacgc tttttttctg gcaagatagt cttgtaaatg 3240

cgggccaaga tctgcacact ggtatttcgg tttttggggc cgcgggcggc gacggggccc 3300

gtgcgtccca gcgcacatgt tcggcgaggc ggggcctgcg agcgcggcca ccgagaatcg 3360

gacgggggta gtctcaagct ggccggcctg ctctggtgcc tggcctcgcg ccgccgtgta 3420

tcgccccgcc ctgggcggca aggctggccc ggtcggcacc agttgcgtga gcggaaagat 3480

ggccgcttcc cggccctgct gcagggagct caaaatggag gacgcggcgc tcgggagagc 3540

gggcgggtga gtcacccaca caaaggaaaa gggcctttcc gtcctcagcc gtcgcttcat 3600

gtgactccac ggagtaccgg gcgccgtcca ggcacctcga ttagttctcg agcttttgga 3660

gtacgtcgtc tttaggttgg ggggaggggt tttatgcgat ggagtttccc cacactgagt 3720

gggtggagac tgaagttagg ccagcttggc acttgatgta attctccttg gaatttgccc 3780

tttttgagtt tggatcttgg ttcattctca agcctcagac agtggttcaa agtttttttc 3840

ttccatttca ggtgtcgtga cgtacggcca ccatgagccc caagaagaag agaaaggtgg 3900

aggccagcga caagaagtac agcatcggcc tggccatcgg caccaactct gtgggctggg 3960

ccgtgatcac cgacgagtac aaggtgccca gcaagaaatt caaggtgctg ggcaacaccg 4020

accggcacag catcaagaag aacctgatcg gagccctgct gttcgacagc ggcgaaacag 4080

ccgaggccac ccggctgaag agaaccgcca gaagaagata caccagacgg aagaaccgga 4140

tctgctatct gcaagagatc ttcagcaacg agatggccaa ggtggacgac agcttcttcc 4200

acagactgga agagtccttc ctggtggaag aggataagaa gcacgagcgg caccccatct 4260

tcggcaacat cgtggacgag gtggcctacc acgagaagta ccccaccatc taccacctga 4320

gaaagaaact ggtggacagc accgacaagg ccgacctgcg gctgatctat ctggccctgg 4380

cccacatgat caagttccgg ggccacttcc tgatcgaggg cgacctgaac cccgacaaca 4440

gcgacgtgga caagctgttc atccagctgg tgcagaccta caaccagctg ttcgaggaaa 4500

accccatcaa cgccagcggc gtggacgcca aggccatcct gtctgccaga ctgagcaaga 4560

gcagacggct ggaaaatctg atcgcccagc tgcccggcga gaagaagaat ggcctgttcg 4620

gcaacctgat tgccctgagc ctgggcctga cccccaactt caagagcaac ttcgacctgg 4680

ccgaggatgc caaactgcag ctgagcaagg acacctacga cgacgacctg gacaacctgc 4740

tggcccagat cggcgaccag tacgccgacc tgtttctggc cgccaagaac ctgtccgacg 4800

ccatcctgct gagcgacatc ctgagagtga acaccgagat caccaaggcc cccctgagcg 4860

cctctatgat caagagatac gacgagcacc accaggacct gaccctgctg aaagctctcg 4920

tgcggcagca gctgcctgag aagtacaaag agattttctt cgaccagagc aagaacggct 4980

acgccggcta cattgacggc ggagccagcc aggaagagtt ctacaagttc atcaagccca 5040

tcctggaaaa gatggacggc accgaggaac tgctcgtgaa gctgaacaga gaggacctgc 5100

tgcggaagca gcggaccttc gacaacggca gcatccccca ccagatccac ctgggagagc 5160

tgcacgccat tctgcggcgg caggaagatt tttacccatt cctgaaggac aaccgggaaa 5220

agatcgagaa gatcctgacc ttccgcatcc cctactacgt gggccctctg gccaggggaa 5280

acagcagatt cgcctggatg accagaaaga gcgaggaaac catcaccccc tggaacttcg 5340

aggaagtggt ggacaagggc gcttccgccc agagcttcat cgagcggatg accaacttcg 5400

ataagaacct gcccaacgag aaggtgctgc ccaagcacag cctgctgtac gagtacttca 5460

ccgtgtataa cgagctgacc aaagtgaaat acgtgaccga gggaatgaga aagcccgcct 5520

tcctgagcgg cgagcagaaa aaggccatcg tggacctgct gttcaagacc aaccggaaag 5580

tgaccgtgaa gcagctgaaa gaggactact tcaagaaaat cgagtgcttc gactccgtgg 5640

aaatctccgg cgtggaagat cggttcaacg cctccctggg cacataccac gatctgctga 5700

aaattatcaa ggacaaggac ttcctggaca atgaggaaaa cgaggacatt ctggaagata 5760

tcgtgctgac cctgacactg tttgaggaca gagagatgat cgaggaacgg ctgaaaacct 5820

atgcccacct gttcgacgac aaagtgatga agcagctgaa gcggcggaga tacaccggct 5880

ggggcaggct gagccggaag ctgatcaacg gcatccggga caagcagtcc ggcaagacaa 5940

tcctggattt cctgaagtcc gacggcttcg ccaacagaaa cttcatgcag ctgatccacg 6000

acgacagcct gacctttaaa gaggacatcc agaaagccca ggtgtccggc cagggcgata 6060

gcctgcacga gcacattgcc aatctggccg gcagccccgc cattaagaag ggcatcctgc 6120

agacagtgaa ggtggtggac gagctcgtga aagtgatggg ccggcacaag cccgagaaca 6180

tcgtgatcga aatggccaga gagaaccaga ccacccagaa gggacagaag aacagccgcg 6240

agagaatgaa gcggatcgaa gagggcatca aagagctggg cagccagatc ctgaaagaac 6300

accccgtgga aaacacccag ctgcagaacg agaagctgta cctgtactac ctgcagaatg 6360

ggcgggatat gtacgtggac caggaactgg acatcaaccg gctgtccgac tacgatgtgg 6420

acgctatcgt gcctcagagc tttctgaagg acgactccat cgacaacaag gtgctgacca 6480

gaagcgacaa gaaccggggc aagagcgaca acgtgccctc cgaagaggtc gtgaagaaga 6540

tgaagaacta ctggcggcag ctgctgaacg ccaagctgat tacccagaga aagttcgaca 6600

atctgaccaa ggccgagaga ggcggcctga gcgaactgga taaggccggc ttcatcaaga 6660

gacagctggt ggaaacccgg cagatcacaa agcacgtggc acagatcctg gactcccgga 6720

tgaacactaa gtacgacgag aatgacaagc tgatccggga agtgaaagtg atcaccctga 6780

agtccaagct ggtgtccgat ttccggaagg atttccagtt ttacaaagtg cgcgagatca 6840

acaactacca ccacgcccac gacgcctacc tgaacgccgt cgtgggaacc gccctgatca 6900

aaaagtaccc taagctggaa agcgagttcg tgtacggcga ctacaaggtg tacgacgtgc 6960

ggaagatgat cgccaagagc gagcaggaaa tcggcaaggc taccgccaag tacttcttct 7020

acagcaacat catgaacttt ttcaagaccg agattaccct ggccaacggc gagatccgga 7080

agcggcctct gatcgagaca aacggcgaaa ccggggagat cgtgtgggat aagggccggg 7140

attttgccac cgtgcggaaa gtgctgagca tgccccaagt gaatatcgtg aaaaagaccg 7200

aggtgcagac aggcggcttc agcaaagagt ctatcctgcc caagaggaac agcgataagc 7260

tgatcgccag aaagaaggac tgggacccta agaagtacgg cggcttcgac agccccaccg 7320

tggcctattc tgtgctggtg gtggccaaag tggaaaaggg caagtccaag aaactgaaga 7380

gtgtgaaaga gctgctgggg atcaccatca tggaaagaag cagcttcgag aagaatccca 7440

tcgactttct ggaagccaag ggctacaaag aagtgaaaaa ggacctgatc atcaagctgc 7500

ctaagtactc cctgttcgag ctggaaaacg gccggaagag aatgctggcc tctgccggcg 7560

aactgcagaa gggaaacgaa ctggccctgc cctccaaata tgtgaacttc ctgtacctgg 7620

ccagccacta tgagaagctg aagggctccc ccgaggataa tgagcagaaa cagctgtttg 7680

tggaacagca caagcactac ctggacgaga tcatcgagca gatcagcgag ttctccaaga 7740

gagtgatcct ggccgacgct aatctggaca aagtgctgtc cgcctacaac aagcaccggg 7800

ataagcccat cagagagcag gccgagaata tcatccacct gtttaccctg accaatctgg 7860

gagcccctgc cgccttcaag tactttgaca ccaccatcga ccggaagagg tacaccagca 7920

ccaaagaggt gctggacgcc accctgatcc accagagcat caccggcctg tacgagacac 7980

ggatcgacct gtctcagctg ggaggcgaca gcgctggagg aggtggaagc ggaggaggag 8040

gaagcggagg aggaggtagc ggacctaaga aaaagaggaa ggtggcggcc gctggatccg 8100

gacgggctga cgcattggac gattttgatc tggatatgct gggaagtgac gccctcgatg 8160

attttgacct tgacatgctt ggttcggatg cccttgatga ctttgacctc gacatgctcg 8220

gcagtgacgc ccttgatgat ttcgacctgg acatgctgat taacgctagc ggcagtggag 8280

agggcagagg aagtctgcta acatgcggtg acgtcgagga gaatcctggc ccagtgagca 8340

agggcgagga gctgttcacc ggggtggtgc ccatcctggt cgagctggac ggcgacgtaa 8400

acggccacaa gttcagcgtg tccggcgagg gcgagggcga tgccacctac ggcaagctga 8460

ccctgaagtt catctgcacc accggcaagc tgcccgtgcc ctggcccacc ctcgtgacca 8520

ccctgaccta cggcgtgcag tgcttcagcc gctaccccga ccacatgaag cagcacgact 8580

tcttcaagtc cgccatgccc gaaggctacg tccaggagcg caccatcttc ttcaaggacg 8640

acggcaacta caagacccgc gccgaggtga agttcgaggg cgacaccctg gtgaaccgca 8700

tcgagctgaa gggcatcgac ttcaaggagg acggcaacat cctggggcac aagctggagt 8760

acaactacaa cagccacaac gtctatatca tggccgacaa gcagaagaac ggcatcaagg 8820

tgaacttcaa gatccgccac aacatcgagg acggcagcgt gcagctcgcc gaccactacc 8880

agcagaacac ccccatcggc gacggccccg tgctgctgcc cgacaaccac tacctgagca 8940

cccagtccgc cctgagcaaa gaccccaacg agaagcgcga tcacatggtc ctgctggagt 9000

tcgtgaccgc cgccgggatc actctcggca tggacgagct gtacaagtaa gaattcgata 9060

tcaagcttat cgataatcaa cctctggatt acaaaatttg tgaaagattg actggtattc 9120

ttaactatgt tgctcctttt acgctatgtg gatacgctgc tttaatgcct ttgtatcatg 9180

ctattgcttc ccgtatggct ttcattttct cctccttgta taaatcctgg ttgctgtctc 9240

tttatgagga gttgtggccc gttgtcaggc aacgtggcgt ggtgtgcact gtgtttgctg 9300

acgcaacccc cactggttgg ggcattgcca ccacctgtca gctcctttcc gggactttcg 9360

ctttccccct ccctattgcc acggcggaac tcatcgccgc ctgccttgcc cgctgctgga 9420

caggggctcg gctgttgggc actgacaatt ccgtggtgtt gtcggggaaa tcatcgtcct 9480

ttccttggct gctcgcctgt gttgccacct ggattctgcg cgggacgtcc ttctgctacg 9540

tcccttcggc cctcaatcca gcggaccttc cttcccgcgg cctgctgccg gctctgcggc 9600

ctcttccgcg tcttcgcctt cgccctcaga cgagtcggat ctccctttgg gccgcctccc 9660

cgcatcgata ccgtcgacct cgagacctag aaaaacatgg agcaatcaca agtagcaata 9720

cagcagctac caatgctgat tgtgcctggc tagaagcaca agaggaggag gaggtgggtt 9780

ttccagtcac acctcaggta cctttaagac caatgactta caaggcagct gtagatctta 9840

gccacttttt aaaagaaaag gggggactgg aagggctaat tcactcccaa cgaagacaag 9900

atatccttga tctgtggatc taccacacac aaggctactt ccctgattgg cagaactaca 9960

caccagggcc agggatcaga tatccactga cctttggatg gtgctacaag ctagtaccag 10020

ttgagcaaga gaaggtagaa gaagccaatg aaggagagaa cacccgcttg ttacaccctg 10080

tgagcctgca tgggatggat gacccggaga gagaagtatt agagtggagg tttgacagcc 10140

gcctagcatt tcatcacatg gcccgagagc tgcatccgga ctgtactggg tctctctggt 10200

tagaccagat ctgagcctgg gagctctctg gctaactagg gaacccactg cttaagcctc 10260

aataaagctt gccttgagtg cttcaagtag tgtgtgcccg tctgttgtgt gactctggta 10320

actagagatc cctcagaccc ttttagtcag tgtggaaaat ctctagcagg gcccgtttaa 10380

acccgctgat cagcctcgac tgtgccttct agttgccagc catctgttgt ttgcccctcc 10440

cccgtgcctt ccttgaccct ggaaggtgcc actcccactg tcctttccta ataaaatgag 10500

gaaattgcat cgcattgtct gagtaggtgt cattctattc tggggggtgg ggtggggcag 10560

gacagcaagg gggaggattg ggaagacaat agcaggcatg ctggggatgc ggtgggctct 10620

atggcttctg aggcggaaag aaccagctgg ggctctaggg ggtatcccca cgcgccctgt 10680

agcggcgcat taagcgcggc gggggggggg gggggtgggg gcaggacagc aagggggagg 10740

attgggaaga caatagcagg catgctgggg atgcggtggg ctctatggct tctgaggcgg 10800

aaagaaccag ctggggctct agggggtatc cccacgcgcc ctgtagcggc gcattaagcg 10860

cggcgggtgt ggtggttacg cgcagcgtga ccgctacact tgccagcgcc ctagcgcccg 10920

ctcctttcgc tttcttccct tcctttctcg ccacgttcgc cggctttccc cgtcaagctc 10980

taaatcgggg gctcccttta gggttccgat ttagtgcttt acggcacctc gaccccaaaa 11040

aacttgatta gggtgatggt tcacgtagtg ggccatcgcc ctgatagacg gtttttcgcc 11100

ctttgacgtt ggagtccacg ttctttaata gtggactctt gttccaaact ggaacaacac 11160

tcaaccctat ctcggtctat tcttttgatt tataagggat tttgccgatt tcggcctatt 11220

ggttaaaaaa tgagctgatt taacaaaaat ttaacgcgaa ttaattctgt ggaatgtgtg 11280

tcagttaggg tgtggaaagt ccccaggctc cccagcaggc agaagtatgc aaagcatgca 11340

tctcaattag tcagcaacca ggtgtggaaa gtccccaggc tccccagcag gcagaagtat 11400

gcaaagcatg catctcaatt agtcagcaac catagtcccg cccctaactc cgcccatccc 11460

gcccctaact ccgcccagtt ccgcccattc tccgccccat ggctgactaa ttttttttat 11520

ttatgcagag gccgaggccg cctctgcctc tgagctattc cagaagtagt gaggaggctt 11580

ttttggaggc ctaggctttt gcaaaaagct cccgggagct tgtatatcca ttttcggatc 11640

tgatcagcac gtgttgacaa ttaatcatcg gcatagtata tcggcatagt ataatacgac 11700

aaggtgagga actaaaccat ggccaagttg accagtgccg ttccggtgct caccgcgcgc 11760

gacgtcgccg gagcggtcga gttctggacc gaccggctcg ggttctcccg ggacttcgtg 11820

gaggacgact tcgccggtgt ggtccgggac gacgtgaccc tgttcatcag cgcggtccag 11880

gaccaggtgg tgccggacaa caccctggcc tgggtgtggg tgcgcggcct ggacgagctg 11940

tacgccgagt ggtcggaggt cgtgtccacg aacttccggg acgcctccgg gccggccatg 12000

accgagatcg gcgagcagcc gtgggggcgg gagttcgccc tgcgcgaccc ggccggcaac 12060

tgcgtgcact tcgtggccga ggagcaggac tgacacgtgc tacgagattt cgattccacc 12120

gccgccttct atgaaaggtt gggcttcgga atcgttttcc gggacgccgg ctggatgatc 12180

ctccagcgcg gggatctcat gctggagttc ttcgcccacc ccaacttgtt tattgcagct 12240

tataatggtt acaaataaag caatagcatc acaaatttca caaataaagc atttttttca 12300

ctgcattcta gttgtggttt gtccaaactc atcaatgtat cttatcatgt ctgtataccg 12360

tcgacctcta gctagagctt ggcgtaatca tggtcatagc tgtttcctgt gtgaaattgt 12420

tatccgctca caattccaca caacatacga gccggaagca taaagtgtaa agcctggggt 12480

gcctaatgag tgagctaact cacattaatt gcgttgcgct cactgcccgc tttccagtcg 12540

ggaaacctgt cgtgccagct gcattaatga atcggccaac gcgcggggag aggcggtttg 12600

cgtattgggc gctcttccgc ttcctcgctc actgactcgc tgcgctcggt cgttcggctg 12660

cggcgagcgg tatcagctca ctcaaaggcg gtaatacggt tatccacaga atcaggggat 12720

aacgcaggaa agaacatgtg agcaaaaggc cagcaaaagg ccaggaaccg taaaaaggcc 12780

gcgttgctgg cgtttttcca taggctccgc ccccctgacg agcatcacaa aaatcgacgc 12840

tcaagtcaga ggtggcgaaa cccgacagga ctataaagat accaggcgtt tccccctgga 12900

agctccctcg tgcgctctcc tgttccgacc ctgccgctta ccggatacct gtccgccttt 12960

ctcccttcgg gaagcgtggc gctttctcat agctcacgct gtaggtatct cagttcggtg 13020

taggtcgttc gctccaagct gggctgtgtg cacgaacccc ccgttcagcc cgaccgctgc 13080

gccttatccg gtaactatcg tcttgagtcc aacccggtaa gacacgactt atcgccactg 13140

gcagcagcca ctggtaacag gattagcaga gcgaggtatg taggcggtgc tacagagttc 13200

ttgaagtggt ggcctaacta cggctacact agaagaacag tatttggtat ctgcgctctg 13260

ctgaagccag ttaccttcgg aaaaagagtt ggtagctctt gatccggcaa acaaaccacc 13320

gctggtagcg gtggtttttt tgtttgcaag cagcagatta cgcgcagaaa aaaaggatct 13380

caagaagatc ctttgatctt ttctacgggg tctgacgctc agtggaacga aaactcacgt 13440

taagggattt tggtcatgag attatcaaaa aggatcttca cctagatcct tttaaattaa 13500

aaatgaagtt ttaaatcaat ctaaagtata tatgagtaaa cttggtctga cagttaccaa 13560

tgcttaatca gtgaggcacc tatctcagcg atctgtctat ttcgttcatc catagttgcc 13620

tgactccccg tcgtgtagat aactacgata cgggagggct taccatctgg ccccagtgct 13680

gcaatgatac cgcgagaccc acgctcaccg gctccagatt tatcagcaat aaaccagcca 13740

gccggaaggg ccgagcgcag aagtggtcct gcaactttat ccgcctccat ccagtctatt 13800

aattgttgcc gggaagctag agtaagtagt tcgccagtta atagtttgcg caacgttgtt 13860

gccattgcta caggcatcgt ggtgtcacgc tcgtcgtttg gtatggcttc attcagctcc 13920

ggttcccaac gatcaaggcg agttacatga tcccccatgt tgtgcaaaaa agcggttagc 13980

tccttcggtc ctccgatcgt tgtcagaagt aagttggccg cagtgttatc actcatggtt 14040

atggcagcac tgcataattc tcttactgtc atgccatccg taagatgctt ttctgtgact 14100

ggtgagtact caaccaagtc attctgagaa tagtgtatgc ggcgaccgag ttgctcttgc 14160

ccggcgtcaa tacgggataa taccgcgcca catagcagaa ctttaaaagt gctcatcatt 14220

ggaaaacgtt cttcggggcg aaaactctca aggatcttac cgctgttgag atccagttcg 14280

atgtaaccca ctcgtgcacc caactgatct tcagcatctt ttactttcac cagcgtttct 14340

gggtgagcaa aaacaggaag gcaaaatgcc gcaaaaaagg gaataagggc gacacggaaa 14400

tgttgaatac tcatactctt cctttttcaa tattattgaa gcatttatca gggttattgt 14460

ctcatgagcg gatacatatt tgaatgtatt tagaaaaata aacaaatagg ggttccgcgc 14520

acatttcccc gaaaagtgcc acctgac 14547

<210> 7
<211> 5687
<212> DNA
<213> Artificial

<220>
<223> CMV-AcrIIA4-2xmir122BS-BGH

<400> 7
agatctcccg atcccctatg gtgcactctc agtacaatct gctctgatgc cgcatagtta 60

agccagtatc tgctccctgc ttgtgtgttg gaggtcgctg agtagtgcgc gagcaaaatt 120

taagctacaa caaggcaagg cttgaccgac aattgcatga agaatctgct tagggttagg 180

cgttttgcgc tgcttcgcga tgtacgggcc agatatacgc gttgacattg attattgact 240

agttattaat agtaatcaat tacggggtca ttagttcata gcccatatat ggagttccgc 300

gttacataac ttacggtaaa tggcccgcct ggctgaccgc ccaacgaccc ccgcccattg 360

acgtcaataa tgacgtatgt tcccatagta acgccaatag ggactttcca ttgacgtcaa 420

tgggtggagt atttacggta aactgcccac ttggcagtac atcaagtgta tcatatgcca 480

agtacgcccc ctattgacgt caatgacggt aaatggcccg cctggcatta tgcccagtac 540

atgaccttat gggactttcc tacttggcag tacatctacg tattagtcat cgctattacc 600

atggtgatgc ggttttggca gtacatcaat gggcgtggat agcggtttga ctcacgggga 660

tttccaagtc tccaccccat tgacgtcaat gggagtttgt tttggcacca aaatcaacgg 720

gactttccaa aatgtcgtaa caactccgcc ccattgacgc aaatgggcgg taggcgtgta 780

cggtgggagg tctatataag cagagctctc tggctaacta gagaacccac tgcttactgg 840

cttatcgaaa ttaatacgac tcactatagg gagacccaag ctggctagca tgaacattaa 900

cgacctgatt cgggaaataa agaacaagga ctacacggtc aagctgtcag gcaccgactc 960

caactctata acacagctca ttataagggt taataatgat ggcaacgaat atgtcatctc 1020

tgaatccgag aatgaatcaa tcgtggagaa attcatcagt gcatttaaaa acggctggaa 1080

ccaggagtac gaagatgagg aagagttcta caatgatatg caaactatca cactgaaatc 1140

tgagctgaac tagctcgagc ggccgccact gtgctggata tctgcagaat tacaaacacc 1200

attgtcacac tccattatat acaaacacca ttgtcacact ccatagctta agtttaaacc 1260

gctgatcagc ctcgactgtg ccttctagtt gccagccatc tgttgtttgc ccctcccccg 1320

tgccttcctt gaccctggaa ggtgccactc ccactgtcct ttcctaataa aatgaggaaa 1380

ttgcatcgca ttgtctgagt aggtgtcatt ctattctggg gggtggggtg gggcaggaca 1440

gcaaggggga ggattgggaa gacaatagca ggcatgctgg ggatgcggtg ggctctatgg 1500

cttctgaggc ggaaagaacc agctggggct ctagggggta tccccacgcg ccctgtagcg 1560

gcgcattaag cgcggcgggt gtggtggtta cgcgcagcgt gaccgctaca cttgccagcg 1620

ccctagcgcc cgctcctttc gctttcttcc cttcctttct cgccacgttc gccggctttc 1680

cccgtcaagc tctaaatcgg gggctccctt tagggttccg atttagtgct ttacggcacc 1740

tcgaccccaa aaaacttgat tagggtgatg gttcacgtag tgggccatcg ccctgataga 1800

cggtttttcg ccctttgacg ttggagtcca cgttctttaa tagtggactc ttgttccaaa 1860

ctggaacaac actcaaccct atctcggtct attcttttga tttataaggg attttgccga 1920

tttcggccta ttggttaaaa aatgagctga tttaacaaaa atttaacgcg aattaattct 1980

gtggaatgtg tgtcagttag ggtgtggaaa gtccccaggc tccccagcag gcagaagtat 2040

gcaaagcatg catctcaatt agtcagcaac caggtgtgga aagtccccag gctccccagc 2100

aggcagaagt atgcaaagca tgcatctcaa ttagtcagca accatagtcc cgcccctaac 2160

tccgcccatc ccgcccctaa ctccgcccag ttccgcccat tctccgcccc atggctgact 2220

aatttttttt atttatgcag aggccgaggc cgcctctgcc tctgagctat tccagaagta 2280

gtgaggaggc ttttttggag gcctaggctt ttgcaaaaag ctcccgggag cttgtatatc 2340

cattttcgga tctgatcaag agacaggatg aggatcgttt cgcatgattg aacaagatgg 2400

attgcacgca ggttctccgg ccgcttgggt ggagaggcta ttcggctatg actgggcaca 2460

acagacaatc ggctgctctg atgccgccgt gttccggctg tcagcgcagg ggcgcccggt 2520

tctttttgtc aagaccgacc tgtccggtgc cctgaatgaa ctgcaggacg aggcagcgcg 2580

gctatcgtgg ctggccacga cgggcgttcc ttgcgcagct gtgctcgacg ttgtcactga 2640

agcgggaagg gactggctgc tattgggcga agtgccgggg caggatctcc tgtcatctca 2700

ccttgctcct gccgagaaag tatccatcat ggctgatgca atgcggcggc tgcatacgct 2760

tgatccggct acctgcccat tcgaccacca agcgaaacat cgcatcgagc gagcacgtac 2820

tcggatggaa gccggtcttg tcgatcagga tgatctggac gaagagcatc aggggctcgc 2880

gccagccgaa ctgttcgcca ggctcaaggc gcgcatgccc gacggcgagg atctcgtcgt 2940

gacccatggc gatgcctgct tgccgaatat catggtggaa aatggccgct tttctggatt 3000

catcgactgt ggccggctgg gtgtggcgga ccgctatcag gacatagcgt tggctacccg 3060

tgatattgct gaagagcttg gcggcgaatg ggctgaccgc ttcctcgtgc tttacggtat 3120

cgccgctccc gattcgcagc gcatcgcctt ctatcgcctt cttgacgagt tcttctgagc 3180

gggactctgg ggttcgaaat gaccgaccaa gcgacgccca acctgccatc acgagatttc 3240

gattccaccg ccgccttcta tgaaaggttg ggcttcggaa tcgttttccg ggacgccggc 3300

tggatgatcc tccagcgcgg ggatctcatg ctggagttct tcgcccaccc caacttgttt 3360

attgcagctt ataatggtta caaataaagc aatagcatca caaatttcac aaataaagca 3420

tttttttcac tgcattctag ttgtggtttg tccaaactca tcaatgtatc ttatcatgtc 3480

tgtataccgt cgacctctag ctagagcttg gcgtaatcat ggtcatagct gtttcctgtg 3540

tgaaattgtt atccgctcac aattccacac aacatacgag ccggaagcat aaagtgtaaa 3600

gcctggggtg cctaatgagt gagctaactc acattaattg cgttgcgctc actgcccgct 3660

ttccagtcgg gaaacctgtc gtgccagctg cattaatgaa tcggccaacg cgcggggaga 3720

ggcggtttgc gtattgggcg ctcttccgct tcctcgctca ctgactcgct gcgctcggtc 3780

gttcggctgc ggcgagcggt atcagctcac tcaaaggcgg taatacggtt atccacagaa 3840

tcaggggata acgcaggaaa gaacatgtga gcaaaaggcc agcaaaaggc caggaaccgt 3900

aaaaaggccg cgttgctggc gtttttccat aggctccgcc cccctgacga gcatcacaaa 3960

aatcgacgct caagtcagag gtggcgaaac ccgacaggac tataaagata ccaggcgttt 4020

ccccctggaa gctccctcgt gcgctctcct gttccgaccc tgccgcttac cggatacctg 4080

tccgcctttc tcccttcggg aagcgtggcg ctttctcata gctcacgctg taggtatctc 4140

agttcggtgt aggtcgttcg ctccaagctg ggctgtgtgc acgaaccccc cgttcagccc 4200

gaccgctgcg ccttatccgg taactatcgt cttgagtcca acccggtaag acacgactta 4260

tcgccactgg cagcagccac tggtaacagg attagcagag cgaggtatgt aggcggtgct 4320

acagagttct tgaagtggtg gcctaactac ggctacacta gaagaacagt atttggtatc 4380

tgcgctctgc tgaagccagt taccttcgga aaaagagttg gtagctcttg atccggcaaa 4440

caaaccaccg ctggtagcgg tttttttgtt tgcaagcagc agattacgcg cagaaaaaaa 4500

ggatctcaag aagatccttt gatcttttct acggggtctg acgctcagtg gaacgaaaac 4560

tcacgttaag ggattttggt catgagatta tcaaaaagga tcttcaccta gatcctttta 4620

aattaaaaat gaagttttaa atcaatctaa agtatatatg agtaaacttg gtctgacagt 4680

taccaatgct taatcagtga ggcacctatc tcagcgatct gtctatttcg ttcatccata 4740

gttgcctgac tccccgtcgt gtagataact acgatacggg agggcttacc atctggcccc 4800

agtgctgcaa tgataccgcg agacccacgc tcaccggctc cagatttatc agcaataaac 4860

cagccagccg gaagggccga gcgcagaagt ggtcctgcaa ctttatccgc ctccatccag 4920

tctattaatt gttgccggga agctagagta agtagttcgc cagttaatag tttgcgcaac 4980

gttgttgcca ttgctacagg catcgtggtg tcacgctcgt cgtttggtat ggcttcattc 5040

agctccggtt cccaacgatc aaggcgagtt acatgatccc ccatgttgtg caaaaaagcg 5100

gttagctcct tcggtcctcc gatcgttgtc agaagtaagt tggccgcagt gttatcactc 5160

atggttatgg cagcactgca taattctctt actgtcatgc catccgtaag atgcttttct 5220

gtgactggtg agtactcaac caagtcattc tgagaatagt gtatgcggcg accgagttgc 5280

tcttgcccgg cgtcaatacg ggataatacc gcgccacata gcagaacttt aaaagtgctc 5340

atcattggaa aacgttcttc ggggcgaaaa ctctcaagga tcttaccgct gttgagatcc 5400

agttcgatgt aacccactcg tgcacccaac tgatcttcag catcttttac tttcaccagc 5460

gtttctgggt gagcaaaaac aggaaggcaa aatgccgcaa aaaagggaat aagggcgaca 5520

cggaaatgtt gaatactcat actcttcctt tttcaatatt attgaagcat ttatcagggt 5580

tattgtctca tgagcggata catatttgaa tgtatttaga aaaataaaca aataggggtt 5640

ccgcgcacat ttccccgaaa agtgccacct gacgtcgacg gatcggg 5687

<210> 8
<211> 5687
<212> DNA
<213> Artificial

<220>
<223> CMV-AcrIIA4-scaffold-BGH

<400> 8
agatctcccg atcccctatg gtgcactctc agtacaatct gctctgatgc cgcatagtta 60

agccagtatc tgctccctgc ttgtgtgttg gaggtcgctg agtagtgcgc gagcaaaatt 120

taagctacaa caaggcaagg cttgaccgac aattgcatga agaatctgct tagggttagg 180

cgttttgcgc tgcttcgcga tgtacgggcc agatatacgc gttgacattg attattgact 240

agttattaat agtaatcaat tacggggtca ttagttcata gcccatatat ggagttccgc 300

gttacataac ttacggtaaa tggcccgcct ggctgaccgc ccaacgaccc ccgcccattg 360

acgtcaataa tgacgtatgt tcccatagta acgccaatag ggactttcca ttgacgtcaa 420

tgggtggagt atttacggta aactgcccac ttggcagtac atcaagtgta tcatatgcca 480

agtacgcccc ctattgacgt caatgacggt aaatggcccg cctggcatta tgcccagtac 540

atgaccttat gggactttcc tacttggcag tacatctacg tattagtcat cgctattacc 600

atggtgatgc ggttttggca gtacatcaat gggcgtggat agcggtttga ctcacgggga 660

tttccaagtc tccaccccat tgacgtcaat gggagtttgt tttggcacca aaatcaacgg 720

gactttccaa aatgtcgtaa caactccgcc ccattgacgc aaatgggcgg taggcgtgta 780

cggtgggagg tctatataag cagagctctc tggctaacta gagaacccac tgcttactgg 840

cttatcgaaa ttaatacgac tcactatagg gagacccaag ctggctagca tgaacattaa 900

cgacctgatt cgggaaataa agaacaagga ctacacggtc aagctgtcag gcaccgactc 960

caactctata acacagctca ttataagggt taataatgat ggcaacgaat atgtcatctc 1020

tgaatccgag aatgaatcaa tcgtggagaa attcatcagt gcatttaaaa acggctggaa 1080

ccaggagtac gaagatgagg aagagttcta caatgatatg caaactatca cactgaaatc 1140

tgagctgaac tagctcgagc ggccgccact gtgctggata tctgcagaat tagagacgcc 1200

gttgatgttg aagtttatat accgttgatg ttgaagtcgt ctctagctta agtttaaacc 1260

gctgatcagc ctcgactgtg ccttctagtt gccagccatc tgttgtttgc ccctcccccg 1320

tgccttcctt gaccctggaa ggtgccactc ccactgtcct ttcctaataa aatgaggaaa 1380

ttgcatcgca ttgtctgagt aggtgtcatt ctattctggg gggtggggtg gggcaggaca 1440

gcaaggggga ggattgggaa gacaatagca ggcatgctgg ggatgcggtg ggctctatgg 1500

cttctgaggc ggaaagaacc agctggggct ctagggggta tccccacgcg ccctgtagcg 1560

gcgcattaag cgcggcgggt gtggtggtta cgcgcagcgt gaccgctaca cttgccagcg 1620

ccctagcgcc cgctcctttc gctttcttcc cttcctttct cgccacgttc gccggctttc 1680

cccgtcaagc tctaaatcgg gggctccctt tagggttccg atttagtgct ttacggcacc 1740

tcgaccccaa aaaacttgat tagggtgatg gttcacgtag tgggccatcg ccctgataga 1800

cggtttttcg ccctttgacg ttggagtcca cgttctttaa tagtggactc ttgttccaaa 1860

ctggaacaac actcaaccct atctcggtct attcttttga tttataaggg attttgccga 1920

tttcggccta ttggttaaaa aatgagctga tttaacaaaa atttaacgcg aattaattct 1980

gtggaatgtg tgtcagttag ggtgtggaaa gtccccaggc tccccagcag gcagaagtat 2040

gcaaagcatg catctcaatt agtcagcaac caggtgtgga aagtccccag gctccccagc 2100

aggcagaagt atgcaaagca tgcatctcaa ttagtcagca accatagtcc cgcccctaac 2160

tccgcccatc ccgcccctaa ctccgcccag ttccgcccat tctccgcccc atggctgact 2220

aatttttttt atttatgcag aggccgaggc cgcctctgcc tctgagctat tccagaagta 2280

gtgaggaggc ttttttggag gcctaggctt ttgcaaaaag ctcccgggag cttgtatatc 2340

cattttcgga tctgatcaag agacaggatg aggatcgttt cgcatgattg aacaagatgg 2400

attgcacgca ggttctccgg ccgcttgggt ggagaggcta ttcggctatg actgggcaca 2460

acagacaatc ggctgctctg atgccgccgt gttccggctg tcagcgcagg ggcgcccggt 2520

tctttttgtc aagaccgacc tgtccggtgc cctgaatgaa ctgcaggacg aggcagcgcg 2580

gctatcgtgg ctggccacga cgggcgttcc ttgcgcagct gtgctcgacg ttgtcactga 2640

agcgggaagg gactggctgc tattgggcga agtgccgggg caggatctcc tgtcatctca 2700

ccttgctcct gccgagaaag tatccatcat ggctgatgca atgcggcggc tgcatacgct 2760

tgatccggct acctgcccat tcgaccacca agcgaaacat cgcatcgagc gagcacgtac 2820

tcggatggaa gccggtcttg tcgatcagga tgatctggac gaagagcatc aggggctcgc 2880

gccagccgaa ctgttcgcca ggctcaaggc gcgcatgccc gacggcgagg atctcgtcgt 2940

gacccatggc gatgcctgct tgccgaatat catggtggaa aatggccgct tttctggatt 3000

catcgactgt ggccggctgg gtgtggcgga ccgctatcag gacatagcgt tggctacccg 3060

tgatattgct gaagagcttg gcggcgaatg ggctgaccgc ttcctcgtgc tttacggtat 3120

cgccgctccc gattcgcagc gcatcgcctt ctatcgcctt cttgacgagt tcttctgagc 3180

gggactctgg ggttcgaaat gaccgaccaa gcgacgccca acctgccatc acgagatttc 3240

gattccaccg ccgccttcta tgaaaggttg ggcttcggaa tcgttttccg ggacgccggc 3300

tggatgatcc tccagcgcgg ggatctcatg ctggagttct tcgcccaccc caacttgttt 3360

attgcagctt ataatggtta caaataaagc aatagcatca caaatttcac aaataaagca 3420

tttttttcac tgcattctag ttgtggtttg tccaaactca tcaatgtatc ttatcatgtc 3480

tgtataccgt cgacctctag ctagagcttg gcgtaatcat ggtcatagct gtttcctgtg 3540

tgaaattgtt atccgctcac aattccacac aacatacgag ccggaagcat aaagtgtaaa 3600

gcctggggtg cctaatgagt gagctaactc acattaattg cgttgcgctc actgcccgct 3660

ttccagtcgg gaaacctgtc gtgccagctg cattaatgaa tcggccaacg cgcggggaga 3720

ggcggtttgc gtattgggcg ctcttccgct tcctcgctca ctgactcgct gcgctcggtc 3780

gttcggctgc ggcgagcggt atcagctcac tcaaaggcgg taatacggtt atccacagaa 3840

tcaggggata acgcaggaaa gaacatgtga gcaaaaggcc agcaaaaggc caggaaccgt 3900

aaaaaggccg cgttgctggc gtttttccat aggctccgcc cccctgacga gcatcacaaa 3960

aatcgacgct caagtcagag gtggcgaaac ccgacaggac tataaagata ccaggcgttt 4020

ccccctggaa gctccctcgt gcgctctcct gttccgaccc tgccgcttac cggatacctg 4080

tccgcctttc tcccttcggg aagcgtggcg ctttctcata gctcacgctg taggtatctc 4140

agttcggtgt aggtcgttcg ctccaagctg ggctgtgtgc acgaaccccc cgttcagccc 4200

gaccgctgcg ccttatccgg taactatcgt cttgagtcca acccggtaag acacgactta 4260

tcgccactgg cagcagccac tggtaacagg attagcagag cgaggtatgt aggcggtgct 4320

acagagttct tgaagtggtg gcctaactac ggctacacta gaagaacagt atttggtatc 4380

tgcgctctgc tgaagccagt taccttcgga aaaagagttg gtagctcttg atccggcaaa 4440

caaaccaccg ctggtagcgg tttttttgtt tgcaagcagc agattacgcg cagaaaaaaa 4500

ggatctcaag aagatccttt gatcttttct acggggtctg acgctcagtg gaacgaaaac 4560

tcacgttaag ggattttggt catgagatta tcaaaaagga tcttcaccta gatcctttta 4620

aattaaaaat gaagttttaa atcaatctaa agtatatatg agtaaacttg gtctgacagt 4680

taccaatgct taatcagtga ggcacctatc tcagcgatct gtctatttcg ttcatccata 4740

gttgcctgac tccccgtcgt gtagataact acgatacggg agggcttacc atctggcccc 4800

agtgctgcaa tgataccgcg agacccacgc tcaccggctc cagatttatc agcaataaac 4860

cagccagccg gaagggccga gcgcagaagt ggtcctgcaa ctttatccgc ctccatccag 4920

tctattaatt gttgccggga agctagagta agtagttcgc cagttaatag tttgcgcaac 4980

gttgttgcca ttgctacagg catcgtggtg tcacgctcgt cgtttggtat ggcttcattc 5040

agctccggtt cccaacgatc aaggcgagtt acatgatccc ccatgttgtg caaaaaagcg 5100

gttagctcct tcggtcctcc gatcgttgtc agaagtaagt tggccgcagt gttatcactc 5160

atggttatgg cagcactgca taattctctt actgtcatgc catccgtaag atgcttttct 5220

gtgactggtg agtactcaac caagtcattc tgagaatagt gtatgcggcg accgagttgc 5280

tcttgcccgg cgtcaatacg ggataatacc gcgccacata gcagaacttt aaaagtgctc 5340

atcattggaa aacgttcttc ggggcgaaaa ctctcaagga tcttaccgct gttgagatcc 5400

agttcgatgt aacccactcg tgcacccaac tgatcttcag catcttttac tttcaccagc 5460

gtttctgggt gagcaaaaac aggaaggcaa aatgccgcaa aaaagggaat aagggcgaca 5520

cggaaatgtt gaatactcat actcttcctt tttcaatatt attgaagcat ttatcagggt 5580

tattgtctca tgagcggata catatttgaa tgtatttaga aaaataaaca aataggggtt 5640

ccgcgcacat ttccccgaaa agtgccacct gacgtcgacg gatcggg 5687

<210> 9
<211> 5302
<212> DNA
<213> Artificial

<220>
<223> H1-scaffold_EF1alpha-AcrIIA4-scaffold-BGH (AAV construct)

<400> 9
   tttctacggg gtctgacgct cagtggaacg aaaactcacg ttaagggatt ttggtcatga 60
   gattatcaaa aaggatcttc acctagatcc ttttaaatta aaaatgaagt tttaaatcaa 120
   tctaaagtat atatgagtaa acttggtctg acagttacca atgcttaatc agtgaggcac 180
   ctatctcagc gatctgtcta tttcgttcat ccatagttgc ctgactcccc gtcgtgtaga 240
   taactacgat acgggagggc ttaccatctg gccccagtgc tgcaatgata ccgcgagacc 300
   cacgctcacc ggctccagat ttatcagcaa taaaccagcc agccggaagg gccgagcgca 360
   gaagtggtcc tgcaacttta tccgcctcca tccagtctat taattgttgc cgggaagcta 420
   gagtaagtag ttcgccagtt aatagtttgc gcaacgttgt tgccattgct acaggcatcg 480
   tggtgtcacg ctcgtcgttt ggtatggctt cattcagctc cggttcccaa cgatcaaggc 540
   gagttacatg atcccccatg ttgtgcaaaa aagcggttag ctccttcggt cctccgatcg 600
   ttgtcagaag taagttggcc gcagtgttat cactcatggt tatggcagca ctgcataatt 660
   ctcttactgt catgccatcc gtaagatgct tttctgtgac tggtgagtac tcaaccaagt 720
   cattctgaga atagtgtatg cggcgaccga gttgctcttg cccggcgtca atacgggata 780
   ataccgcgcc acatagcaga actttaaaag tgctcatcat tggaaaacgt tcttcggggc 840
   gaaaactctc aaggatctta ccgctgttga gatccagttc gatgtaaccc actcgtgcac 900
   ccaactgatc ttcagcatct tttactttca ccagcgtttc tgggtgagca aaaacaggaa 960
   ggcaaaatgc cgcaaaaaag ggaataaggg cgacacggaa atgttgaata ctcatactct 1020
   tcctttttca atattattga agcatttatc agggttattg tctcatgagc ggatacatat 1080
   ttgaatgtat ttagaaaaat aaacaaatag gggttccgcg cacatttccc cgaaaagtgc 1140
   cacctaaatt gtaagcgtta atattttgtt aaaattcgcg ttaaattttt gttaaatcag 1200
   ctcatttttt aaccaatagg ccgaaatcgg caaaatccct tataaatcaa aagaatagac 1260
   cgagataggg ttgagtgttg ttccagtttg gaacaagagt ccactattaa agaacgtgga 1320
   ctccaacgtc aaagggcgaa aaaccgtcta tcagggcgat ggcccactac gtgaaccatc 1380
   accctaatca agttttttgg ggtcgaggtg ccgtaaagca ctaaatcgga accctaaagg 1440
   gagcccccga tttagagctt gacggggaaa gccggcgaac gtggcgagaa aggaagggaa 1500
   gaaagcgaaa ggagcgggcg ctagggcgct ggcaagtgta gcggtcacgc tgcgcgtaac 1560
   caccacaccc gccgcgctta atgcgccgct acagggcgcg tcccattcgc cattcaggct 1620
   gcgcaactgt tgggaagggc gatcggtgcg ggcctcttcg ctattacgcc agctggcgaa 1680
   agggggatgt gctgcaaggc gattaagttg ggtaacgcca gggttttccc agtcacgacg 1740
   ttgtaaaacg acggccagtg agcgcgcgta atacgactca ctatagggcg aattggagct 1800
   ctctagaatg cagggggggg gggggggggg ggccactccc tctctgcgcg ctcgctcgct 1860
   cactgaggcc gggcgaccaa aggtcgcccg acgcccgggc tttgcccggg cggcctcagt 1920
   gagcgagcga gcgcgcagag agggagtggc caactccatc actaggggtt cctagatctg 1980
   atatcgtcga ccatagagcc caccgcatcc ccagcatgcc tgctattgtc ttcccaatcc 2040
   tcccccttgc tgtcctgccc caccccaccc cccagaatag aatgacacct actcagacaa 2100
   tgcgatgcaa tttcctcatt ttattaggaa aggacagtgg gagtggcacc ttccagggtc 2160
   aaggaaggca cgggggaggg gcaaacaaca gatggctggc aactagaagg cacagtcgag 2220
   gctgatcagc ggtttaaact taagctagag acgacttcaa catcaacggt atataaactt 2280
   caacatcaac ggcgtctcta attctgcaga tatccagcac agtggcggcc gctcgagcta 2340
   gttcagctca gatttcagtg tgatagtttg catatcattg tagaactctt cctcatcttc 2400
   gtactcctgg ttccagccgt ttttaaatgc actgatgaat ttctccacga ttgattcatt 2460
   ctcggattca gagatgacat attcgttgcc atcattatta acccttataa tgagctgtgt 2520
   tatagagttg gagtcggtgc ctgacagctt gaccgtgtag tccttgttct ttatttcccg 2580
   aatcaggtcg ttaatgttca tggtggcgga tccgctaatt cctcacgaca cctgaaatgg 2640
   aagaaaaaaa ctttgaacca ctgtctgagg cttgagaatg aaccaagatc caaactcaaa 2700
   aagggcaaat tccaaggaga attacatcaa gtgccaagct ggcctaactt cagtctccac 2760
   ccactcagtg tggggaaact ccatcgcata aaacccctcc ccccaaccta aagacgacgt 2820
   actccaaaag ctcgagaact aatcgaggtg cctggacggc gcccggtact ccgtggagtc 2880
   acatgaagcg acggctgagg acggaaaggc ccttttcctt tgtgtgggtg actcacccgc 2940
   ccgctctccc gagcgccgcg tcctccattt tgagctccct gcagcagggc cgggaagcgg 3000
   ccatctttcc gctcacgcaa ctggtgccga ccgggccagc cttgccgccc agggcggggc 3060
   gatacacggc ggcgcgaggc caggcaccag agcaggccgg ccagcttgag actacccccg 3120
   tccgattctc ggtggccgcg ctcgcaggcc ccgcctcgcc gaacatgtgc gctgggacgc 3180
   acgggccccg tcgccgcccg cggccccaaa aaccgaaata ccagtgtgca gatcttggcc 3240
   cgcatttaca agactatctt gccagaaaaa aagcgtcgca gcaggtcatc aaaaatttta 3300
   aatggctaga gacttatcga aagcagcgag acaggcgcga aggtgccacc agattcgcac 3360
   gcggcggccc cagcgcccag gccaggcctc aactcaagca cgaggcgaag gggctcctta 3420
   agcgcaaggc ctcgaactct cccacccact tccaacccga agctcgggat caagaatcac 3480
   gtactgcagc caggtggaag taattcaagg cacgcaaggg ccataacccg taaagaggcc 3540
   aggcccgcgg gaaccacaca cggcacttac ctgtgttctg gcggcaaacc cgttgcgaaa 3600
   aagaacgttc acggcgacta ctgcacttat atacggttct cccccaccct cgggaaaaag 3660
   gcggagccag tacacgacat cactttccca gtttaccccg cgccaccttc tctaggcacc 3720
   ggttcaattg ccgacccctc cccccaactt ctcggggact gtgggcgatg tgcgctctgc 3780
   ccactgacgg gcaccggagc ctcacgggcc gcaaaaaaag caccgactcg gtgccacttt 3840
   ttcaagttga taacggacta gccttattta aacttgctat gctgtttcca gcatagctct 3900
   taaacctgtc ttcctcgaag actcggtgta ccgtggtctc atacagaact tataagattc 3960
   ccaaatccaa agacatttca cgtttatggt gatttcccag aacacatagc gacatgcaaa 4020
   tatgggcgcg ccgatatcag atctggcaaa cctagatgat ggaggtaccc actccctcta 4080
   tgcgcgctcg ctcactcact cggccctgcc ggccagaggc cggcagtctg gagacctttg 4140
   gtctccaggg ccgagtgagt gagcgagcgc gcatagaggg agtgggtagg acgcgtcctg 4200
   caggatgcat actagtggta cccagctttt gttcccttta gtgagggtta attgcgcgct 4260
   tggcgtaatc atggtcatag ctgtttcctg tgtgaaattg ttatccgctc acaattccac 4320
   acaacatacg agccggaagc ataaagtgta aagcctgggg tgcctaatga gtgagctaac 4380
   tcacattaat tgcgttgcgc tcactgcccg ctttccagtc gggaaacctg tcgtgccagc 4440
   tgcattaatg aatcggccaa cgcgcgggga gaggcggttt gcgtattggg cgctcttccg 4500
   cttcctcgct cactgactcg ctgcgctcgg tcgttcggct gcggcgagcg gtatcagctc 4560
   actcaaaggc ggtaatacgg ttatccacag aatcagggga taacgcagga aagaacatgt 4620
   gagcaaaagg ccagcaaaag gccaggaacc gtaaaaaggc cgcgttgctg gcgtttttcc 4680
   ataggctccg cccccctgac gagcatcaca aaaatcgacg ctcaagtcag aggtggcgaa 4740
   acccgacagg actataaaga taccaggcgt ttccccctgg aagctccctc gtgcgctctc 4800
   ctgttccgac cctgccgctt accggatacc tgtccgcctt tctcccttcg ggaagcgtgg 4860
   cgctttctca tagctcacgc tgtaggtatc tcagttcggt gtaggtcgtt cgctccaagc 4920
   tgggctgtgt gcacgaaccc cccgttcagc ccgaccgctg cgccttatcc ggtaactatc 4980
   gtcttgagtc caacccggta agacacgact tatcgccact ggcagcagcc actggtaaca 5040
   ggattagcag agcgaggtat gtaggcggtg ctacagagtt cttgaagtgg tggcctaact 5100
   acggctacac tagaaggaca gtatttggta tctgcgctct gctgaagcca gttaccttcg 5160
   gaaaaagagt tggtagctct tgatccggca aacaaaccac cgctggtagc ggtggttttt 5220
   ttgtttgcaa gcagcagatt acgcgcagaa aaaaaggatc tcaagaagat cctttgatct 5280
   tctcaagaag atcctttgat ct                                            5302

<210> 10
<211> 5302
<212> DNA
<213> Artificial

<220>
<223> H1-scaffold_EF1alpha-AcrIIA4-2xmir122BS-BGH (AAV construct)

<400> 10
agatcaaagg atcttcttga gatccttttt ttctgcgcgt aatctgctgc ttgcaaacaa 60

aaaaaccacc gctaccagcg gtggtttgtt tgccggatca agagctacca actctttttc 120

cgaaggtaac tggcttcagc agagcgcaga taccaaatac tgtccttcta gtgtagccgt 180

agttaggcca ccacttcaag aactctgtag caccgcctac atacctcgct ctgctaatcc 240

tgttaccagt ggctgctgcc agtggcgata agtcgtgtct taccgggttg gactcaagac 300

gatagttacc ggataaggcg cagcggtcgg gctgaacggg gggttcgtgc acacagccca 360

gcttggagcg aacgacctac accgaactga gatacctaca gcgtgagcta tgagaaagcg 420

ccacgcttcc cgaagggaga aaggcggaca ggtatccggt aagcggcagg gtcggaacag 480

gagagcgcac gagggagctt ccagggggaa acgcctggta tctttatagt cctgtcgggt 540

aatatgtata gtcctgtcgg gtttcgccac ctctgacttg agcgtcgatt tttgtgatgc 600

tcgtcagggg ggcggagcct atggaaaaac gccagcaacg cggccttttt acggttcctg 660

gccttttgct ggccttttgc tcacatgttc tttcctgcgt tatcccctga ttctgtggat 720

aaccgtatta ccgcctttga gtgagctgat accgctcgcc gcagccgaac gaccgagcgc 780

agcgagtcag tgagcgagga agcggaagag cgcccaatac gcaaaccgcc tctccccgcg 840

cgttggccga ttcattaatg cagctggcac gacaggtttc ccgactggaa agcgggcagt 900

gagcgcaacg caattaatgt gagttagctc actcattagg caccccaggc tttacacttt 960

atgcttccgg ctcgtatgtt gtgtggaatt gtgagcggat aacaatttca cacaggaaac 1020

agctatgacc atgattacgc caagcgcgca attaaccctc actaaaggga acaaaagctg 1080

ggtaccacta gtatgcatcc tgcaggacgc gtcctaccca ctccctctat gcgcgctcgc 1140

tcactcactc ggccctggag accaaaggtc tccagactgc cggcctctgg ccggcagggc 1200

cgagtgagtg agcgagcgcg catagaggga gtgggtacct ccatcatcta ggtttgccag 1260

atctgatatc ggcgcgccca tatttgcatg tcgctatgtg ttctgggaaa tcaccataaa 1320

cgtgaaatgt ctttggattt gggaatctta taagttctgt atgagaccac ggtacaccga 1380

gtcttcgagg aagacaggtt taagagctat gctggaaaca gcatagcaag tttaaataag 1440

gctagtccgt tatcaacttg aaaaagtggc accgagtcgg tgcttttttt gcggcccgtg 1500

aggctccggt gcccgtcagt gggcagagcg cacatcgccc acagtccccg agaagttggg 1560

gggaggggtc ggcaattgaa ccggtgccta gagaaggtgg cgcggggtaa actgggaaag 1620

tgatgtcgtg tactggctcc gcctttttcc cgagggtggg ggagaaccgt atataagtgc 1680

agtagtcgcc gtgaacgttc tttttcgcaa cgggtttgcc gccagaacac aggtaagtgc 1740

cgtgtgtggt tcccgcgggc ctggcctctt tacgggttat ggcccttgcg tgccttgaat 1800

tacttccacc tggctgcagt acgtgattct tgatcccgag cttcgggttg gaagtgggtg 1860

ggagagttcg aggccttgcg cttaaggagc cccttcgcct cgtgcttgag ttgaggcctg 1920

gcctgggcgc tggggccgcc gcgtgcgaat ctggtggcac cttcgcgcct gtctcgctgc 1980

tttcgataag tctctagcca tttaaaattt ttgatgacct gctgcgacgc tttttttctg 2040

gcaagatagt cttgtaaatg cgggccaaga tctgcacact ggtatttcgg tttttggggc 2100

cgcgggcggc gacggggccc gtgcgtccca gcgcacatgt tcggcgaggc ggggcctgcg 2160

agcgcggcca ccgagaatcg gacgggggta gtctcaagct ggccggcctg ctctggtgcc 2220

tggcctcgcg ccgccgtgta tcgccccgcc ctgggcggca aggctggccc ggtcggcacc 2280

agttgcgtga gcggaaagat ggccgcttcc cggccctgct gcagggagct caaaatggag 2340

gacgcggcgc tcgggagagc gggcgggtga gtcacccaca caaaggaaaa gggcctttcc 2400

gtcctcagcc gtcgcttcat gtgactccac ggagtaccgg gcgccgtcca ggcacctcga 2460

ttagttctcg agcttttgga gtacgtcgtc tttaggttgg ggggaggggt tttatgcgat 2520

ggagtttccc cacactgagt gggtggagac tgaagttagg ccagcttggc acttgatgta 2580

attctccttg gaatttgccc tttttgagtt tggatcttgg ttcattctca agcctcagac 2640

agtggttcaa agtttttttc ttccatttca ggtgtcgtga ggaattagcg gatccgccac 2700

catgaacatt aacgacctga ttcgggaaat aaagaacaag gactacacgg tcaagctgtc 2760

aggcaccgac tccaactcta taacacagct cattataagg gttaataatg atggcaacga 2820

atatgtcatc tctgaatccg agaatgaatc aatcgtggag aaattcatca gtgcatttaa 2880

aaacggctgg aaccaggagt acgaagatga ggaagagttc tacaatgata tgcaaactat 2940

cacactgaaa tctgagctga actagctcga gcggccgcca ctgtgctgga tatctgcaga 3000

attacaaaca ccattgtcac actccattat atacaaacac cattgtcaca ctccatagct 3060

taagtttaaa ccgctgatca gcctcgactg tgccttctag ttgccagcca tctgttgttt 3120

gcccctcccc cgtgccttcc ttgaccctgg aaggtgccac tcccactgtc ctttcctaat 3180

aaaatgagga aattgcatcg cattgtctga gtaggtgtca ttctattctg gggggtgggg 3240

tggggcagga cagcaagggg gaggattggg aagacaatag caggcatgct ggggatgcgg 3300

tgggctctat ggtcgacgat atcagatcta ggaaccccta gtgatggagt tggccactcc 3360

ctctctgcgc gctcgctcgc tcactgaggc cgcccgggca aagcccgggc gtcgggcgac 3420

ctttggtcgc ccggcctcag tgagcgagcg agcgcgcaga gagggagtgg cccccccccc 3480

cccccccccc tgcattctag agagctccaa ttcgccctat agtgagtcgt attacgcgcg 3540

ctcactggcc gtcgttttac aacgtcgtga ctgggaaaac cctggcgtta cccaacttaa 3600

tcgccttgca gcacatcccc ctttcgccag ctggcgtaat agcgaagagg cccgcaccga 3660

tcgcccttcc caacagttgc gcagcctgaa tggcgaatgg gacgcgccct gtagcggcgc 3720

attaagcgcg gcgggtgtgg tggttacgcg cagcgtgacc gctacacttg ccagcgccct 3780

agcgcccgct cctttcgctt tcttcccttc ctttctcgcc acgttcgccg gctttccccg 3840

tcaagctcta aatcgggggc tccctttagg gttccgattt agtgctttac ggcacctcga 3900

ccccaaaaaa cttgattagg gtgatggttc acgtagtggg ccatcgccct gatagacggt 3960

ttttcgccct ttgacgttgg agtccacgtt ctttaatagt ggactcttgt tccaaactgg 4020

aacaacactc aaccctatct cggtctattc ttttgattta taagggattt tgccgatttc 4080

ggcctattgg ttaaaaaatg agctgattta acaaaaattt aacgcgaatt ttaacaaaat 4140

attaacgctt acaatttagg tggcactttt cggggaaatg tgcgcggaac ccctatttgt 4200

ttatttttct aaatacattc aaatatgtat ccgctcatga gacaataacc ctgataaatg 4260

cttcaataat attgaaaaag gaagagtatg agtattcaac atttccgtgt cgcccttatt 4320

cccttttttg cggcattttg ccttcctgtt tttgctcacc cagaaacgct ggtgaaagta 4380

aaagatgctg aagatcagtt gggtgcacga gtgggttaca tcgaactgga tctcaacagc 4440

ggtaagatcc ttgagagttt tcgccccgaa gaacgttttc caatgatgag cacttttaaa 4500

gttctgctat gtggcgcggt attatcccgt attgacgccg ggcaagagca actcggtcgc 4560

cgcatacact attctcagaa tgacttggtt gagtactcac cagtcacaga aaagcatctt 4620

acggatggca tgacagtaag agaattatgc agtgctgcca taaccatgag tgataacact 4680

gcggccaact tacttctgac aacgatcgga ggaccgaagg agctaaccgc ttttttgcac 4740

aacatggggg atcatgtaac tcgccttgat cgttgggaac cggagctgaa tgaagccata 4800

ccaaacgacg agcgtgacac cacgatgcct gtagcaatgg caacaacgtt gcgcaaacta 4860

ttaactggcg aactacttac tctagcttcc cggcaacaat taatagactg gatggaggcg 4920

gataaagttg caggaccact tctgcgctcg gcccttccgg ctggctggtt tattgctgat 4980

aaatctggag ccggtgagcg tgggtctcgc ggtatcattg cagcactggg gccagatggt 5040

aagccctccc gtatcgtagt tatctacacg acggggagtc aggcaactat ggatgaacga 5100

aatagacaga tcgctgagat aggtgcctca ctgattaagc attggtaact gtcagaccaa 5160

gtttactcat atatacttta gattgattta aaacttcatt tttaatttaa aaggatctag 5220

gtgaagatcc tttttgataa tctcatgacc aaaatccctt aacgtgagtt ttcgttccac 5280

tgagcgtcag accccgtaga aa 5302

<210> 11
<211> 4693
<212> DNA
<213> Artificial

<220>
<223> U6-scaffold_EFS-AcrIIA4-scaffold-BGH (AAV construct)

<400> 11
ctaaattgta agcgttaata ttttgttaaa attcgcgtta aatttttgtt aaatcagctc 60

attttttaac caataggccg aaatcggcaa aatcccttat aaatcaaaag aatagaccga 120

gatagggttg agtgttgttc cagtttggaa caagagtcca ctattaaaga acgtggactc 180

caacgtcaaa gggcgaaaaa ccgtctatca gggcgatggc ccactacgtg aaccatcacc 240

ctaatcaagt tttttggggt cgaggtgccg taaagcacta aatcggaacc ctaaagggag 300

cccccgattt agagcttgac ggggaaagcc ggcgaacgtg gcgagaaagg aagggaagaa 360

agcgaaagga gcgggcgcta gggcgctggc aagtgtagcg gtcacgctgc gcgtaaccac 420

cacacccgcc gcgcttaatg cgccgctaca gggcgcgtcc cattcgccat tcaggctgcg 480

caactgttgg gaagggcgat cggtgcgggc ctcttcgcta ttacgccagc tggcgaaagg 540

gggatgtgct gcaaggcgat taagttgggt aacgccaggg ttttcccagt cacgacgttg 600

taaaacgacg gccagtgagc gcgcgtaata cgactcacta tagggcgaat tggagctctc 660

tagaatgcag gggggggggg gggggggggc cactccctct ctgcgcgctc gctcgctcac 720

tgaggccggg cgaccaaagg tcgcccgacg cccgggcttt gcccgggcgg cctcagtgag 780

cgagcgagcg cgcagagagg gagtggccaa ctccatcact aggggttcct agatctgata 840

tcgtcgacca tagagcccac cgcatcccca gcatgcctgc tattgtcttc ccaatcctcc 900

cccttgctgt cctgccccac cccacccccc agaatagaat gacacctact cagacaatgc 960

gatgcaattt cctcatttta ttaggaaagg acagtgggag tggcaccttc cagggtcaag 1020

gaaggcacgg gggaggggca aacaacagat ggctggcaac tagaaggcac agtcgaggct 1080

gatcagcggt ttaaacttaa gctagagacg acttcaacat caacggtata taaacttcaa 1140

catcaacggc gtctctaatt ctgcagatat ccagcacagt ggcggccgct cgagctagtt 1200

cagctcagat ttcagtgtga tagtttgcat atcattgtag aactcttcct catcttcgta 1260

ctcctggttc cagccgtttt taaatgcact gatgaatttc tccacgattg attcattctc 1320

ggattcagag atgacatatt cgttgccatc attattaacc cttataatga gctgtgttat 1380

agagttggag tcggtgcctg acagcttgac cgtgtagtcc ttgttcttta tttcccgaat 1440

caggtcgtta atgttcatgg tggcggatcc ctgtgttctg gcggcaaacc cgttgcgaaa 1500

aagaacgttc acggcgacta ctgcacttat atacggttct cccccaccct cgggaaaaag 1560

gcggagccag tacacgacat cactttccca gtttaccccg cgccaccttc tctaggcacc 1620

ggatcaattg ccgacccctc cccccaactt ctcggggact gtgggcgatg tgcgctctgc 1680

ccactgacgg gcaccggagc ggccgcaaaa aaagcaccga ctcggtgcca ctttttcaag 1740

ttgataacgg actagcctta ttttaacttg ctatgctgtt tccagcatag ctcttaaacc 1800

tgtcttcctc gaagactcgg tgtttcgtcc tttccacaag atatataaag ccaagaaatc 1860

gaaatacttt caagttacgg taagcatatg atagtccatt ttaaaacata attttaaaac 1920

tgcaaactac ccaagaaatt attactttct acgtcacgta ttttgtacta atatctttgt 1980

gtttacagtc aaattaattc taattatctc tctaacagcc ttgtatcgta tatgcaaata 2040

tgaaggaatc atgggaaata ggccctcttc ctgcccgacc ttggcgcgcg ctcggcgcgc 2100

ggtcacgctc cgtcacgtgg tgcgttttgc ctgcgcgtct ttccactggg gccggggcgg 2160

cgcggagggt tcagacgcgg gctcttactc aaacgctgat ccccgaaact accgagcagt 2220

gtctgcgcga cgcgaaagca tttttggagc gaggggccat ggtgcccatg ggattcccac 2280

gggtgttgga ctcgggcgcg ccgatatcag atctggcaaa cctagatgat ggaggtaccc 2340
actccctcta tgcgcgctcg ctcactcact cggccctgcc ggccagaggc cggcagtctg 2400
gagacctttg gtctccaggg ccgagtgagt gagcgagcgc gcatagaggg agtgggtagg 2460
acgcgtcctg caggatgcat actagtggta cccagctttt gttcccttta gtgagggtta 2520
attgcgcgct tggcgtaatc atggtcatag ctgtttcctg tgtgaaattg ttatccgctc 2580
acaattccac acaacatacg agccggaagc ataaagtgta aagcctgggg tgcctaatga 2640
gtgagctaac tcacattaat tgcgttgcgc tcactgcccg ctttccagtc gggaaacctg 2700
tcgtgccagc tgcattaatg aatcggccaa cgcgcgggga gaggcggttt gcgtattggg 2760
cgctcttccg cttcctcgct cactgactcg ctgcgctcgg tcgttcggct gcggcgagcg 2820
gtatcagctc actcaaaggc ggtaatacgg ttatccacag aatcagggga taacgcagga 2880
aagaacatgt gagcaaaagg ccagcaaaag gccaggaacc gtaaaaaggc cgcgttgctg 2940
gcgtttttcc ataggctccg cccccctgac gagcatcaca aaaatcgacg ctcaagtcag 3000
aggtggcgaa acccgacagg actataaaga taccaggcgt ttccccctgg aagctccctc 3060
gtgcgctctc ctgttccgac cctgccgctt accggatacc tgtccgcctt tctcccttcg 3120
ggaagcgtgg cgctttctca tagctcacgc tgtaggtatc tcagttcggt gtaggtcgtt 3180
cgctccaagc tgggctgtgt gcacgaaccc cccgttcagc ccgaccgctg cgccttatcc 3240
ggtaactatc gtcttgagtc caacccggta agacacgact tatcgccact ggcagcagcc 3300
actggtaaca ggattagcag agcgaggtat gtaggcggtg ctacagagtt cttgaagtgg 3360
tggcctaact acggctacac tagaaggaca gtatttggta tctgcgctct gctgaagcca 3420
gttaccttcg gaaaaagagt tggtagctct tgatccggca aacaaaccac cgctggtagc 3480
ggtggttttt ttgtttgcaa gcagcagatt acgcgcagaa aaaaaggatc tcaagaagat 3540
cctttgatct tttctacggg gtctgacgct cagtggaacg aaaactcacg ttaagggatt 3600
ttggtcatga gattatcaaa aaggatcttc acctagatcc ttttaaatta aaaatgaagt 3660
tttaaatcaa tctaaagtat atatgagtaa acttggtctg acagttacca atgcttaatc 3720
agtgaggcac ctatctcagc gatctgtcta tttcgttcat ccatagttgc ctgactcccc 3780
gtcgtgtaga taactacgat acgggagggc ttaccatctg gccccagtgc tgcaatgata 3840
ccgcgagacc cacgctcacc ggctccagat ttatcagcaa taaaccagcc agccggaagg 3900
gccgagcgca gaagtggtcc tgcaacttta tccgcctcca tccagtctat taattgttgc 3960
cgggaagcta gagtaagtag ttcgccagtt aatagtttgc gcaacgttgt tgccattgct 4020
acaggcatcg tggtgtcacg ctcgtcgttt ggtatggctt cattcagctc cggttcccaa 4080
cgatcaaggc gagttacatg atcccccatg ttgtgcaaaa aagcggttag ctccttcggt 4140
cctccgatcg ttgtcagaag taagttggcc gcagtgttat cactcatggt tatggcagca 4200

ctgcataatt ctcttactgt catgccatcc gtaagatgct tttctgtgac tggtgagtac 4260

tcaaccaagt cattctgaga atagtgtatg cggcgaccga gttgctcttg cccggcgtca 4320

atacgggata ataccgcgcc acatagcaga actttaaaag tgctcatcat tggaaaacgt 4380

tcttcggggc gaaaactctc aaggatctta ccgctgttga gatccagttc gatgtaaccc 4440

actcgtgcac ccaactgatc ttcagcatct tttactttca ccagcgtttc tgggtgagca 4500

aaaacaggaa ggcaaaatgc cgcaaaaaag ggaataaggg cgacacggaa atgttgaata 4560

ctcatactct tcctttttca atattattga agcatttatc agggttattg tctcatgagc 4620

ggatacatat ttgaatgtat ttagaaaaat aaacaaatag gggttccgcg cacatttccc 4680

cgaaaagtgc cac 4693

<210> 12
<211> 4693
<212> DNA
<213> Artificial

<220>
<223> U6-scaffold_EFS-AcrIIA4-2xmir122BS-BGH (AAV construct)

<400> 12
ctaaattgta agcgttaata ttttgttaaa attcgcgtta aatttttgtt aaatcagctc 60

attttttaac caataggccg aaatcggcaa aatcccttat aaatcaaaag aatagaccga 120

gatagggttg agtgttgttc cagtttggaa caagagtcca ctattaaaga acgtggactc 180

caacgtcaaa gggcgaaaaa ccgtctatca gggcgatggc ccactacgtg aaccatcacc 240

ctaatcaagt tttttggggt cgaggtgccg taaagcacta aatcggaacc ctaaagggag 300

cccccgattt agagcttgac ggggaaagcc ggcgaacgtg gcgagaaagg aagggaagaa 360

agcgaaagga gcgggcgcta gggcgctggc aagtgtagcg gtcacgctgc gcgtaaccac 420

cacacccgcc gcgcttaatg cgccgctaca gggcgcgtcc cattcgccat tcaggctgcg 480

caactgttgg gaagggcgat cggtgcgggc ctcttcgcta ttacgccagc tggcgaaagg 540

gggatgtgct gcaaggcgat taagttgggt aacgccaggg ttttcccagt cacgacgttg 600

taaaacgacg gccagtgagc gcgcgtaata cgactcacta tagggcgaat tggagctctc 660

tagaatgcag gggggggggg gggggggggc cactccctct ctgcgcgctc gctcgctcac 720

tgaggccggg cgaccaaagg tcgcccgacg cccgggcttt gcccgggcgg cctcagtgag 780

cgagcgagcg cgcagagagg gagtggccaa ctccatcact aggggttcct agatctgata 840

tcgtcgacca tagagcccac cgcatcccca gcatgcctgc tattgtcttc ccaatcctcc 900

cccttgctgt cctgccccac cccacccccc agaatagaat gacacctact cagacaatgc 960

gatgcaattt cctcatttta ttaggaaagg acagtgggag tggcaccttc cagggtcaag 1020

gaaggcacgg gggaggggca aacaacagat ggctggcaac tagaaggcac agtcgaggct 1080

gatcagcggt ttaaacttaa gctatggagt gtgacaatgg tgtttgtata taatggagtg 1140

tgacaatggt gtttgtaatt ctgcagatat ccagcacagt ggcggccgct cgagctagtt 1200

cagctcagat ttcagtgtga tagtttgcat atcattgtag aactcttcct catcttcgta 1260

ctcctggttc cagccgtttt taaatgcact gatgaatttc tccacgattg attcattctc 1320

ggattcagag atgacatatt cgttgccatc attattaacc cttataatga gctgtgttat 1380

agagttggag tcggtgcctg acagcttgac cgtgtagtcc ttgttcttta tttcccgaat 1440

caggtcgtta atgttcatgg tggcggatcc ctgtgttctg gcggcaaacc cgttgcgaaa 1500

aagaacgttc acggcgacta ctgcacttat atacggttct cccccaccct cgggaaaaag 1560

gcggagccag tacacgacat cactttccca gtttaccccg cgccaccttc tctaggcacc 1620

ggatcaattg ccgacccctc cccccaactt ctcggggact gtgggcgatg tgcgctctgc 1680

ccactgacgg gcaccggagc ggccgcaaaa aaagcaccga ctcggtgcca ctttttcaag 1740

ttgataacgg actagcctta ttttaacttg ctatgctgtt tccagcatag ctcttaaacc 1800

tgtcttcctc gaagactcgg tgtttcgtcc tttccacaag atatataaag ccaagaaatc 1860

gaaatacttt caagttacgg taagcatatg atagtccatt ttaaaacata attttaaaac 1920

tgcaaactac ccaagaaatt attactttct acgtcacgta ttttgtacta atatctttgt 1980

gtttacagtc aaattaattc taattatctc tctaacagcc ttgtatcgta tatgcaaata 2040

tgaaggaatc atgggaaata ggccctcttc ctgcccgacc ttggcgcgcg ctcggcgcgc 2100

ggtcacgctc cgtcacgtgg tgcgttttgc ctgcgcgtct ttccactggg gccggggcgg 2160

cgcggagggt tcagacgcgg gctcttactc aaacgctgat ccccgaaact accgagcagt 2220

gtctgcgcga cgcgaaagca tttttggagc gaggggccat ggtgcccatg ggattcccac 2280

gggtgttgga ctcgggcgcg ccgatatcag atctggcaaa cctagatgat ggaggtaccc 2340

actccctcta tgcgcgctcg ctcactcact cggccctgcc ggccagaggc cggcagtctg 2400

gagacctttg gtctccaggg ccgagtgagt gagcgagcgc gcatagaggg agtgggtagg 2460

acgcgtcctg caggatgcat actagtggta cccagctttt gttcccttta gtgagggtta 2520

attgcgcgct tggcgtaatc atggtcatag ctgtttcctg tgtgaaattg ttatccgctc 2580

acaattccac acaacatacg agccggaagc ataaagtgta aagcctgggg tgcctaatga 2640

gtgagctaac tcacattaat tgcgttgcgc tcactgcccg ctttccagtc gggaaacctg 2700

tcgtgccagc tgcattaatg aatcggccaa cgcgcgggga gaggcggttt gcgtattggg 2760

cgctcttccg cttcctcgct cactgactcg ctgcgctcgg tcgttcggct gcggcgagcg 2820

gtatcagctc actcaaaggc ggtaatacgg ttatccacag aatcagggga taacgcagga 2880

aagaacatgt gagcaaaagg ccagcaaaag gccaggaacc gtaaaaaggc cgcgttgctg 2940

gcgtttttcc ataggctccg cccccctgac gagcatcaca aaaatcgacg ctcaagtcag 3000

aggtggcgaa acccgacagg actataaaga taccaggcgt ttccccctgg aagctccctc 3060

gtgcgctctc ctgttccgac cctgccgctt accggatacc tgtccgcctt tctcccttcg 3120

ggaagcgtgg cgctttctca tagctcacgc tgtaggtatc tcagttcggt gtaggtcgtt 3180

cgctccaagc tgggctgtgt gcacgaaccc cccgttcagc ccgaccgctg cgccttatcc 3240

ggtaactatc gtcttgagtc caacccggta agacacgact tatcgccact ggcagcagcc 3300

actggtaaca ggattagcag agcgaggtat gtaggcggtg ctacagagtt cttgaagtgg 3360

tggcctaact acggctacac tagaaggaca gtatttggta tctgcgctct gctgaagcca 3420

gttaccttcg gaaaaagagt tggtagctct tgatccggca aacaaaccac cgctggtagc 3480

ggtggttttt ttgtttgcaa gcagcagatt acgcgcagaa aaaaaggatc tcaagaagat 3540

cctttgatct tttctacggg gtctgacgct cagtggaacg aaaactcacg ttaagggatt 3600

ttggtcatga gattatcaaa aaggatcttc acctagatcc ttttaaatta aaaatgaagt 3660

tttaaatcaa tctaaagtat atatgagtaa acttggtctg acagttacca atgcttaatc 3720

agtgaggcac ctatctcagc gatctgtcta tttcgttcat ccatagttgc ctgactcccc 3780

gtcgtgtaga taactacgat acgggagggc ttaccatctg gccccagtgc tgcaatgata 3840

ccgcgagacc cacgctcacc ggctccagat ttatcagcaa taaaccagcc agccggaagg 3900

gccgagcgca gaagtggtcc tgcaacttta tccgcctcca tccagtctat taattgttgc 3960

cgggaagcta gagtaagtag ttcgccagtt aatagtttgc gcaacgttgt tgccattgct 4020

acaggcatcg tggtgtcacg ctcgtcgttt ggtatggctt cattcagctc cggttcccaa 4080

cgatcaaggc gagttacatg atcccccatg ttgtgcaaaa aagcggttag ctccttcggt 4140

cctccgatcg ttgtcagaag taagttggcc gcagtgttat cactcatggt tatggcagca 4200

ctgcataatt ctcttactgt catgccatcc gtaagatgct tttctgtgac tggtgagtac 4260

tcaaccaagt cattctgaga atagtgtatg cggcgaccga gttgctcttg cccggcgtca 4320

atacgggata ataccgcgcc acatagcaga actttaaaag tgctcatcat tggaaaacgt 4380

tcttcggggc gaaaactctc aaggatctta ccgctgttga gatccagttc gatgtaaccc 4440

actcgtgcac ccaactgatc ttcagcatct tttactttca ccagcgtttc tgggtgagca 4500

aaaacaggaa ggcaaaatgc cgcaaaaaag ggaataaggg cgacacggaa atgttgaata 4560

ctcatactct tcctttttca atattattga agcatttatc agggttattg tctcatgagc 4620

ggatacatat ttgaatgtat ttagaaaaat aaacaaatag gggttccgcg cacatttccc 4680

cgaaaagtgc cac 4693

<210> 13

<211>  7475
<212>  DNA
<213>  Artificial

<220>
<223>  N-Cas9-Intein_U6short-scaffold (AAV construct)

<400>  13
ggccgcggaa tgtgtgtcag ttagggtgtg gaaagtcccc aggctcccca gcaggcagaa      60

gtatgcaaag catgcatctc aattagtcag caaccagtcc cggtctcctc ccatgcatgt     120

caatattggc cattagccat attattcatt ggttatatag cataaatcaa tattggctat     180

tggccattgc atacgttgta tctatatcat aatatgtaca tttatattgg ctcatgtcca     240

atatgaccgc catgttggca ttgattattg actagttatt aatagtaatc aattacgggg     300

tcattagttc atagcccata tatggagttc cgcgttacat aacttacggt aaatggcccg     360

cctggctgac cgcccaacga cccccgccca ttgacgtcaa taatgacgta tgttcccata     420

gtaacgccaa tagggacttt ccattgacgt caatgggtgg agtatttacg gtaaactgcc     480

cacttggcag tacatcaagt gtatcatatg ccaagtccgc cccctattga cgtcaatgac     540

ggtaaatggc ccgcctggca ttatgcccag tacatgacct tacgggactt tcctacttgg     600

cagtacatct acgtattagt catcgctatt accatggtga tgcggttttg gcagtacacc     660

aatgggcgtg gatagcggtt tgactcacgg ggatttccaa gtctccaccc cattgacgtc     720

aatgggagtt tgttttggca ccaaaatcaa cgggactttc caaaatgtcg taataacccc     780

gccccgttga cgcaaatggg cggtaggcgt gtacggtggg aggtctatat aagcagaggt     840

cgtttagtga accgtcagat cactagtagc tttattgcgg tagtttatca cagttaaatt     900

gctaacgcag tcagtgctcg actgatcaca ggtaagtatc aaggttacaa gacaggttta     960

aggaggccaa tagaaactgg gcttgtcgag acagagaaga ttcttgcgtt tctgataggc    1020

acctattggt cttactgaca tccactttgc ctttctctcc acaggggtac cgaagccgct    1080

agcgctaccg gtcgccacca tggccccaaa gaagaagcgg aaggtcggta tccacggagt    1140

cccagcagcc gacaagaagt actccattgg gctcgatatc ggcacaaaca gcgtcggctg    1200

ggccgtcatt acggacgagt acaaggtgcc gagcaaaaaa ttcaaagttc tgggcaatac    1260

cgatcgccac agcataaaga agaacctcat tggcgccctc ctgttcgact ccggggaaac    1320

ggccgaagcc acgcggctca aaagaacagc acggcgcaga tatacccgca gaaagaatcg    1380

gatctgctac ctgcaggaga tctttagtaa tgagatggct aaggtggatg actctttctt    1440

ccataggctg gaggagtcct ttttggtgga ggaggataaa aagcacgagc gccacccaat    1500

ctttggcaat atcgtggacg aggtggcgta ccatgaaaag tacccaacca tatatcatct    1560

gaggaagaag cttgtagaca gtactgataa ggctgacttg cggttgatct atctcgcgct    1620

ggcgcatatg atcaaatttc ggggacactt cctcatcgag ggggacctga acccagacaa    1680

cagcgatgtc gacaaactct ttatccaact ggttcagact tacaatcagc ttttcgaaga 1740
gaacccgatc aacgcatccg gagttgacgc caaagcaatc ctgagcgcta ggctgtccaa 1800
atcccggcgg ctcgaaaacc tcatcgcaca gctccctggg gagaagaaga acggcctgtt 1860
tggtaatctt atcgccctgt cactcgggct gacccccaac tttaaatcta acttcgacct 1920
ggccgaagat gccaagcttc aactgagcaa agacacctac gatgatgatc tcgacaatct 1980
gctggcccag atcggcgacc agtacgcaga cctttttttg gcggcaaaga acctgtcaga 2040
cgccattctg ctgagtgata ttctgcgagt gaacacggag atcaccaaag ctccgctgag 2100
cgctagtatg atcaagcgct atgatgagca ccaccaagac ttgactttgc tgaaggccct 2160
tgtcagacag caactgcctg agaagtacaa ggaaattttc ttcgatcagt ctaaaaatgg 2220
ctacgccgga tacattgacg gcggagcaag ccaggaggaa ttttacaaat ttattaagcc 2280
catcttggaa aaaatggacg gcaccgagga gctgctggta aagcttaaca gagaagatct 2340
gttgcgcaaa cagcgcactt tcgacaatgg aagcatcccc caccagattc acctgggcga 2400
actgcacgct atcctcaggc ggcaagagga tttctacccc tttttgaaag ataacaggga 2460
aaagattgag aaaatcctca catttcggat accctactat gtaggccccc tcgcccgggg 2520
aaattccaga ttcgcgtgga tgactcgcaa atcagaagag accatcactc cctggaactt 2580
cgaggaagtc gtggataagg gggcctctgc ccagtccttc atcgaaagga tgactaactt 2640
tgataaaaat ctgcctaacg aaaaggtgct tcctaaacac tctctgctgt acgagtactt 2700
cacagtttat aacgagctca ccaaggtcaa atacgtcaca gaagggatga gaaagccagc 2760
attcctgtct ggagagcaga agaaagctat cgtggacctc ctcttcaaga cgaaccggaa 2820
agttaccgtg aaacagctca aagaagacta tttcaaaaag attgaatgtt tcgactctgt 2880
tgaaatcagc ggagtggagg atcgcttcaa cgcatccctg ggaacgtatc acgatctcct 2940
gaaaatcatt aaagacaagg acttcctgga caatgaggag aacgaggaca ttcttgagga 3000
cattgtcctc acccttacgt tgtttgaaga tagggagatg attgaagaac gcttgaaaac 3060
ttacgctcat ctcttcgacg acaaagtcat gaaacagctc aagaggcgcc gatatacagg 3120
atgggggcgg ctgtcaagaa aactgatcaa tgggatccga gacaagcaga gtggaaagac 3180
aatcctggat tttcttaagt ccgatggatt tgccaaccgg aacttcatgc agttgatcca 3240
tgatgactct ctcaccttta aggaggacat ccagaaagca caagtttctc tggctggcga 3300
tactctcatt accctggccg atggacgacg agtgcctatt agagaactgg tgtcacagca 3360
gaatttttcc gtgtgggctc tgaatcctca gacttaccgc ctggagaggg ctagagtgag 3420
tagagctttc tgtaccggca tcaaacctgt gtaccgcctc accactagac tggggagatc 3480
cattagggcc actgccaacc accgatttct cacacctcag ggctggaaac gagtcgatga 3540

actccagcct ggagattacc tggctctgcc taggagaatc cctactgcct cctgatctcg 3600
agctcgatga gtttggacaa accacaacta gaatgcagtg aaaaaaatgc tttatttgtg 3660
aaatttgtga tgctattgct ttatttgtaa ccattataag ctgcaataaa caagttaaca 3720
acaacaattg cattcatttt atgtttcagg ttcaggggga ggtgtgggag gttttttaaa 3780
gcaagtaaaa cctctacaaa tgtggtactt aagaggggga gaccaaaggg cacgcgttta 3840
aggcctcacg tgacatgtga gcaaaaggcc agcaaaatgt acaaaaaagc aggctttaaa 3900
ggaaccaatt cagtcgactg gatccggtac caaggtcggg caggaagagg gcctatttcc 3960
catgattcct tcatatttgc atatacgata caaggctgtt agagagataa ttagaattaa 4020
tttgactgta aacacaaaga tattagtaca aaatacgtga cgtagaaagt aataatttct 4080
tgggtagttt gcagttttaa aattatgttt taaaatggac tatcatatgc ttaccgtaac 4140
ttgaaagtat ttcgatttct tggctttata tatcttgtgg aaaggacgaa acaccagaga 4200
cgacttcaac atcaacggcg tctctgtttt agagctagaa atagcaagtt aaaataaggc 4260
tagtccgtta tcaacttgaa aaagtggcac cgagtcggtg cttttttttc tagagggcgg 4320
ccgcaggtag ataagtagca tggcgggtta atcattaact acaaggaacc cctagtgatg 4380
gagttggcca ctccctctct gcgcgctcgc tcgctcactg aggccgggcg accaaaggtc 4440
gcccgacgcc cgggctttgc ccgggcggcc tcagtgagcg agcgagcgcg cagccttaat 4500
taaatctggc gtaatcatgg tcatagctgt ttcctgtgtg aaattgttat ccgctcacaa 4560
ttccacacaa catacgagcc ggaagcataa agtgtaaagc ctggggtgcc taatgagtga 4620
gctaactcac attaattgcg ttgcgctcac tgcccgcttt ccagtcggga aacctgtcgt 4680
gccagctgca ttaatgaatc ggccaacgcg cggggagagg cggtttgcgt attgggcgct 4740
cttccgcttc ctcgctcact gactcgctgc gctcggtcgt tcggctgcgg cgagcggtat 4800
cagctcactc aaaggcggta atacggttat ccacagaatc aggggataac gcaggaaaga 4860
acatgtgagc aaaaccgcag caaaaggcca ggaaccgtaa aaaggccgcg ttgctggcgt 4920
ttttccatag gctccgcccc cctgacgagc atcacaaaaa tcgacgctca agtcagaggt 4980
ggcgaaaccc gacaggacta taaagatacc aggcgtttcc ccctggaagc tccctcgtgc 5040
gctctcctgt tccgaccctg ccgcttaccg gatacctgtc cgcctttctc ccttcgggaa 5100
gcgtggcgct ttctcatagc tcacgctgta ggtatctcag ttcggtgtag gtcgttcgct 5160
ccaagctggg ctgtgtgcac gaaccccccg ttcagcccga ccgctgcgcc ttatccggta 5220
actatcgtct tgagtccaac ccggtaagac acgacttatc gccactggca gcagccactg 5280
gtaacaggat tagcagagcg aggtatgtag gcggtgctac agagttcttg aagtggtggc 5340
ctaactacgg ctacactaga aggacagtat ttggtatctg cgctctgctg aagccagtta 5400
ccttcggaaa aagagttggt agctcttgat ccggcaaaca aaccaccgct ggtagcggtg 5460

gtttttttgt ttgcaagcag cagattacgc gcagaaaaaa aggatctcaa gaagatcctt 5520

tgatcttttc tacggggtct gacgctcagt ggaacgaaaa ctcacgttaa gggattttgg 5580

tcatgagatt atcaaaaagg atcttcacct agatcctttt aaattaaaaa tgaagtttta 5640

aatcaatcta aagtatatat gagtaaactt ggtctgacag ttaccaatgc ttaatcagtg 5700

aggcacctat ctcagcgatc tgtctatttc gttcatccat agttgcctga ctccccgtcg 5760

tgtagataac tacgatacgg gagggcttac catctggccc cagtgctgca atgataccgc 5820

gagacccacg ctcaccggct ccagatttat cagcaataaa ccagccagcc ggaagggccg 5880

agcgcagaag tggtcctgca actttatccg cctccatcca gtctattaat tgttgccggg 5940

aagctagagt aagtagttcg ccagttaata gtttgcgcaa cgttgttacc attactacag 6000

gcatcgtggt gtcacgctcg tcgtttggta tggcttcatt cagctccggt tcccaacgat 6060

caaggcgagt tacatgatcc cccatgttgt gcaaaaaagc ggttagctcc ttcggtcctc 6120

cgatcgttgt cagaagtaag ttggccgcag tgttatcact catggttatg gcagcactgc 6180

ataattctct tactgtcatg ccatccgtaa gatgcttttc tgtgactggt gagtactcaa 6240

ccaagtcatt ctgagaatag tgtatgcggc gaccgagttg ctcttgcccg gcgtcaatac 6300

gggataatac cgcgccacat agcagaactt taaaagtgct catcattgga aaacgttctt 6360

cggggcgaaa actctcaagg atcttaccac tattgagatc cagttcgatg taacccactc 6420

gtgcacccaa ctgatcttca gcatctttta ctttcaccag cgtttctggg tgagcaaaaa 6480

caggaaggca aaatgccgca aaaaagggaa taagggcgac acggaaatgt tgaatactca 6540

tactcttcct ttttcaatat tattgaagca tttatcaggg ttattgtctc atgagcggat 6600

acatatttga atgtatttag aaaaataaac aaataggggt tccgcgcaca tttccccgaa 6660

agatgccacc tgaaattata aacgttaata ttttgttaaa attcgcgtta aatttttgtt 6720

aaatcagctc attttttaac caataggccg aaatcggcaa aatcccttat aaatcaaaag 6780

aatagaccga gatagggttg agtgttgttc cagtttggaa caagagtcca ctattaaaga 6840

acgtggactc caacgtcaaa gggcgaaaaa ccgtctatca gggcgatggc ccactacgtg 6900

aaccatcacc ctaatcaagt tttttggggt cgaggtgccg taaagcacta aatcggaacc 6960

ctaaagggag cccccgattt agagcttgac ggggaaagcc ggcgaacgtg gcgagaaagg 7020

aagggaagaa agcgaaagga gcgggcgcta gggcgctggc aagtgtagcg gtcacgctgc 7080

gcgtaaccac cacacccgcc gcgcttaatg cgccgctaca gggcgcgtcc cattcgccat 7140

tcaggctgcg caactgttgg gaagggcgat cggtgcgggc ctcttcgcta ttacgccagc 7200

tggcgaaagg gggatgtgct gcaaggcgat taagttgggt aacgccaggg ttttcccagt 7260

cacgacgttg taaaacgacg gccagtgaat taggttaatt aaggctgcgc gctcgctcgc 7320

tcactgaggc cgcccgggca aagcccgggc gtcgggcgac ctttggtcgc ccggcctcag 7380

tgagcgagcg agcgcgcaga gagggagtgg ccaactccat cactaggggt tccttgtagt 7440

taatgattaa cccgccatgc tacttatcta cctgc 7475

<210> 14
<211> 7930
<212> DNA
<213> Artificial

<220>
<223> C-Cas9-Intein_CMV-AcrIIA4-scaffold-BGH (AAV construct)

<400> 14
ggccgccatt atggaatgtg tgtcagttag ggtgtggaaa gtccccaggc tccccagcag 60

gcagaagtat gcaaagcatg catctcaatt agtcagcaac cagtcccggt ctcctcccat 120

gcatgtcaat attggccatt agccatatta ttcattggtt atatagcata aatcaatatt 180

ggctattggc cattgcatac gttgtatcta tatcataata tgtacattta tattggctca 240

tgtccaatat gaccgccatg ttggcattga ttattgacta gttattaata gtaatcaatt 300

acggggtcat tagttcatag cccatatatg gagttccgcg ttacataact tacggtaaat 360

ggcccgcctg gctgaccgcc caacgacccc cgcccattga cgtcaataat gacgtatgtt 420

cccatagtaa cgccaatagg gactttccat tgacgtcaat gggtggagta tttacggtaa 480

actgcccact tggcagtaca tcaagtgtat catatgccaa gtccgccccc tattgacgtc 540

aatgacggta aatggcccgc ctggcattat gcccagtaca tgaccttacg ggactttcct 600

acttggcagt acatctacgt attagtcatc gctattacca tggtgatgcg gttttggcag 660

tacaccaatg ggcgtggata gcggtttgac tcacggggat ttccaagtct ccaccccatt 720

gacgtcaatg ggagtttgtt ttggcaccaa aatcaacggg actttccaaa atgtcgtaat 780

aaccccgccc cgttgacgca aatgggcggt aggcgtgtac ggtgggaggt ctatataagc 840

agaggtcgtt tagtgaaccg tcagatcact agtagcttta ttgcggtagt ttatcacagt 900

taaattgcta acgcagtcag tgctcgactg atcacaggta agtatcaagg ttacaagaca 960

ggtttaagga ggccaataga aactgggctt gtcgagacag agaagattct tgcgtttctg 1020

ataggcacct attggtctta ctgacatcca ctttgccttt ctctccacag gggtaccgaa 1080

gccgctagcg ctaccggtcg ccaccatggc ggcggcgtgc ccggaactgc gtcagctggc 1140

gcagagcgat gtgtattggg atccgattgt gagcattgaa ccggatggcg tggaagaagt 1200

gtttgatctg accgtgccgg gcccgcataa ctttgtggcg aacgatatta ttgcgcataa 1260

ctctggccag ggggacagtc ttcacgagca catcgctaat cttgcaggta gcccagctat 1320

caaaaaggga atactgcaga ccgttaaggt cgtggatgaa ctcgtcaaag taatgggaag 1380

gcataagccc gagaatatcg ttatcgagat ggcccgagag aaccaaacta cccagaaggg 1440

acagaagaac agtagggaaa ggatgaagag gattgaagag ggtataaaag aactggggtc 1500

ccaaatcctt aaggaacacc cagttgaaaa cacccagctt cagaatgaga agctctacct 1560

gtactacctg cagaacggca gggacatgta cgtggatcag gaactggaca tcaatcggct 1620

ctccgactac gacgtggatc atatcgtgcc ccagtctttt ctcaaagatg attctattga 1680

taataaagtg ttgacaagat ccgataaaaa tagagggaag agtgataacg tcccctcaga 1740

agaagttgtc aagaaaatga aaaattattg gcggcagctg ctgaacgcca aactgatcac 1800

acaacggaag ttcgataatc tgactaaggc tgaacgaggt ggcctgtctg agttggataa 1860

agccggcttc atcaaaaggc agcttgttga gacacgccag atcaccaagc acgtggccca 1920

aattctcgat tcacgcatga acaccaagta cgatgaaaat gacaaactga ttcgagaggt 1980

gaaagttatt actctgaagt ctaagctggt ctcagatttc agaaaggact ttcagtttta 2040

taaggtgaga gagatcaaca attaccacca tgcgcatgat gcctacctga atgcagtggt 2100

aggcactgca cttatcaaaa aatatcccaa gcttgaatct gaatttgttt acggagacta 2160

taaagtgtac gatgttagga aaatgatcgc aaagtctgag caggaaatag gcaaggccac 2220

cgctaagtac ttcttttaca gcaatattat gaattttttc aagaccgaga ttacactggc 2280

caatggagag attcggaagc gaccacttat cgaaacaaac ggagaaacag gagaaatcgt 2340

gtgggacaag ggtagggatt tcgcgacagt ccggaaggtc ctgtccatgc cgcaggtgaa 2400

catcgttaaa aagaccgaag tacagaccgg aggcttctcc aaggaaagta tcctcccgaa 2460

aaggaacagc gacaagctga tcgcacgcaa aaaagattgg gaccccaaga aatacggcgg 2520

attcgattct cctacagtcg cttacagtgt actggttgtg gccaaagtgg agaaagggaa 2580

gtctaaaaaa ctcaaaagcg tcaaggaact gctgggcatc acaatcatgg agcgatcaag 2640

cttcgaaaaa aaccccatcg actttctcga ggcgaaagga tataaagagg tcaaaaaaga 2700

cctcatcatt aagcttccca agtactctct ctttgagctt gaaaacggcc ggaaacgaat 2760

gctcgctagt gcgggcgagc tgcagaaagg taacgagctg gcactgccct ctaaatacgt 2820

taatttcttg tatctggcca gccactatga aaagctcaaa gggtctcccg aagataatga 2880

gcagaagcag ctgttcgtgg aacaacacaa acactacctt gatgagatca tcgagcaaat 2940

aagcgaattc tccaaaagag tgatcctcgc cgacgctaac ctcgataagg tgctttctgc 3000

ttacaataag cacagggata agcccatcag ggagcaggca gaaaacatta tccacttgtt 3060

tactctgacc aacttgggcg cgcctgcagc cttcaagtac ttcgacacca ccatagacag 3120

aaagcggtac acctctacaa aggaggtcct ggacgccaca ctgattcatc agtcaattac 3180

ggggctctat gaaacaagaa tcgacctctc tcagctcggt ggagacagca gggctgaccc 3240

caagaagaag aggaaggtgt ctcgagctcg atgagtttgg acaaaccaca actagaatgc 3300

agtgaaaaaa atgctttatt tgtgaaattt gtgatgctat tgctttattt gtaaccatta 3360

taagctgcaa taaacaagtt aacaacaaca attgcattca ttttatgttt caggttcagg 3420

gggaggtgtg ggaggttttt taaagcaagt aaaacctcta caaatgtggt atctagagac 3480

attgattatt gactagttat taatagtaat caattacggg gtcattagtt catagcccat 3540

atatggagtt ccgcgttaca taacttacgg taaatggccc gcctggctga ccgcccaacg 3600

acccccgccc attgacgtca ataatgacgt atgttcccat agtaacgcca atagggactt 3660

tccattgacg tcaatgggtg gagtatttac ggtaaactgc ccacttggca gtacatcaag 3720

tgtatcatat gccaagtacg ccccctattg acgtcaatga cggtaaatgg cccgcctggc 3780

attatgccca gtacatgacc ttatgggact ttcctacttg gcagtacatc tacgtattag 3840

tcatcgctat taccatggtg atgcggtttt ggcagtacat caatgggcgt ggatagcggt 3900

ttgactcacg gggatttcca agtctccacc ccattgacgt caatgggagt ttgttttggc 3960

accaaaatca acgggacttt ccaaaatgtc gtaacaactc cgccccattg acgcaaatgg 4020

gcggtaggcg tgtacggtgg gaggtctata taagcagagc tctctggcta actagagaac 4080

ccactgctta ctggcttatc gaaattaata cgactcacta tagggagacc caagctggct 4140

agcatgaaca ttaacgacct gattcgggaa ataaagaaca aggactacac ggtcaagctg 4200

tcaggcaccg actccaactc tataacacag ctcattataa gggttaataa tgatggcaac 4260

gaatatgtca tctctgaatc cgagaatgaa tcaatcgtgg agaaattcat cagtgcattt 4320

aaaaacggct ggaaccagga gtacgaagat gaggaagagt tctacaatga tatgcaaact 4380

atcacactga aatctgagct gaactagctc gagcggccgc cactgtgctg gatatctgca 4440

gaattagaga cgccgttgat gttgaagttt atataccgtt gatgttgaag tcgtctctag 4500

cttaagttta aaccgctgat cagcctcgac tgtgccttct agttgccagc catctgttgt 4560

ttgcccctcc cccgtgcctt ccttgaccct ggaaggtgcc actcccactg tcctttccta 4620

ataaaatgag gaaattgcat cgcattgtct gagtaggtgt cattctattc tggggggtgg 4680

ggtggggcag gacagcaagg gggaggattg ggaagacaat agcaggcatg ctggggatgc 4740

ggtgggctct atggacgcgt gcggccgcag gtagataagt agcatggcgg gttaatcatt 4800

aactacaagg aacccctagt gatggagttg gccactccct ctctgcgcgc tcgctcgctc 4860

actgaggccg ggcgaccaaa ggtcgcccga cgcccgggct ttgcccgggc ggcctcagtg 4920

agcgagcgag cgcgcagcct aattaaggcc ttaattaaat ctggcgtaat catggtcata 4980

gctgtttcct gtgtgaaatt gttatccgct cacaattcca cacaacatac gagccggaag 5040

cataaagtgt aaagcctggg gtgcctaatg agtgagctaa ctcacattaa ttgcgttgcg 5100

ctcactgccc gctttccagt cgggaaacct gtcgtgccag ctgcattaat gaatcggcca 5160

acgcgcgggg agaggcggtt tgcgtattgg gcgctcttcc gcttcctcgc tcactgactc 5220

gctgcgctcg gtcgttcggc tgcggcgagc ggtatcagct cactcaaagg cggtaatacg 5280

gttatccaca gaatcagggg ataacgcagg aaagaacatg tgagcaaaac cgcagcaaaa 5340

ggccaggaac cgtaaaaagg ccgcgttgct ggcgtttttc cataggctcc gcccccctga 5400

cgagcatcac aaaaatcgac gctcaagtca gaggtggcga aacccgacag gactataaag 5460

ataccaggcg tttccccctg gaagctccct cgtgcgctct cctgttccga ccctgccgct 5520

taccggatac ctgtccgcct ttctcccttc gggaagcgtg gcgctttctc atagctcacg 5580

ctgtaggtat ctcagttcgg tgtaggtcgt tcgctccaag ctgggctgtg tgcacgaacc 5640

ccccgttcag cccgaccgct gcgccttatc cggtaactat cgtcttgagt ccaacccggt 5700

aagacacgac ttatcgccac tggcagcagc cactggtaac aggattagca gagcgaggta 5760

tgtaggcggt gctacagagt tcttgaagtg gtggcctaac tacggctaca ctagaaggac 5820

agtatttggt atctgcgctc tgctgaagcc agttaccttc ggaaaaagag ttggtagctc 5880

ttgatccggc aaacaaacca ccgctggtag cggtggtttt tttgtttgca agcagcagat 5940

tacgcgcaga aaaaaaggat ctcaagaaga tcctttgatc ttttctacgg ggtctgacgc 6000

tcagtggaac gaaaactcac gttaagggat tttggtcatg agattatcaa aaaggatctt 6060

cacctagatc cttttaaatt aaaaatgaag ttttaaatca atctaaagta tatatgagta 6120

aacttggtct gacagttacc aatgcttaat cagtgaggca cctatctcag cgatctgtct 6180

atttcgttca tccatagttg cctgactccc cgtcgtgtag ataactacga tacgggaggg 6240

cttaccatct ggccccagtg ctgcaatgat accgcgagac ccacgctcac cggctccaga 6300

tttatcagca ataaaccagc cagccggaag ggccgagcgc agaagtggtc ctgcaacttt 6360

atccgcctcc atccagtcta ttaattgttg ccgggaagct agagtaagta gttcgccagt 6420

taatagtttg cgcaacgttg ttaccattac tacaggcatc gtggtgtcac gctcgtcgtt 6480

tggtatggct tcattcagct ccggttccca acgatcaagg cgagttacat gatcccccat 6540

gttgtgcaaa aaagcggtta gctccttcgg tcctccgatc gttgtcagaa gtaagttggc 6600

cgcagtgtta tcactcatgg ttatggcagc actgcataat tctcttactg tcatgccatc 6660

cgtaagatgc ttttctgtga ctggtgagta ctcaaccaag tcattctgag aatagtgtat 6720

gcggcgaccg agttgctctt gcccggcgtc aatacgggat aataccgcgc cacatagcag 6780

aactttaaaa gtgctcatca ttggaaaacg ttcttcgggg cgaaaactct caaggatctt 6840

accactattg agatccagtt cgatgtaacc cactcgtgca cccaactgat cttcagcatc 6900

ttttactttc accagcgttt ctgggtgagc aaaaacagga aggcaaaatg ccgcaaaaaa 6960

gggaataagg gcgacacgga aatgttgaat actcatactc ttcctttttc aatattattg 7020

aagcatttat cagggttatt gtctcatgag cggatacata tttgaatgta tttagaaaaa 7080

taaacaaata ggggttccgc gcacatttcc ccgaaaagtg ccacctgaaa ttataaacgt 7140

taatattttg ttaaaattcg cgttaaattt ttgttaaatc agctcatttt ttaaccaata 7200

ggccgaaatc ggcaaaatcc cttataaatc aaaagaatag accgagatag ggttgagtgt 7260

tgttccagtt tggaacaaga gtccactatt aaagaacgtg gactccaacg tcaaagggcg 7320

aaaaaccgtc tatcagggcg atggcccact acgtgaacca tcaccctaat caagtttttt 7380

ggggtcgagg tgccgtaaag cactaaatcg gaaccctaaa gggagccccc gatttagagc 7440

ttgacgggga aagccggcga acgtggcgag aaaggaaggg aagaaagcga aaggagcggg 7500

cgctagggcg ctggcaagtg tagcggtcac gctgcgcgta accaccacac ccgccgcgct 7560

taatgcgccg ctacagggcg cgtcccattc gccattcagg ctgcgcaact gttgggaagg 7620

gcgatcggtg cgggcctctt cgctattacg ccagctggcg aaagggggat gtgctgcaag 7680

gcgattaagt tgggtaacgc cagggttttc ccagtcacga cgttgtaaaa cgacggccag 7740

tgaattaggt taattaaggc tgcgcgctcg ctcgctcact gaggccgccc gggcaaagcc 7800

cgggcgtcgg gcgacctttg gtcgcccggc ctcagtgagc gagcgagcgc gcagagaggg 7860

agtggccaac tccatcacta ggggttcctt gtagttaatg attaacccgc catgctactt 7920

atctacctgc 7930

<210> 15
<211> 7930
<212> DNA
<213> Artificial

<220>
<223> C-Cas9-Intein_CMV-AcrIIA4-2xmir122BS-BGH (AAV construct)

<400> 15
ggccgccatt atggaatgtg tgtcagttag ggtgtggaaa gtccccaggc tccccagcag 60

gcagaagtat gcaaagcatg catctcaatt agtcagcaac cagtcccggt ctcctcccat 120

gcatgtcaat attggccatt agccatatta ttcattggtt atatagcata aatcaatatt 180

ggctattggc cattgcatac gttgtatcta tatcataata tgtacattta tattggctca 240

tgtccaatat gaccgccatg ttggcattga ttattgacta gttattaata gtaatcaatt 300

acggggtcat tagttcatag cccatatatg gagttccgcg ttacataact tacggtaaat 360

ggcccgcctg gctgaccgcc caacgacccc cgcccattga cgtcaataat gacgtatgtt 420

cccatagtaa cgccaatagg gactttccat tgacgtcaat gggtggagta tttacggtaa 480

actgcccact tggcagtaca tcaagtgtat catatgccaa gtccgccccc tattgacgtc 540

aatgacggta aatggcccgc ctggcattat gcccagtaca tgaccttacg ggactttcct 600

acttggcagt acatctacgt attagtcatc gctattacca tggtgatgcg gttttggcag 660

tacaccaatg ggcgtggata gcggtttgac tcacggggat ttccaagtct ccaccccatt 720

gacgtcaatg ggagtttgtt ttggcaccaa aatcaacggg actttccaaa atgtcgtaat 780
aaccccgccc cgttgacgca aatgggcggt aggcgtgtac ggtgggaggt ctatataagc 840
agaggtcgtt tagtgaaccg tcagatcact agtagcttta ttgcggtagt ttatcacagt 900
taaattgcta acgcagtcag tgctcgactg atcacaggta agtatcaagg ttacaagaca 960
ggtttaagga ggccaataga aactgggctt gtcgagacag agaagattct tgcgtttctg 1020
ataggcacct attggtctta ctgacatcca ctttgccttt ctctccacag gggtaccgaa 1080
gccgctagcg ctaccggtcg ccaccatggc ggcggcgtgc ccggaactgc gtcagctggc 1140
gcagagcgat gtgtattggg atccgattgt gagcattgaa ccggatggcg tggaagaagt 1200
gtttgatctg accgtgccgg gcccgcataa ctttgtggcg aacgatatta ttgcgcataa 1260
ctctggccag ggggacagtc ttcacgagca catcgctaat cttgcaggta gcccagctat 1320
caaaaaggga atactgcaga ccgttaaggt cgtggatgaa ctcgtcaaag taatgggaag 1380
gcataagccc gagaatatcg ttatcgagat ggcccgagag aaccaaacta cccagaaggg 1440
acagaagaac agtagggaaa ggatgaagag gattgaagag ggtataaaag aactggggtc 1500
ccaaatcctt aaggaacacc cagttgaaaa cacccagctt cagaatgaga agctctacct 1560
gtactacctg cagaacggca gggacatgta cgtggatcag gaactggaca tcaatcggct 1620
ctccgactac gacgtggatc atatcgtgcc ccagtctttt ctcaaagatg attctattga 1680
taataaagtg ttgacaagat ccgataaaaa tagagggaag agtgataacg tcccctcaga 1740
agaagttgtc aagaaaatga aaaattattg gcggcagctg ctgaacgcca aactgatcac 1800
acaacggaag ttcgataatc tgactaaggc tgaacgaggt ggcctgtctg agttggataa 1860
agccggcttc atcaaaaggc agcttgttga gacacgccag atcaccaagc acgtggccca 1920
aattctcgat tcacgcatga acaccaagta cgatgaaaat gacaaactga ttcgagaggt 1980
gaaagttatt actctgaagt ctaagctggt ctcagatttc agaaaggact ttcagtttta 2040
taaggtgaga gagatcaaca attaccacca tgcgcatgat gcctacctga atgcagtggt 2100
aggcactgca cttatcaaaa aatatcccaa gcttgaatct gaatttgttt acggagacta 2160
taaagtgtac gatgttagga aaatgatcgc aaagtctgag caggaaatag gcaaggccac 2220
cgctaagtac ttcttttaca gcaatattat gaattttttc aagaccgaga ttacactggc 2280
caatggagag attcggaagc gaccacttat cgaaacaaac ggagaaacag gagaaatcgt 2340
gtgggacaag ggtagggatt tcgcgacagt ccggaaggtc ctgtccatgc cgcaggtgaa 2400
catcgttaaa aagaccgaag tacagaccgg aggcttctcc aaggaaagta tcctcccgaa 2460
aaggaacagc gacaagctga tcgcacgcaa aaaagattgg gaccccaaga aatacggcgg 2520
attcgattct cctacagtcg cttacagtgt actggttgtg gccaaagtgg agaaagggaa 2580

gtctaaaaaa ctcaaaagcg tcaaggaact gctgggcatc acaatcatgg agcgatcaag 2640

cttcgaaaaa aaccccatcg actttctcga ggcgaaagga tataaagagg tcaaaaaaga 2700

cctcatcatt aagcttccca agtactctct ctttgagctt gaaaacggcc ggaaacgaat 2760

gctcgctagt gcgggcgagc tgcagaaagg taacgagctg gcactgccct ctaaatacgt 2820

taatttcttg tatctggcca gccactatga aaagctcaaa gggtctcccg aagataatga 2880

gcagaagcag ctgttcgtgg aacaacacaa acactacctt gatgagatca tcgagcaaat 2940

aagcgaattc tccaaaagag tgatcctcgc cgacgctaac ctcgataagg tgctttctgc 3000

ttacaataag cacagggata agcccatcag ggagcaggca gaaaacatta tccacttgtt 3060

tactctgacc aacttgggcg cgcctgcagc cttcaagtac ttcgacacca ccatagacag 3120

aaagcggtac acctctacaa aggaggtcct ggacgccaca ctgattcatc agtcaattac 3180

ggggctctat gaaacaagaa tcgacctctc tcagctcggt ggagacagca gggctgaccc 3240

caagaagaag aggaaggtgt ctcgagctcg atgagtttgg acaaaccaca actagaatgc 3300

agtgaaaaaa atgctttatt tgtgaaattt gtgatgctat tgctttattt gtaaccatta 3360

taagctgcaa taaacaagtt aacaacaaca attgcattca ttttatgttt caggttcagg 3420

gggaggtgtg ggaggttttt taaagcaagt aaaacctcta caaatgtggt atctagagac 3480

attgattatt gactagttat taatagtaat caattacggg gtcattagtt catagcccat 3540

atatggagtt ccgcgttaca taacttacgg taaatggccc gcctggctga ccgcccaacg 3600

acccccgccc attgacgtca ataatgacgt atgttcccat agtaacgcca atagggactt 3660

tccattgacg tcaatgggtg gagtatttac ggtaaactgc ccacttggca gtacatcaag 3720

tgtatcatat gccaagtacg ccccctattg acgtcaatga cggtaaatgg cccgcctggc 3780

attatgccca gtacatgacc ttatgggact ttcctacttg gcagtacatc tacgtattag 3840

tcatcgctat taccatggtg atgcggtttt ggcagtacat caatgggcgt ggatagcggt 3900

ttgactcacg gggatttcca agtctccacc ccattgacgt caatgggagt ttgttttggc 3960

accaaaatca acgggacttt ccaaaatgtc gtaacaactc cgccccattg acgcaaatgg 4020

gcggtaggcg tgtacggtgg gaggtctata taagcagagc tctctggcta actagagaac 4080

ccactgctta ctggcttatc gaaattaata cgactcacta tagggagacc caagctggct 4140

agcatgaaca ttaacgacct gattcgggaa ataaagaaca aggactacac ggtcaagctg 4200

tcaggcaccg actccaactc tataacacag ctcattataa gggttaataa tgatggcaac 4260

gaatatgtca tctctgaatc cgagaatgaa tcaatcgtgg agaaattcat cagtgcattt 4320

aaaaacggct ggaaccagga gtacgaagat gaggaagagt tctacaatga tatgcaaact 4380

atcacactga aatctgagct gaactagctc gagcggccgc cactgtgctg gatatctgca 4440

gaattacaaa caccattgtc acactccatt atatacaaac accattgtca cactccatag 4500

cttaagttta aaccgctgat cagcctcgac tgtgccttct agttgccagc catctgttgt 4560

ttgcccctcc cccgtgcctt ccttgaccct ggaaggtgcc actcccactg tcctttccta 4620

ataaaatgag gaaattgcat cgcattgtct gagtaggtgt cattctattc tggggggtgg 4680

ggtggggcag gacagcaagg gggaggattg ggaagacaat agcaggcatg ctggggatgc 4740

ggtgggctct atggacgcgt gcggccgcag gtagataagt agcatggcgg gttaatcatt 4800

aactacaagg aacccctagt gatggagttg gccactccct ctctgcgcgc tcgctcgctc 4860

actgaggccg ggcgaccaaa ggtcgcccga cgcccgggct ttgcccgggc ggcctcagtg 4920

agcgagcgag cgcgcagcct aattaaggcc ttaattaaat ctggcgtaat catggtcata 4980

gctgtttcct gtgtgaaatt gttatccgct cacaattcca cacaacatac gagccggaag 5040

cataaagtgt aaagcctggg gtgcctaatg agtgagctaa ctcacattaa ttgcgttgcg 5100

ctcactgccc gctttccagt cgggaaacct gtcgtgccag ctgcattaat gaatcggcca 5160

acgcgcgggg agaggcggtt tgcgtattgg gcgctcttcc gcttcctcgc tcactgactc 5220

gctgcgctcg gtcgttcggc tgcggcgagc ggtatcagct cactcaaagg cggtaatacg 5280

gttatccaca gaatcagggg ataacgcagg aaagaacatg tgagcaaaac cgcagcaaaa 5340

ggccaggaac cgtaaaaagg ccgcgttgct ggcgtttttc cataggctcc gcccccctga 5400

cgagcatcac aaaaatcgac gctcaagtca gaggtggcga aacccgacag gactataaag 5460

ataccaggcg tttccccctg gaagctccct cgtgcgctct cctgttccga ccctgccgct 5520

taccggatac ctgtccgcct ttctcccttc gggaagcgtg gcgctttctc atagctcacg 5580

ctgtaggtat ctcagttcgg tgtaggtcgt tcgctccaag ctgggctgtg tgcacgaacc 5640

ccccgttcag cccgaccgct gcgccttatc cggtaactat cgtcttgagt ccaacccggt 5700

aagacacgac ttatcgccac tggcagcagc cactggtaac aggattagca gagcgaggta 5760

tgtaggcggt gctacagagt tcttgaagtg gtggcctaac tacggctaca ctagaaggac 5820

agtatttggt atctgcgctc tgctgaagcc agttaccttc ggaaaaagag ttggtagctc 5880

ttgatccggc aaacaaacca ccgctggtag cggtggtttt tttgtttgca agcagcagat 5940

tacgcgcaga aaaaaaggat ctcaagaaga tcctttgatc ttttctacgg ggtctgacgc 6000

tcagtggaac gaaaactcac gttaagggat tttggtcatg agattatcaa aaaggatctt 6060

cacctagatc cttttaaatt aaaaatgaag ttttaaatca atctaaagta tatatgagta 6120

aacttggtct gacagttacc aatgcttaat cagtgaggca cctatctcag cgatctgtct 6180

atttcgttca tccatagttg cctgactccc cgtcgtgtag ataactacga tacgggaggg 6240

cttaccatct ggccccagtg ctgcaatgat accgcgagac ccacgctcac cggctccaga 6300

tttatcagca ataaaccagc cagccggaag ggccgagcgc agaagtggtc ctgcaacttt 6360

atccgcctcc atccagtcta ttaattgttg ccgggaagct agagtaagta gttcgccagt 6420

taatagtttg cgcaacgttg ttaccattac tacaggcatc gtggtgtcac gctcgtcgtt 6480

tggtatggct tcattcagct ccggttccca acgatcaagg cgagttacat gatcccccat 6540

gttgtgcaaa aaagcggtta gctccttcgg tcctccgatc gttgtcagaa gtaagttggc 6600

cgcagtgtta tcactcatgg ttatggcagc actgcataat tctcttactg tcatgccatc 6660

cgtaagatgc ttttctgtga ctggtgagta ctcaaccaag tcattctgag aatagtgtat 6720

gcggcgaccg agttgctctt gcccggcgtc aatacgggat aataccgcgc cacatagcag 6780

aactttaaaa gtgctcatca ttggaaaacg ttcttcgggg cgaaaactct caaggatctt 6840

accactattg agatccagtt cgatgtaacc cactcgtgca cccaactgat cttcagcatc 6900

ttttactttc accagcgttt ctgggtgagc aaaaacagga aggcaaaatg ccgcaaaaaa 6960

gggaataagg gcgacacgga aatgttgaat actcatactc ttcctttttc aatattattg 7020

aagcatttat cagggttatt gtctcatgag cggatacata tttgaatgta tttagaaaaa 7080

taaacaaata ggggttccgc gcacatttcc ccgaaagatg ccacctgaaa ttataaacgt 7140

taatattttg ttaaaattcg cgttaaattt ttgttaaatc agctcatttt ttaaccaata 7200

ggccgaaatc ggcaaaatcc cttataaatc aaaagaatag accgagatag ggttgagtgt 7260

tgttccagtt tggaacaaga gtccactatt aaagaacgtg gactccaacg tcaaagggcg 7320

aaaaaccgtc tatcagggcg atggcccact acgtgaacca tcaccctaat caagtttttt 7380

ggggtcgagg tgccgtaaag cactaaatcg gaaccctaaa gggagccccc gatttagagc 7440

ttgacgggga aagccggcga acgtggcgag aaaggaaggg aagaaagcga aaggagcggg 7500

cgctagggcg ctggcaagtg tagcggtcac gctgcgcgta accaccacac ccgccgcgct 7560

taatgcgccg ctacagggcg cgtcccattc gccattcagg ctgcgcaact gttgggaagg 7620

gcgatcggtg cgggcctctt cgctattacg ccagctggcg aaagggggat gtgctgcaag 7680

gcgattaagt tgggtaacgc cagggttttc ccagtcacga cgttgtaaaa cgacggccag 7740

tgaattaggt taattaaggc tgcgcgctcg ctcgctcact gaggccgccc gggcaaagcc 7800

cgggcgtcgg gcgacctttg gtcgcccggc ctcagtgagc gagcgagcgc gcagagaggg 7860

agtggccaac tccatcacta ggggttcctt gtagttaatg attaacccgc catgctactt 7920

atctacctgc 7930

<210> 16
<211> 7506
<212> DNA
<213> Artificial

<220>
<223> C-Cas9-Intein_EFS-AcrIIA4-scaffold-BGH (AAV construct)

<400>  16
ggccgccatt atggaatgtg tgtcagttag ggtgtggaaa gtccccaggc tccccagcag      60

gcagaagtat gcaaagcatg catctcaatt agtcagcaac cagtcccggt ctcctcccat     120

gcatgtcaat attggccatt agccatatta ttcattggtt atatagcata aatcaatatt     180

ggctattggc cattgcatac gttgtatcta tatcataata tgtacattta tattggctca     240

tgtccaatat gaccgccatg ttggcattga ttattgacta gttattaata gtaatcaatt     300

acggggtcat tagttcatag cccatatatg gagttccgcg ttacataact tacggtaaat     360

ggcccgcctg gctgaccgcc caacgacccc cgcccattga cgtcaataat gacgtatgtt     420

cccatagtaa cgccaatagg gactttccat tgacgtcaat gggtggagta tttacggtaa     480

actgcccact tggcagtaca tcaagtgtat catatgccaa gtccgccccc tattgacgtc     540

aatgacggta aatggcccgc ctggcattat gcccagtaca tgaccttacg ggactttcct     600

acttggcagt acatctacgt attagtcatc gctattacca tggtgatgcg gttttggcag     660

tacaccaatg ggcgtggata gcggtttgac tcacggggat ttccaagtct ccaccccatt     720

gacgtcaatg ggagtttgtt ttggcaccaa aatcaacggg actttccaaa atgtcgtaat     780

aaccccgccc cgttgacgca aatgggcggt aggcgtgtac ggtgggaggt ctatataagc     840

agaggtcgtt tagtgaaccg tcagatcact agtagcttta ttgcggtagt ttatcacagt     900

taaattgcta acgcagtcag tgctcgactg atcacaggta agtatcaagg ttacaagaca     960

ggtttaagga ggccaataga aactgggctt gtcgagacag agaagattct tgcgtttctg    1020

ataggcacct attggtctta ctgacatcca ctttgccttt ctctccacag gggtaccgaa    1080

gccgctagcg ctaccggtcg ccaccatggc ggcggcgtgc ccggaactgc gtcagctggc    1140

gcagagcgat gtgtattggg atccgattgt gagcattgaa ccggatggcg tggaagaagt    1200

gtttgatctg accgtgccgg gcccgcataa ctttgtggcg aacgatatta ttgcgcataa    1260

ctctggccag ggggacagtc ttcacgagca catcgctaat cttgcaggta gcccagctat    1320

caaaaaggga atactgcaga ccgttaaggt cgtggatgaa ctcgtcaaag taatgggaag    1380

gcataagccc gagaatatcg ttatcgagat ggcccgagag aaccaaacta cccagaaggg    1440

acagaagaac agtagggaaa ggatgaagag gattgaagag ggtataaaag aactggggtc    1500

ccaaatcctt aaggaacacc cagttgaaaa cacccagctt cagaatgaga agctctacct    1560

gtactacctg cagaacggca gggacatgta cgtggatcag gaactggaca tcaatcggct    1620

ctccgactac gacgtggatc atatcgtgcc ccagtctttt ctcaaagatg attctattga    1680

taataaagtg ttgacaagat ccgataaaaa tagagggaag agtgataacg tcccctcaga    1740

agaagttgtc aagaaaatga aaaattattg gcggcagctg ctgaacgcca aactgatcac    1800

acaacggaag ttcgataatc tgactaaggc tgaacgaggt ggcctgtctg agttggataa    1860

agccggcttc atcaaaaggc agcttgttga gacacgccag atcaccaagc acgtggccca 1920

aattctcgat tcacgcatga acaccaagta cgatgaaaat gacaaactga ttcgagaggt 1980

gaaagttatt actctgaagt ctaagctggt ctcagatttc agaaaggact ttcagtttta 2040

taaggtgaga gagatcaaca attaccacca tgcgcatgat gcctacctga atgcagtggt 2100

aggcactgca cttatcaaaa aatatcccaa gcttgaatct gaatttgttt acggagacta 2160

taaagtgtac gatgttagga aaatgatcgc aaagtctgag caggaaatag gcaaggccac 2220

cgctaagtac ttcttttaca gcaatattat gaattttttc aagaccgaga ttacactggc 2280

caatggagag attcggaagc gaccacttat cgaaacaaac ggagaaacag gagaaatcgt 2340

gtgggacaag ggtagggatt tcgcgacagt ccggaaggtc ctgtccatgc cgcaggtgaa 2400

catcgttaaa aagaccgaag tacagaccgg aggcttctcc aaggaaagta tcctcccgaa 2460

aaggaacagc gacaagctga tcgcacgcaa aaaagattgg gaccccaaga aatacggcgg 2520

attcgattct cctacagtcg cttacagtgt actggttgtg gccaaagtgg agaaagggaa 2580

gtctaaaaaa ctcaaaagcg tcaaggaact gctgggcatc acaatcatgg agcgatcaag 2640

cttcgaaaaa aaccccatcg actttctcga ggcgaaagga tataaagagg tcaaaaaaga 2700

cctcatcatt aagcttccca agtactctct ctttgagctt gaaaacggcc ggaaacgaat 2760

gctcgctagt gcgggcgagc tgcagaaagg taacgagctg gcactgccct ctaaatacgt 2820

taatttcttg tatctggcca gccactatga aaagctcaaa gggtctcccg aagataatga 2880

gcagaagcag ctgttcgtgg aacaacacaa acactacctt gatgagatca tcgagcaaat 2940

aagcgaattc tccaaaagag tgatcctcgc cgacgctaac ctcgataagg tgctttctgc 3000

ttacaataag cacagggata agcccatcag ggagcaggca gaaaacatta tccacttgtt 3060

tactctgacc aacttgggcg cgcctgcagc cttcaagtac ttcgacacca ccatagacag 3120

aaagcggtac acctctacaa aggaggtcct ggacgccaca ctgattcatc agtcaattac 3180

ggggctctat gaaacaagaa tcgacctctc tcagctcggt ggagacagca gggctgaccc 3240

caagaagaag aggaaggtgt ctcgagctcg atgagtttgg acaaaccaca actagaatgc 3300

agtgaaaaaa atgctttatt tgtgaaattt gtgatgctat tgctttattt gtaaccatta 3360

taagctgcaa taaacaagtt aacaacaaca attgcattca ttttatgttt caggttcagg 3420

gggaggtgtg ggaggttttt taaagcaagt aaaacctcta caaatgtggt atctagagct 3480

ccggtgcccg tcagtgggca gagcgcacat cgcccacagt ccccgagaag ttggggggag 3540

gggtcggcaa ttgatccggt gcctagagaa ggtggcgcgg ggtaaactgg gaaagtgatg 3600

tcgtgtactg gctccgcctt tttcccgagg gtgggggaga accgtatata agtgcagtag 3660

tcgccgtgaa cgttcttttt cgcaacgggt ttgccgccag aacacaggga tccgccacca 3720

tgaacattaa cgacctgatt cgggaaataa agaacaagga ctacacggtc aagctgtcag 3780

gcaccgactc caactctata acacagctca ttataagggt taataatgat ggcaacgaat 3840

atgtcatctc tgaatccgag aatgaatcaa tcgtggagaa attcatcagt gcatttaaaa 3900

acggctggaa ccaggagtac gaagatgagg aagagttcta caatgatatg caaactatca 3960

cactgaaatc tgagctgaac tagctcgagc ggccgccact gtgctggata tctgcagaat 4020

tagagacgcc gttgatgttg aagtttatat accgttgatg ttgaagtcgt ctctagctta 4080

agtttaaacc gctgatcagc ctcgactgtg ccttctagtt gccagccatc tgttgtttgc 4140

ccctcccccg tgccttcctt gaccctggaa ggtgccactc ccactgtcct ttcctaataa 4200

aatgaggaaa ttgcatcgca ttgtctgagt aggtgtcatt ctattctggg gggtggggtg 4260

gggcaggaca gcaaggggga ggattgggaa gacaatagca ggcatgctgg ggatgcggtg 4320

ggctctatgg acgcgtgcgg ccgcaggtag ataagtagca tggcgggtta atcattaact 4380

acaaggaacc cctagtgatg gagttggcca ctccctctct gcgcgctcgc tcgctcactg 4440

aggccgggcg accaaaggtc gcccgacgcc cgggctttgc ccgggcggcc tcagtgagcg 4500

agcgagcgcg cagcctaatt aaggccttaa ttaaatctgg cgtaatcatg gtcatagctg 4560

tttcctgtgt gaaattgtta tccgctcaca attccacaca acatacgagc cggaagcata 4620

aagtgtaaag cctggggtgc ctaatgagtg agctaactca cattaattgc gttgcgctca 4680

ctgcccgctt tccagtcggg aaacctgtcg tgccagctgc attaatgaat cggccaacgc 4740

gcggggagag gcggtttgcg tattgggcgc tcttccgctt cctcgctcac tgactcgctg 4800

cgctcggtcg ttcggctgcg gcgagcggta tcagctcact caaaggcggt aatacggtta 4860

tccacagaat caggggataa cgcaggaaag aacatgtgag caaaaccgca gcaaaaggcc 4920

aggaaccgta aaaaggccgc gttgctggcg tttttccata ggctccgccc ccctgacgag 4980

catcacaaaa atcgacgctc aagtcagagg tggcgaaacc cgacaggact ataaagatac 5040

caggcgtttc cccctggaag ctccctcgtg cgctctcctg ttccgaccct gccgcttacc 5100

ggatacctgt ccgcctttct cccttcggga agcgtggcgc tttctcatag ctcacgctgt 5160

aggtatctca gttcggtgta ggtcgttcgc tccaagctgg gctgtgtgca cgaacccccc 5220

gttcagcccg accgctgcgc cttatccggt aactatcgtc ttgagtccaa cccggtaaga 5280

cacgacttat cgccactggc agcagccact ggtaacagga ttagcagagc gaggtatgta 5340

ggcggtgcta cagagttctt gaagtggtgg cctaactacg gctacactag aaggacagta 5400

tttggtatct gcgctctgct gaagccagtt accttcggaa aaagagttgg tagctcttga 5460

tccggcaaac aaaccaccgc tggtagcggt ggtttttttg tttgcaagca gcagattacg 5520

cgcagaaaaa aaggatctca agaagatcct ttgatctttt ctacggggtc tgacgctcag 5580

tggaacgaaa actcacgtta agggattttg gtcatgagat tatcaaaaag gatcttcacc 5640

tagatccttt taaattaaaa atgaagtttt aaatcaatct aaagtatata tgagtaaact 5700

tggtctgaca gttaccaatg cttaatcagt gaggcaccta tctcagcgat ctgtctattt 5760

cgttcatcca tagttgcctg actccccgtc gtgtagataa ctacgatacg ggagggctta 5820

ccatctggcc ccagtgctgc aatgataccg cgagacccac gctcaccggc tccagattta 5880

tcagcaataa accagccagc cggaagggcc gagcgcagaa gtggtcctgc aactttatcc 5940

gcctccatcc agtctattaa ttgttgccgg gaagctagag taagtagttc gccagttaat 6000

agtttgcgca acgttgttac cattactaca ggcatcgtgg tgtcacgctc gtcgtttggt 6060

atggcttcat tcagctccgg ttcccaacga tcaaggcgag ttacatgatc ccccatgttg 6120

tgcaaaaaag cggttagctc cttcggtcct ccgatcgttg tcagaagtaa gttggccgca 6180

gtgttatcac tcatggttat ggcagcactg cataattctc ttactgtcat gccatccgta 6240

agatgctttt ctgtgactgg tgagtactca accaagtcat tctgagaata gtgtatgcgg 6300

cgaccgagtt gctcttgccc ggcgtcaata cgggataata ccgcgccaca tagcagaact 6360

ttaaaagtgc tcatcattgg aaaacgttct tcggggcgaa aactctcaag gatcttacca 6420

ctattgagat ccagttcgat gtaacccact cgtgcaccca actgatcttc agcatctttt 6480

actttcacca gcgtttctgg gtgagcaaaa acaggaaggc aaaatgccgc aaaaaaggga 6540

ataagggcga cacggaaatg ttgaatactc atactcttcc tttttcaata ttattgaagc 6600

atttatcagg gttattgtct catgagcgga tacatatttg aatgtattta gaaaaataaa 6660

caaatagggg ttccgcgcac atttccccga aagatgccac ctgaaattat aaacgttaat 6720

attttgttaa aattcgcgtt aaatttttgt taaatcagct cattttttaa ccaataggcc 6780

gaaatcggca aaatccctta taaatcaaaa gaatagaccg agatagggtt gagtgttgtt 6840

ccagtttgga acaagagtcc actattaaag aacgtggact ccaacgtcaa agggcgaaaa 6900

accgtctatc agggcgatgg cccactacgt gaaccatcac cctaatcaag ttttttgggg 6960

tcgaggtgcc gtaaagcact aaatcggaac cctaaaggga gcccccgatt tagagcttga 7020

cggggaaagc cggcgaacgt ggcgagaaag gaagggaaga aagcgaaagg agcgggcgct 7080

agggcgctgg caagtgtagc ggtcacgctg cgcgtaacca ccacacccgc cgcgcttaat 7140

gcgccgctac agggcgcgtc ccattcgcca ttcaggctgc gcaactgttg ggaagggcga 7200

tcggtgcggg cctcttcgct attacgccag ctggcgaaag ggggatgtgc tgcaaggcga 7260

ttaagttggg taacgccagg gttttcccag tcacgacgtt gtaaaacgac ggccagtgaa 7320

ttaggttaat taaggctgcg cgctcgctcg ctcactgagg ccgcccgggc aaagcccggg 7380

cgtcgggcga cctttggtcg cccggcctca gtgagcgagc gagcgcgcag agagggagtg 7440

gccaactcca tcactagggg ttccttgtag ttaatgatta acccgccatg ctacttatct 7500

acctgc 7506

<210>  17
<211>  7506
<212>  DNA
<213>  Artificial

<220>
<223>   C-Cas9-Intein_EFS-AcrIIA4-2xmir122BS-BGH (AAV construct)

<400>  17
ggccgccatt atggaatgtg tgtcagttag ggtgtggaaa gtccccaggc tccccagcag      60

gcagaagtat gcaaagcatg catctcaatt agtcagcaac cagtcccggt ctcctcccat     120

gcatgtcaat attggccatt agccatatta ttcattggtt atatagcata aatcaatatt     180

ggctattggc cattgcatac gttgtatcta tatcataata tgtacattta tattggctca     240

tgtccaatat gaccgccatg ttggcattga ttattgacta gttattaata gtaatcaatt     300

acggggtcat tagttcatag cccatatatg gagttccgcg ttacataact tacggtaaat     360

ggcccgcctg gctgaccgcc caacgacccc cgcccattga cgtcaataat gacgtatgtt     420

cccatagtaa cgccaatagg gactttccat tgacgtcaat gggtggagta tttacggtaa     480

actgcccact tggcagtaca tcaagtgtat catatgccaa gtccgccccc tattgacgtc     540

aatgacggta aatggcccgc ctggcattat gcccagtaca tgaccttacg ggactttcct     600

acttggcagt acatctacgt attagtcatc gctattacca tggtgatgcg gttttggcag     660

tacaccaatg ggcgtggata gcggtttgac tcacggggat ttccaagtct ccaccccatt     720

gacgtcaatg ggagtttgtt ttggcaccaa aatcaacggg actttccaaa atgtcgtaat     780

aaccccgccc cgttgacgca aatgggcggt aggcgtgtac ggtgggaggt ctatataagc     840

agaggtcgtt tagtgaaccg tcagatcact agtagcttta ttgcggtagt ttatcacagt     900

taaattgcta acgcagtcag tgctcgactg atcacaggta agtatcaagg ttacaagaca     960

ggtttaagga ggccaataga aactgggctt gtcgagacag agaagattct tgcgtttctg    1020

ataggcacct attggtctta ctgacatcca ctttgccttt ctctccacag gggtaccgaa     1080

gccgctagcg ctaccggtcg ccaccatggc ggcggcgtgc ccggaactgc gtcagctggc    1140

gcagagcgat gtgtattggg atccgattgt gagcattgaa ccggatggcg tggaagaagt    1200

gtttgatctg accgtgccgg gcccgcataa ctttgtggcg aacgatatta ttgcgcataa    1260

ctctggccag ggggacagtc ttcacgagca catcgctaat cttgcaggta gcccagctat    1320

caaaaaggga atactgcaga ccgttaaggt cgtggatgaa ctcgtcaaag taatgggaag    1380

gcataagccc gagaatatcg ttatcgagat ggcccgagag aaccaaacta cccagaaggg    1440

acagaagaac agtagggaaa ggatgaagag gattgaagag ggtataaaag aactggggtc    1500

ccaaatcctt aaggaacacc cagttgaaaa cacccagctt cagaatgaga agctctacct    1560

gtactacctg cagaacggca gggacatgta cgtggatcag gaactggaca tcaatcggct 1620

ctccgactac gacgtggatc atatcgtgcc ccagtctttt ctcaaagatg attctattga 1680

taataaagtg ttgacaagat ccgataaaaa tagagggaag agtgataacg tcccctcaga 1740

agaagttgtc aagaaaatga aaaattattg gcggcagctg ctgaacgcca aactgatcac 1800

acaacggaag ttcgataatc tgactaaggc tgaacgaggt ggcctgtctg agttggataa 1860

agccggcttc atcaaaaggc agcttgttga gacacgccag atcaccaagc acgtggccca 1920

aattctcgat tcacgcatga acaccaagta cgatgaaaat gacaaactga ttcgagaggt 1980

gaaagttatt actctgaagt ctaagctggt ctcagatttc agaaaggact ttcagtttta 2040

taaggtgaga gagatcaaca attaccacca tgcgcatgat gcctacctga atgcagtggt 2100

aggcactgca cttatcaaaa aatatcccaa gcttgaatct gaatttgttt acggagacta 2160

taaagtgtac gatgttagga aaatgatcgc aaagtctgag caggaaatag gcaaggccac 2220

cgctaagtac ttcttttaca gcaatattat gaattttttc aagaccgaga ttacactggc 2280

caatggagag attcggaagc gaccacttat cgaaacaaac ggagaaacag gagaaatcgt 2340

gtgggacaag ggtagggatt tcgcgacagt ccggaaggtc ctgtccatgc cgcaggtgaa 2400

catcgttaaa aagaccgaag tacagaccgg aggcttctcc aaggaaagta tcctcccgaa 2460

aaggaacagc gacaagctga tcgcacgcaa aaaagattgg gaccccaaga aatacggcgg 2520

attcgattct cctacagtcg cttacagtgt actggttgtg gccaaagtgg agaaagggaa 2580

gtctaaaaaa ctcaaaagcg tcaaggaact gctgggcatc acaatcatgg agcgatcaag 2640

cttcgaaaaa aaccccatcg actttctcga ggcgaaagga tataaagagg tcaaaaaaga 2700

cctcatcatt aagcttccca agtactctct ctttgagctt gaaaacggcc ggaaacgaat 2760

gctcgctagt gcgggcgagc tgcagaaagg taacgagctg gcactgccct ctaaatacgt 2820

taatttcttg tatctggcca gccactatga aaagctcaaa gggtctcccg aagataatga 2880

gcagaagcag ctgttcgtgg aacaacacaa acactacctt gatgagatca tcgagcaaat 2940

aagcgaattc tccaaaagag tgatcctcgc cgacgctaac ctcgataagg tgctttctgc 3000

ttacaataag cacagggata agcccatcag ggagcaggca gaaaacatta tccacttgtt 3060

tactctgacc aacttgggcg cgcctgcagc cttcaagtac ttcgacacca ccatagacag 3120

aaagcggtac acctctacaa aggaggtcct ggacgccaca ctgattcatc agtcaattac 3180

ggggctctat gaaacaagaa tcgacctctc tcagctcggt ggagacagca gggctgaccc 3240

caagaagaag aggaaggtgt ctcgagctcg atgagtttgg acaaaccaca actagaatgc 3300

agtgaaaaaa atgctttatt tgtgaaattt gtgatgctat tgctttattt gtaaccatta 3360

taagctgcaa taaacaagtt aacaacaaca attgcattca ttttatgttt caggttcagg 3420

gggaggtgtg ggaggttttt taaagcaagt aaaacctcta caaatgtggt atctagagct 3480

ccggtgcccg tcagtgggca gagcgcacat cgcccacagt ccccgagaag ttggggggag 3540

gggtcggcaa ttgatccggt gcctagagaa ggtggcgcgg ggtaaactgg gaaagtgatg 3600

tcgtgtactg gctccgcctt tttcccgagg gtgggggaga accgtatata agtgcagtag 3660

tcgccgtgaa cgttcttttt cgcaacgggt ttgccgccag aacacaggga tccgccacca 3720

tgaacattaa cgacctgatt cgggaaataa agaacaagga ctacacggtc aagctgtcag 3780

gcaccgactc caactctata acacagctca ttataagggt taataatgat ggcaacgaat 3840

atgtcatctc tgaatccgag aatgaatcaa tcgtggagaa attcatcagt gcatttaaaa 3900

acggctggaa ccaggagtac gaagatgagg aagagttcta caatgatatg caaactatca 3960

cactgaaatc tgagctgaac tagctcgagc ggccgccact gtgctggata tctgcagaat 4020

tacaaacacc attgtcacac tccattatat acaaacacca ttgtcacact ccatagctta 4080

agtttaaacc gctgatcagc ctcgactgtg ccttctagtt gccagccatc tgttgtttgc 4140

ccctcccccg tgccttcctt gaccctggaa ggtgccactc ccactgtcct ttcctaataa 4200

aatgaggaaa ttgcatcgca ttgtctgagt aggtgtcatt ctattctggg gggtggggtg 4260

gggcaggaca gcaaggggga ggattgggaa gacaatagca ggcatgctgg ggatgcggtg 4320

ggctctatgg acgcgtgcgg ccgcaggtag ataagtagca tggcgggtta atcattaact 4380

acaaggaacc cctagtgatg gagttggcca ctccctctct gcgcgctcgc tcgctcactg 4440

aggccgggcg accaaaggtc gcccgacgcc cgggctttgc ccgggcggcc tcagtgagcg 4500

agcgagcgcg cagcctaatt aaggccttaa ttaaatctgg cgtaatcatg gtcatagctg 4560

tttcctgtgt gaaattgtta tccgctcaca attccacaca acatacgagc cggaagcata 4620

aagtgtaaag cctggggtgc ctaatgagtg agctaactca cattaattgc gttgcgctca 4680

ctgcccgctt tccagtcggg aaacctgtcg tgccagctgc attaatgaat cggccaacgc 4740

gcggggagag gcggtttgcg tattgggcgc tcttccgctt cctcgctcac tgactcgctg 4800

cgctcggtcg ttcggctgcg gcgagcggta tcagctcact caaaggcggt aatacggtta 4860

tccacagaat caggggataa cgcaggaaag aacatgtgag caaaaccgca gcaaaaggcc 4920

aggaaccgta aaaaggccgc gttgctggcg tttttccata ggctccgccc ccctgacgag 4980

catcacaaaa atcgacgctc aagtcagagg tggcgaaacc cgacaggact ataaagatac 5040

caggcgtttc cccctggaag ctccctcgtg cgctctcctg ttccgaccct gccgcttacc 5100

ggatacctgt ccgcctttct cccttcggga agcgtggcgc tttctcatag ctcacgctgt 5160

aggtatctca gttcggtgta ggtcgttcgc tccaagctgg gctgtgtgca cgaacccccc 5220

gttcagcccg accgctgcgc cttatccggt aactatcgtc ttgagtccaa cccggtaaga 5280

cacgacttat cgccactggc agcagccact ggtaacagga ttagcagagc gaggtatgta 5340

ggcggtgcta cagagttctt gaagtggtgg cctaactacg gctacactag aaggacagta 5400

tttggtatct gcgctctgct gaagccagtt accttcggaa aaagagttgg tagctcttga 5460

tccggcaaac aaaccaccgc tggtagcggt ggtttttttg tttgcaagca gcagattacg 5520

cgcagaaaaa aaggatctca agaagatcct ttgatctttt ctacggggtc tgacgctcag 5580

tggaacgaaa actcacgtta agggattttg gtcatgagat tatcaaaaag gatcttcacc 5640

tagatccttt taaattaaaa atgaagtttt aaatcaatct aaagtatata tgagtaaact 5700

tggtctgaca gttaccaatg cttaatcagt gaggcaccta tctcagcgat ctgtctattt 5760

cgttcatcca tagttgcctg actccccgtc gtgtagataa ctacgatacg ggagggctta 5820

ccatctggcc ccagtgctgc aatgataccg cgagacccac gctcaccggc tccagattta 5880

tcagcaataa accagccagc cggaagggcc gagcgcagaa gtggtcctgc aactttatcc 5940

gcctccatcc agtctattaa ttgttgccgg gaagctagag taagtagttc gccagttaat 6000

agtttgcgca acgttgttac cattactaca ggcatcgtgg tgtcacgctc gtcgtttggt 6060

atggcttcat tcagctccgg ttcccaacga tcaaggcgag ttacatgatc ccccatgttg 6120

tgcaaaaaag cggttagctc cttcggtcct ccgatcgttg tcagaagtaa gttggccgca 6180

gtgttatcac tcatggttat ggcagcactg cataattctc ttactgtcat gccatccgta 6240

agatgctttt ctgtgactgg tgagtactca accaagtcat tctgagaata gtgtatgcgg 6300

cgaccgagtt gctcttgccc ggcgtcaata cgggataata ccgcgccaca tagcagaact 6360

ttaaaagtgc tcatcattgg aaaacgttct tcggggcgaa aactctcaag gatcttacca 6420

ctattgagat ccagttcgat gtaacccact cgtgcaccca actgatcttc agcatctttt 6480

actttcacca gcgtttctgg gtgagcaaaa acaggaaggc aaaatgccgc aaaaaaggga 6540

ataagggcga cacggaaatg ttgaatactc atactcttcc tttttcaata ttattgaagc 6600

atttatcagg gttattgtct catgagcgga tacatatttg aatgtattta gaaaaataaa 6660

caaatagggg ttccgcgcac atttccccga aagatgccac ctgaaattat aaacgttaat 6720

attttgttaa aattcgcgtt aaatttttgt taaatcagct cattttttaa ccaataggcc 6780

gaaatcggca aaatccctta taaatcaaaa gaatagaccg agatagggtt gagtgttgtt 6840

ccagtttgga acaagagtcc actattaaag aacgtggact ccaacgtcaa agggcgaaaa 6900

accgtctatc agggcgatgg cccactacgt gaaccatcac cctaatcaag ttttttgggg 6960

tcgaggtgcc gtaaagcact aaatcggaac cctaaaggga gcccccgatt tagagcttga 7020

cggggaaagc cggcgaacgt ggcgagaaag gaagggaaga aagcgaaagg agcgggcgct 7080

agggcgctgg caagtgtagc ggtcacgctg cgcgtaacca ccacacccgc cgcgcttaat 7140

gcgccgctac agggcgcgtc ccattcgcca ttcaggctgc gcaactgttg ggaagggcga 7200

tcggtgcggg cctcttcgct attacgccag ctggcgaaag ggggatgtgc tgcaaggcga 7260

ttaagttggg taacgccagg gttttcccag tcacgacgtt gtaaaacgac ggccagtgaa 7320

ttaggttaat taaggctgcg cgctcgctcg ctcactgagg ccgcccgggc aaagcccggg 7380

cgtcgggcga cctttggtcg cccggcctca gtgagcgagc gagcgcgcag agagggagtg 7440

gccaactcca tcactagggg ttccttgtag ttaatgatta acccgccatg ctacttatct 7500

acctgc 7506

<210> 18
<211> 7409
<212> DNA
<213> Artificial

<220>
<223> C-Cas9-Intein_EFS-FLAG-NLS-AcrIIA4-2xmir122BS-miniPolyA (AAV
      construct)

<400> 18
ggccgccatt atggaatgtg tgtcagttag ggtgtggaaa gtccccaggc tccccagcag 60

gcagaagtat gcaaagcatg catctcaatt agtcagcaac cagtcccggt ctcctcccat 120

gcatgtcaat attggccatt agccatatta ttcattggtt atatagcata aatcaatatt 180

ggctattggc cattgcatac gttgtatcta tatcataata tgtacattta tattggctca 240

tgtccaatat gaccgccatg ttggcattga ttattgacta gttattaata gtaatcaatt 300

acggggtcat tagttcatag cccatatatg gagttccgcg ttacataact tacggtaaat 360

ggcccgcctg gctgaccgcc caacgacccc cgcccattga cgtcaataat gacgtatgtt 420

cccatagtaa cgccaatagg gactttccat tgacgtcaat gggtggagta tttacggtaa 480

actgcccact tggcagtaca tcaagtgtat catatgccaa gtccgccccc tattgacgtc 540

aatgacggta aatggcccgc ctggcattat gcccagtaca tgaccttacg ggactttcct 600

acttggcagt acatctacgt attagtcatc gctattacca tggtgatgcg gttttggcag 660

tacaccaatg ggcgtggata gcggtttgac tcacggggat ttccaagtct ccaccccatt 720

gacgtcaatg ggagtttgtt ttggcaccaa aatcaacggg actttccaaa atgtcgtaat 780

aaccccgccc cgttgacgca aatgggcggt aggcgtgtac ggtgggaggt ctatataagc 840

agaggtcgtt tagtgaaccg tcagatcact agtagcttta ttgcggtagt ttatcacagt 900

taaattgcta acgcagtcag tgctcgactg atcacaggta agtatcaagg ttacaagaca 960

ggtttaagga ggccaataga aactgggctt gtcgagacag agaagattct tgcgtttctg 1020

ataggcacct attggtctta ctgacatcca ctttgccttt ctctccacag gggtaccgaa 1080

gccgctagcg ctaccggtcg ccaccatggc ggcggcgtgc ccggaactgc gtcagctggc 1140

gcagagcgat gtgtattggg atccgattgt gagcattgaa ccggatggcg tggaagaagt 1200

gtttgatctg accgtgccgg gcccgcataa ctttgtggcg aacgatatta ttgcgcataa 1260

ctctggccag ggggacagtc ttcacgagca catcgctaat cttgcaggta gcccagctat 1320

caaaaaggga atactgcaga ccgttaaggt cgtggatgaa ctcgtcaaag taatgggaag 1380

gcataagccc gagaatatcg ttatcgagat ggcccgagag aaccaaacta cccagaaggg 1440

acagaagaac agtagggaaa ggatgaagag gattgaagag ggtataaaag aactggggtc 1500

ccaaatcctt aaggaacacc cagttgaaaa cacccagctt cagaatgaga agctctacct 1560

gtactacctg cagaacggca gggacatgta cgtggatcag gaactggaca tcaatcggct 1620

ctccgactac gacgtggatc atatcgtgcc ccagtctttt ctcaaagatg attctattga 1680

taataaagtg ttgacaagat ccgataaaaa tagagggaag agtgataacg tcccctcaga 1740

agaagttgtc aagaaaatga aaaattattg gcggcagctg ctgaacgcca aactgatcac 1800

acaacggaag ttcgataatc tgactaaggc tgaacgaggt ggcctgtctg agttggataa 1860

agccggcttc atcaaaaggc agcttgttga gacacgccag atcaccaagc acgtggccca 1920

aattctcgat tcacgcatga acaccaagta cgatgaaaat gacaaactga ttcgagaggt 1980

gaaagttatt actctgaagt ctaagctggt ctcagatttc agaaaggact ttcagtttta 2040

taaggtgaga gagatcaaca attaccacca tgcgcatgat gcctacctga atgcagtggt 2100

aggcactgca cttatcaaaa aatatcccaa gcttgaatct gaatttgttt acggagacta 2160

taaagtgtac gatgttagga aaatgatcgc aaagtctgag caggaaatag gcaaggccac 2220

cgctaagtac ttcttttaca gcaatattat gaattttttc aagaccgaga ttacactggc 2280

caatggagag attcggaagc gaccacttat cgaaacaaac ggagaaacag gagaaatcgt 2340

gtgggacaag ggtagggatt tcgcgacagt ccggaaggtc ctgtccatgc cgcaggtgaa 2400

catcgttaaa aagaccgaag tacagaccgg aggcttctcc aaggaaagta tcctcccgaa 2460

aaggaacagc gacaagctga tcgcacgcaa aaaagattgg gaccccaaga aatacggcgg 2520

attcgattct cctacagtcg cttacagtgt actggttgtg gccaaagtgg agaaagggaa 2580

gtctaaaaaa ctcaaaagcg tcaaggaact gctgggcatc acaatcatgg agcgatcaag 2640

cttcgaaaaa aaccccatcg actttctcga ggcgaaagga tataaagagg tcaaaaaaga 2700

cctcatcatt aagcttccca agtactctct ctttgagctt gaaaacggcc ggaaacgaat 2760

gctcgctagt gcgggcgagc tgcagaaagg taacgagctg gcactgccct ctaaatacgt 2820

taatttcttg tatctggcca gccactatga aaagctcaaa gggtctcccg aagataatga 2880

gcagaagcag ctgttcgtgg aacaacacaa acactacctt gatgagatca tcgagcaaat 2940

aagcgaattc tccaaaagag tgatcctcgc cgacgctaac ctcgataagg tgctttctgc 3000

ttacaataag cacagggata agcccatcag ggagcaggca gaaaacatta tccacttgtt 3060

tactctgacc aacttgggcg cgcctgcagc cttcaagtac ttcgacacca ccatagacag 3120

aaagcggtac acctctacaa aggaggtcct ggacgccaca ctgattcatc agtcaattac 3180

ggggctctat gaaacaagaa tcgacctctc tcagctcggt ggagacagca gggctgaccc 3240

caagaagaag aggaaggtgt ctcgagctcg atgagtttgg acaaaccaca actagaatgc 3300

agtgaaaaaa atgctttatt tgtgaaattt gtgatgctat tgctttattt gtaaccatta 3360

taagctgcaa taaacaagtt aacaacaaca attgcattca ttttatgttt caggttcagg 3420

gggaggtgtg ggaggttttt taaagcaagt aaaacctcta caaatgtggt atctagagct 3480

ccggtgcccg tcagtgggca gagcgcacat cgcccacagt ccccgagaag ttggggggag 3540

gggtcggcaa ttgatccggt gcctagagaa ggtggcgcgg ggtaaactgg gaaagtgatg 3600

tcgtgtactg gctccgcctt tttcccgagg gtgggggaga accgtatata agtgcagtag 3660

tcgccgtgaa cgttcttttt cgcaacgggt ttgccgccag aacacaggga tccgccacca 3720

tggactataa ggacgatgac gataaaccca agaagaagcg aaaagtcgga ggttccatga 3780

acattaacga cctgattcgg gaaataaaga acaaggacta cacggtcaag ctgtcaggca 3840

ccgactccaa ctctataaca cagctcatta taagggttaa taatgatggc aacgaatatg 3900

tcatctctga atccgagaat gaatcaatcg tggagaaatt catcagtgca tttaaaaacg 3960

gctggaacca ggagtacgaa gatgaggaag agttctacaa tgatatgcaa actatcacac 4020

tgaaatctga gctgaactag ctcgagcggc cgccactgtg ctggatatct gcagaattca 4080

aacaccattg tcacactcca ttatatcaaa caccattgtc acactccatt aattagagac 4140

gagctcgtct ctagcttaag tttaaaccgc tgatcagcct cgagctagca ataaaggatc 4200

gtttattttc attggaagcg tgtgttggtt tttacgcgtg cggccgcagg tagataagta 4260

gcatggcggg ttaatcatta actacaagga acccctagtg atggagttgg ccactccctc 4320

tctgcgcgct cgctcgctca ctgaggccgg gcgaccaaag gtcgcccgac gcccgggctt 4380

tgcccgggcg gcctcagtga gcgagcgagc gcgcagccta attaaggcct taattaaatc 4440

tggcgtaatc atggtcatag ctgtttcctg tgtgaaattg ttatccgctc acaattccac 4500

acaacatacg agccggaagc ataaagtgta aagcctgggg tgcctaatga gtgagctaac 4560

tcacattaat tgcgttgcgc tcactgcccg ctttccagtc gggaaacctg tcgtgccagc 4620

tgcattaatg aatcggccaa cgcgcgggga gaggcggttt gcgtattggg cgctcttccg 4680

cttcctcgct cactgactcg ctgcgctcgg tcgttcggct gcggcgagcg gtatcagctc 4740

actcaaaggc ggtaatacgg ttatccacag aatcagggga taacgcagga aagaacatgt 4800

gagcaaaacc gcagcaaaag gccaggaacc gtaaaaaggc cgcgttgctg gcgtttttcc 4860

ataggctccg cccccctgac gagcatcaca aaaatcgacg ctcaagtcag aggtggcgaa 4920

acccgacagg actataaaga taccaggcgt ttccccctgg aagctccctc gtgcgctctc 4980

ctgttccgac cctgccgctt accggatacc tgtccgcctt tctcccttcg ggaagcgtgg 5040

cgctttctca tagctcacgc tgtaggtatc tcagttcggt gtaggtcgtt cgctccaagc 5100

tgggctgtgt gcacgaaccc cccgttcagc ccgaccgctg cgccttatcc ggtaactatc 5160

gtcttgagtc caacccggta agacacgact tatcgccact ggcagcagcc actggtaaca 5220

ggattagcag agcgaggtat gtaggcggtg ctacagagtt cttgaagtgg tggcctaact 5280

acggctacac tagaaggaca gtatttggta tctgcgctct gctgaagcca gttaccttcg 5340

gaaaaagagt tggtagctct tgatccggca aacaaaccac cgctggtagc ggtggttttt 5400

ttgtttgcaa gcagcagatt acgcgcagaa aaaaaggatc tcaagaagat cctttgatct 5460

tttctacggg gtctgacgct cagtggaacg aaaactcacg ttaagggatt ttggtcatga 5520

gattatcaaa aaggatcttc acctagatcc ttttaaatta aaaatgaagt tttaaatcaa 5580

tctaaagtat atatgagtaa acttggtctg acagttacca atgcttaatc agtgaggcac 5640

ctatctcagc gatctgtcta tttcgttcat ccatagttgc ctgactcccc gtcgtgtaga 5700

taactacgat acgggagggc ttaccatctg gccccagtgc tgcaatgata ccgcgagacc 5760

cacgctcacc ggctccagat ttatcagcaa taaaccagcc agccggaagg gccgagcgca 5820

gaagtggtcc tgcaacttta tccgcctcca tccagtctat taattgttgc cgggaagcta 5880

gagtaagtag ttcgccagtt aatagtttgc gcaacgttgt taccattact acaggcatcg 5940

tggtgtcacg ctcgtcgttt ggtatggctt cattcagctc cggttcccaa cgatcaaggc 6000

gagttacatg atcccccatg ttgtgcaaaa aagcggttag ctccttcggt cctccgatcg 6060

ttgtcagaag taagttggcc gcagtgttat cactcatggt tatggcagca ctgcataatt 6120

ctcttactgt catgccatcc gtaagatgct tttctgtgac tggtgagtac tcaaccaagt 6180

cattctgaga atagtgtatg cggcgaccga gttgctcttg cccggcgtca atacgggata 6240

ataccgcgcc acatagcaga actttaaaag tgctcatcat tggaaaacgt tcttcggggc 6300

gaaaactctc aaggatctta ccgctgttga gatccagttc gatgtaaccc actcgtgcac 6360

ccaactgatc ttcagcatct tttactttca ccagcgtttc tgggtgagca aaaacaggaa 6420

ggcaaaatgc cgcaaaaaag ggaataaggg cgacacggaa atgttgaata ctcatactct 6480

tcctttttca atattattga agcatttatc agggttattg tctcatgagc ggatacatat 6540

ttgaatgtat ttagaaaaat aaacaaatag gggttccgcg cacatttccc cgaaaagtgc 6600

cacctgaaat tataaacgtt aatattttgt taaaattcgc gttaaatttt tgttaaatca 6660

gctcattttt taaccaatag gccgaaatcg gcaaaatccc ttataaatca aaagaataga 6720

ccgagatagg gttgagtgtt gttccagttt ggaacaagag tccactatta aagaacgtgg 6780

actccaacgt caaagggcga aaaaccgtct atcagggcga tggcccacta cgtgaaccat 6840

caccctaatc aagttttttg gggtcgaggt gccgtaaagc actaaatcgg aaccctaaag 6900

ggagcccccg atttagagct tgacggggaa agccggcgaa cgtggcgaga aaggaaggga 6960

agaaagcgaa aggagcgggc gctagggcgc tggcaagtgt agcggtcacg ctgcgcgtaa 7020

ccaccacacc cgccgcgctt aatgcgccgc tacagggcgc gtcccattcg ccattcaggc 7080

tgcgcaactg ttgggaaggg cgatcggtgc gggcctcttc gctattacgc cagctggcga 7140

aagggggatg tgctgcaagg cgattaagtt gggtaacgcc agggttttcc cagtcacgac 7200

gttgtaaaac gacggccagt gaattaggtt aattaaggct gcgcgctcgc tcgctcactg 7260

aggccgcccg ggcaaagccc gggcgtcggg cgacctttgg tcgcccggcc tcagtgagcg 7320

agcgagcgcg cagagaggga gtggccaact ccatcactag gggttccttg tagttaatga 7380

ttaacccgcc atgctactta tctacctgc 7409

<210> 19
<211> 7388
<212> DNA
<213> Artificial

<220>
<223> C-Cas9-Intein_EFS-FLAG-NLS-AcrIIA4-scaffold-miniPolyA (AAV
      construct)

<400> 19
ggccgccatt atggaatgtg tgtcagttag ggtgtggaaa gtccccaggc tccccagcag 60

gcagaagtat gcaaagcatg catctcaatt agtcagcaac cagtcccggt ctcctcccat 120

gcatgtcaat attggccatt agccatatta ttcattggtt atatagcata aatcaatatt 180

ggctattggc cattgcatac gttgtatcta tatcataata tgtacattta tattggctca 240

tgtccaatat gaccgccatg ttggcattga ttattgacta gttattaata gtaatcaatt 300

acggggtcat tagttcatag cccatatatg gagttccgcg ttacataact tacggtaaat 360

ggcccgcctg gctgaccgcc caacgacccc cgcccattga cgtcaataat gacgtatgtt 420

cccatagtaa cgccaatagg gactttccat tgacgtcaat gggtggagta tttacggtaa 480

actgcccact tggcagtaca tcaagtgtat catatgccaa gtccgccccc tattgacgtc 540

aatgacggta aatggcccgc ctggcattat gcccagtaca tgaccttacg ggactttcct 600

acttggcagt acatctacgt attagtcatc gctattacca tggtgatgcg gttttggcag 660

tacaccaatg ggcgtggata gcggtttgac tcacggggat ttccaagtct ccaccccatt 720

gacgtcaatg ggagtttgtt ttggcaccaa aatcaacggg actttccaaa atgtcgtaat 780

aaccccgccc cgttgacgca aatgggcggt aggcgtgtac ggtgggaggt ctatataagc 840

agaggtcgtt tagtgaaccg tcagatcact agtagcttta ttgcggtagt ttatcacagt 900

taaattgcta acgcagtcag tgctcgactg atcacaggta agtatcaagg ttacaagaca 960

ggtttaagga ggccaataga aactgggctt gtcgagacag agaagattct tgcgtttctg 1020

ataggcacct attggtctta ctgacatcca ctttgccttt ctctccacag gggtaccgaa 1080

gccgctagcg ctaccggtcg ccaccatggc ggcggcgtgc ccggaactgc gtcagctggc 1140

gcagagcgat gtgtattggg atccgattgt gagcattgaa ccggatggcg tggaagaagt 1200

gtttgatctg accgtgccgg gcccgcataa ctttgtggcg aacgatatta ttgcgcataa 1260

ctctggccag ggggacagtc ttcacgagca catcgctaat cttgcaggta gcccagctat 1320

caaaaaggga atactgcaga ccgttaaggt cgtggatgaa ctcgtcaaag taatgggaag 1380

gcataagccc gagaatatcg ttatcgagat ggcccgagag aaccaaacta cccagaaggg 1440

acagaagaac agtagggaaa ggatgaagag gattgaagag ggtataaaag aactggggtc 1500

ccaaatcctt aaggaacacc cagttgaaaa cacccagctt cagaatgaga agctctacct 1560

gtactacctg cagaacggca gggacatgta cgtggatcag gaactggaca tcaatcggct 1620

ctccgactac gacgtggatc atatcgtgcc ccagtctttt ctcaaagatg attctattga 1680

taataaagtg ttgacaagat ccgataaaaa tagagggaag agtgataacg tcccctcaga 1740

agaagttgtc aagaaaatga aaaattattg gcggcagctg ctgaacgcca aactgatcac 1800

acaacggaag ttcgataatc tgactaaggc tgaacgaggt ggcctgtctg agttggataa 1860

agccggcttc atcaaaaggc agcttgttga gacacgccag atcaccaagc acgtggccca 1920

aattctcgat tcacgcatga acaccaagta cgatgaaaat gacaaactga ttcgagaggt 1980

gaaagttatt actctgaagt ctaagctggt ctcagatttc agaaaggact ttcagtttta 2040

taaggtgaga gagatcaaca attaccacca tgcgcatgat gcctacctga atgcagtggt 2100

aggcactgca cttatcaaaa aatatcccaa gcttgaatct gaatttgttt acggagacta 2160

taaagtgtac gatgttagga aaatgatcgc aaagtctgag caggaaatag gcaaggccac 2220

cgctaagtac ttcttttaca gcaatattat gaattttttc aagaccgaga ttacactggc 2280

caatggagag attcggaagc gaccacttat cgaaacaaac ggagaaacag gagaaatcgt 2340

gtgggacaag ggtagggatt tcgcgacagt ccggaaggtc ctgtccatgc cgcaggtgaa 2400

catcgttaaa aagaccgaag tacagaccgg aggcttctcc aaggaaagta tcctcccgaa 2460

aaggaacagc gacaagctga tcgcacgcaa aaaagattgg gaccccaaga aatacggcgg 2520

attcgattct cctacagtcg cttacagtgt actggttgtg gccaaagtgg agaaagggaa 2580

gtctaaaaaa ctcaaaagcg tcaaggaact gctgggcatc acaatcatgg agcgatcaag 2640

cttcgaaaaa aaccccatcg actttctcga ggcgaaagga tataaagagg tcaaaaaaga 2700

cctcatcatt aagcttccca agtactctct ctttgagctt gaaaacggcc ggaaacgaat 2760

gctcgctagt gcgggcgagc tgcagaaagg taacgagctg gcactgccct ctaaatacgt 2820

taatttcttg tatctggcca gccactatga aaagctcaaa gggtctcccg aagataatga 2880

gcagaagcag ctgttcgtgg aacaacacaa acactacctt gatgagatca tcgagcaaat 2940

aagcgaattc tccaaaagag tgatcctcgc cgacgctaac ctcgataagg tgctttctgc 3000

ttacaataag cacagggata agcccatcag ggagcaggca gaaaacatta tccacttgtt 3060

tactctgacc aacttgggcg cgcctgcagc cttcaagtac ttcgacacca ccatagacag 3120

aaagcggtac acctctacaa aggaggtcct ggacgccaca ctgattcatc agtcaattac 3180

ggggctctat gaaacaagaa tcgacctctc tcagctcggt ggagacagca gggctgaccc 3240

caagaagaag aggaaggtgt ctcgagctcg atgagtttgg acaaaccaca actagaatgc 3300

agtgaaaaaa atgctttatt tgtgaaattt gtgatgctat tgctttattt gtaaccatta 3360

taagctgcaa taaacaagtt aacaacaaca attgcattca ttttatgttt caggttcagg 3420

gggaggtgtg ggaggttttt taaagcaagt aaaacctcta caaatgtggt atctagagct 3480

ccggtgcccg tcagtgggca gagcgcacat cgcccacagt ccccgagaag ttggggggag 3540

gggtcggcaa ttgatccggt gcctagagaa ggtggcgcgg ggtaaactgg gaaagtgatg 3600

tcgtgtactg gctccgcctt tttcccgagg gtgggggaga accgtatata agtgcagtag 3660

tcgccgtgaa cgttcttttt cgcaacgggt ttgccgccag aacacaggga tccgccacca 3720

tggactataa ggacgatgac gataaaccca agaagaagcg aaaagtcgga ggttccatga 3780

acattaacga cctgattcgg gaaataaaga acaaggacta cacggtcaag ctgtcaggca 3840

ccgactccaa ctctataaca cagctcatta taagggttaa taatgatggc aacgaatatg 3900

tcatctctga atccgagaat gaatcaatcg tggagaaatt catcagtgca tttaaaaacg 3960

gctggaacca ggagtacgaa gatgaggaag agttctacaa tgatatgcaa actatcacac 4020

tgaaatctga gctgaactag ctcgagcggc cgccactgtg ctggatatct gcagaattag 4080

agacgccgtt gatgttgaag tttatatacc gttgatgttg aagtcgtctc tagcttaagt 4140

ttaaaccgct gatcagcctc gagctagcaa taaaggatcg tttattttca ttggaagcgt 4200

gtgttggttt ttacgcgtgc ggccgcaggt agataagtag catggcgggt taatcattaa 4260

ctacaaggaa cccctagtga tggagttggc cactccctct ctgcgcgctc gctcgctcac 4320

tgaggccggg cgaccaaagg tcgcccgacg cccgggcttt gcccgggcgg cctcagtgag 4380

cgagcgagcg cgcagcctaa ttaaggcctt aattaaatct ggcgtaatca tggtcatagc 4440

tgtttcctgt gtgaaattgt tatccgctca caattccaca caacatacga gccggaagca 4500

taaagtgtaa agcctggggt gcctaatgag tgagctaact cacattaatt gcgttgcgct 4560

cactgcccgc tttccagtcg ggaaacctgt cgtgccagct gcattaatga atcggccaac 4620

gcgcggggag aggcggtttg cgtattgggc gctcttccgc ttcctcgctc actgactcgc 4680

tgcgctcggt cgttcggctg cggcgagcgg tatcagctca ctcaaaggcg gtaatacggt 4740

tatccacaga atcaggggat aacgcaggaa agaacatgtg agcaaaaccg cagcaaaagg 4800

ccaggaaccg taaaaaggcc gcgttgctgg cgtttttcca taggctccgc ccccctgacg 4860

agcatcacaa aaatcgacgc tcaagtcaga ggtggcgaaa cccgacagga ctataaagat 4920

accaggcgtt tccccctgga agctccctcg tgcgctctcc tgttccgacc ctgccgctta 4980

ccggatacct gtccgccttt ctcccttcgg gaagcgtggc gctttctcat agctcacgct 5040

gtaggtatct cagttcggtg taggtcgttc gctccaagct gggctgtgtg cacgaacccc 5100

ccgttcagcc cgaccgctgc gccttatccg gtaactatcg tcttgagtcc aacccggtaa 5160

gacacgactt atcgccactg gcagcagcca ctggtaacag gattagcaga gcgaggtatg 5220

taggcggtgc tacagagttc ttgaagtggt ggcctaacta cggctacact agaaggacag 5280

tatttggtat ctgcgctctg ctgaagccag ttaccttcgg aaaaagagtt ggtagctctt 5340

gatccggcaa acaaaccacc gctggtagcg gtggtttttt tgtttgcaag cagcagatta 5400

cgcgcagaaa aaaaggatct caagaagatc ctttgatctt ttctacgggg tctgacgctc 5460

agtggaacga aaactcacgt taagggattt tggtcatgag attatcaaaa aggatcttca 5520

cctagatcct tttaaattaa aaatgaagtt ttaaatcaat ctaaagtata tatgagtaaa 5580

cttggtctga cagttaccaa tgcttaatca gtgaggcacc tatctcagcg atctgtctat 5640

ttcgttcatc catagttgcc tgactccccg tcgtgtagat aactacgata cgggagggct 5700

taccatctgg ccccagtgct gcaatgatac cgcgagaccc acgctcaccg gctccagatt 5760

tatcagcaat aaaccagcca gccggaaggg ccgagcgcag aagtggtcct gcaactttat 5820

ccgcctccat ccagtctatt aattgttgcc gggaagctag agtaagtagt tcgccagtta 5880

atagtttgcg caacgttgtt accattacta caggcatcgt ggtgtcacgc tcgtcgtttg 5940

gtatggcttc attcagctcc ggttcccaac gatcaaggcg agttacatga tcccccatgt 6000

tgtgcaaaaa agcggttagc tccttcggtc ctccgatcgt tgtcagaagt aagttggccg 6060

cagtgttatc actcatggtt atggcagcac tgcataattc tcttactgtc atgccatccg 6120

taagatgctt ttctgtgact ggtgagtact caaccaagtc attctgagaa tagtgtatgc 6180

ggcgaccgag ttgctcttgc ccggcgtcaa tacgggataa taccgcgcca catagcagaa 6240

ctttaaaagt gctcatcatt ggaaaacgtt cttcggggcg aaaactctca aggatcttac 6300

cactattgag atccagttcg atgtaaccca ctcgtgcacc caactgatct tcagcatctt 6360

ttactttcac cagcgtttct gggtgagcaa aaacaggaag gcaaaatgcc gcaaaaaagg 6420

gaataagggc gacacggaaa tgttgaatac tcatactctt cctttttcaa tattattgaa 6480

gcatttatca gggttattgt ctcatgagcg gatacatatt tgaatgtatt tagaaaaata 6540

aacaaatagg ggttccgcgc acatttcccc gaaaagtgcc acctgaaatt ataaacgtta 6600

atattttgtt aaaattcgcg ttaaattttt gttaaatcag ctcatttttt aaccaatagg 6660

ccgaaatcgg caaaatccct tataaatcaa aagaatagac cgagataggg ttgagtgttg 6720

ttccagtttg gaacaagagt ccactattaa agaacgtgga ctccaacgtc aaagggcgaa 6780

aaaccgtcta tcagggcgat ggcccactac gtgaaccatc accctaatca agttttttgg 6840

ggtcgaggtg ccgtaaagca ctaaatcgga accctaaagg gagcccccga tttagagctt 6900

gacggggaaa gccggcgaac gtggcgagaa aggaagggaa gaaagcgaaa ggagcgggcg 6960

ctagggcgct ggcaagtgta gcggtcacgc tgcgcgtaac caccacaccc gccgcgctta 7020

atgcgccgct acagggcgcg tcccattcgc cattcaggct gcgcaactgt tgggaagggc 7080

gatcggtgcg ggcctcttcg ctattacgcc agctggcgaa agggggatgt gctgcaaggc 7140

gattaagttg ggtaacgcca gggttttccc agtcacgacg ttgtaaaacg acggccagtg 7200

aattaggtta attaaggctg cgcgctcgct cgctcactga ggccgcccgg gcaaagcccg 7260

ggcgtcgggc gacctttggt cgcccggcct cagtgagcga gcgagcgcgc agagagggag 7320

tggccaactc catcactagg ggttccttgt agttaatgat taacccgcca tgctacttat 7380

ctacctgc 7388

<210> 20
<211> 87
<212> PRT
<213> Listeria monocytogenes

<400> 20

<210> 21
<211> 261
<212> DNA
<213> Listeria monocytogenes

<400> 21

atgaacatta acgacctgat tcgggaaata aagaacaagg actacacggt caagctgtca 60

ggcaccgact ccaactctat aacacagctc attataaggg ttaataatga tggcaacgaa 120

tatgtcatct ctgaatccga gaatgaatca atcgtggaga aattcatcag tgcatttaaa 180

aacggctgga accaggagta cgaagatgag gaagagttct acaatgatat gcaaactatc 240

acactgaaat ctgagctgaa c 261

<210> 22
<211> 712
<212> PRT
<213> Artificial

<220>
<223> Cas9 S. pyogenes N-terminal fragment (N-Cas9)

<400> 22

Asp Lys Lys Tyr Ser Ile Gly Leu Asp Ile Gly Thr Asn Ser Val Gly
1               5                   10                  15

Trp Ala Val Ile Thr Asp Glu Tyr Lys Val Pro Ser Lys Lys Phe Lys
            20                  25                  30

Val Leu Gly Asn Thr Asp Arg His Ser Ile Lys Lys Asn Leu Ile Gly
        35                  40                  45

Ala Leu Leu Phe Asp Ser Gly Glu Thr Ala Glu Ala Thr Arg Leu Lys
    50                  55                  60

Arg Thr Ala Arg Arg Arg Tyr Thr Arg Arg Lys Asn Arg Ile Cys Tyr
65                  70                  75                  80

Leu Gln Glu Ile Phe Ser Asn Glu Met Ala Lys Val Asp Asp Ser Phe
                85                  90                  95

Phe His Arg Leu Glu Glu Ser Phe Leu Val Glu Glu Asp Lys Lys His
            100                 105                 110

Glu Arg His Pro Ile Phe Gly Asn Ile Val Asp Glu Val Ala Tyr His
        115                 120                 125

Glu Lys Tyr Pro Thr Ile Tyr His Leu Arg Lys Lys Leu Val Asp Ser
    130                 135                 140

Thr Asp Lys Ala Asp Leu Arg Leu Ile Tyr Leu Ala Leu Ala His Met
145                 150                 155                 160

Ile Lys Phe Arg Gly His Phe Leu Ile Glu Gly Asp Leu Asn Pro Asp
                165                 170                 175

Asn Ser Asp Val Asp Lys Leu Phe Ile Gln Leu Val Gln Thr Tyr Asn
            180                 185                 190

Gln Leu Phe Glu Glu Asn Pro Ile Asn Ala Ser Gly Val Asp Ala Lys
        195                 200                 205

Ala Ile Leu Ser Ala Arg Leu Ser Lys Ser Arg Arg Leu Glu Asn Leu
    210                 215                 220

Ile Ala Gln Leu Pro Gly Glu Lys Lys Asn Gly Leu Phe Gly Asn Leu
225                 230                 235                 240

Ile Ala Leu Ser Leu Gly Leu Thr Pro Asn Phe Lys Ser Asn Phe Asp
                245                 250                 255

Leu Ala Glu Asp Ala Lys Leu Gln Leu Ser Lys Asp Thr Tyr Asp Asp
            260                 265                 270

Asp Leu Asp Asn Leu Leu Ala Gln Ile Gly Asp Gln Tyr Ala Asp Leu
        275                 280                 285

Phe Leu Ala Ala Lys Asn Leu Ser Asp Ala Ile Leu Leu Ser Asp Ile
    290                 295                 300

Leu Arg Val Asn Thr Glu Ile Thr Lys Ala Pro Leu Ser Ala Ser Met
305                 310                 315                 320

Ile Lys Arg Tyr Asp Glu His His Gln Asp Leu Thr Leu Leu Lys Ala
                325                 330                 335

Leu Val Arg Gln Gln Leu Pro Glu Lys Tyr Lys Glu Ile Phe Phe Asp
            340                 345                 350

Gln Ser Lys Asn Gly Tyr Ala Gly Tyr Ile Asp Gly Gly Ala Ser Gln
        355                 360                 365

Glu Glu Phe Tyr Lys Phe Ile Lys Pro Ile Leu Glu Lys Met Asp Gly
    370                 375                 380

Thr Glu Glu Leu Leu Val Lys Leu Asn Arg Glu Asp Leu Leu Arg Lys
385                 390                 395                 400

Gln Arg Thr Phe Asp Asn Gly Ser Ile Pro His Gln Ile His Leu Gly
                405                 410                 415

Glu Leu His Ala Ile Leu Arg Arg Gln Glu Asp Phe Tyr Pro Phe Leu

            420                 425                 430

Lys Asp Asn Arg Glu Lys Ile Glu Lys Ile Leu Thr Phe Arg Ile Pro
        435                 440                 445

Tyr Tyr Val Gly Pro Leu Ala Arg Gly Asn Ser Arg Phe Ala Trp Met
    450                 455                 460

Thr Arg Lys Ser Glu Glu Thr Ile Thr Pro Trp Asn Phe Glu Glu Val
465                 470                 475                 480

Val Asp Lys Gly Ala Ser Ala Gln Ser Phe Ile Glu Arg Met Thr Asn
                485                 490                 495

Phe Asp Lys Asn Leu Pro Asn Glu Lys Val Leu Pro Lys His Ser Leu
            500                 505                 510

Leu Tyr Glu Tyr Phe Thr Val Tyr Asn Glu Leu Thr Lys Val Lys Tyr
        515                 520                 525

Val Thr Glu Gly Met Arg Lys Pro Ala Phe Leu Ser Gly Glu Gln Lys
    530                 535                 540

Lys Ala Ile Val Asp Leu Leu Phe Lys Thr Asn Arg Lys Val Thr Val
545                 550                 555                 560

Lys Gln Leu Lys Glu Asp Tyr Phe Lys Lys Ile Glu Cys Phe Asp Ser
                565                 570                 575

Val Glu Ile Ser Gly Val Glu Asp Arg Phe Asn Ala Ser Leu Gly Thr
            580                 585                 590

Tyr His Asp Leu Leu Lys Ile Ile Lys Asp Lys Asp Phe Leu Asp Asn
        595                 600                 605

Glu Glu Asn Glu Asp Ile Leu Glu Asp Ile Val Leu Thr Leu Thr Leu
    610                 615                 620

Phe Glu Asp Arg Glu Met Ile Glu Glu Arg Leu Lys Thr Tyr Ala His
625                 630                 635                 640

Leu Phe Asp Asp Lys Val Met Lys Gln Leu Lys Arg Arg Arg Tyr Thr
                645                 650                 655

Gly Trp Gly Arg Leu Ser Arg Lys Leu Ile Asn Gly Ile Arg Asp Lys
            660                 665                 670

Gln Ser Gly Lys Thr Ile Leu Asp Phe Leu Lys Ser Asp Gly Phe Ala
        675                 680                 685

Asn Arg Asn Phe Met Gln Leu Ile His Asp Asp Ser Leu Thr Phe Lys
    690                 695                 700

Glu Asp Ile Gln Lys Ala Gln Val
705                 710

<210>  23
<211>  2136
<212>  DNA
<213>  Artificial

<220>
<223>  N-Cas9 encoding sequence

<400>  23
gacaagaagt actccattgg gctcgatatc ggcacaaaca gcgtcggctg ggccgtcatt      60

acggacgagt acaaggtgcc gagcaaaaaa ttcaaagttc tgggcaatac cgatcgccac     120

agcataaaga agaacctcat tggcgccctc ctgttcgact ccggggaaac ggccgaagcc     180

acgcggctca aaagaacagc acggcgcaga tatacccgca gaaagaatcg gatctgctac     240

ctgcaggaga tctttagtaa tgagatggct aaggtggatg actctttctt ccataggctg     300

gaggagtcct ttttggtgga ggaggataaa aagcacgagc gccacccaat ctttggcaat     360

atcgtggacg aggtggcgta ccatgaaaag tacccaacca tatatcatct gaggaagaag     420

cttgtagaca gtactgataa ggctgacttg cggttgatct atctcgcgct ggcgcatatg     480

atcaaatttc ggggacactt cctcatcgag ggggacctga acccagacaa cagcgatgtc     540

gacaaactct ttatccaact ggttcagact tacaatcagc ttttcgaaga gaacccgatc     600

aacgcatccg gagttgacgc caaagcaatc ctgagcgcta ggctgtccaa atcccggcgg     660

ctcgaaaacc tcatcgcaca gctccctggg gagaagaaga acggcctgtt tggtaatctt     720

atcgccctgt cactcgggct gacccccaac tttaaatcta acttcgacct ggccgaagat     780

gccaagcttc aactgagcaa agacacctac gatgatgatc tcgacaatct gctggcccag     840

atcggcgacc agtacgcaga cctttttttg gcggcaaaga acctgtcaga cgccattctg     900

ctgagtgata ttctgcgagt gaacacggag atcaccaaag ctccgctgag cgctagtatg     960

atcaagcgct atgatgagca ccaccaagac ttgactttgc tgaaggccct tgtcagacag    1020

caactgcctg agaagtacaa ggaaattttc ttcgatcagt ctaaaaatgg ctacgccgga    1080

tacattgacg gcggagcaag ccaggaggaa ttttacaaat ttattaagcc catcttggaa    1140

aaaatggacg gcaccgagga gctgctggta aagcttaaca gagaagatct gttgcgcaaa    1200

cagcgcactt tcgacaatgg aagcatcccc caccagattc acctgggcga actgcacgct    1260

atcctcaggc ggcaagagga tttctacccc tttttgaaag ataacaggga aaagattgag 1320

aaaatcctca catttcggat accctactat gtaggccccc tcgcccgggg aaattccaga 1380

ttcgcgtgga tgactcgcaa atcagaagag accatcactc cctggaactt cgaggaagtc 1440

gtggataagg gggcctctgc ccagtccttc atcgaaagga tgactaactt tgataaaaat 1500

ctgcctaacg aaaaggtgct tcctaaacac tctctgctgt acgagtactt cacagtttat 1560

aacgagctca ccaaggtcaa atacgtcaca gaagggatga gaaagccagc attcctgtct 1620

ggagagcaga agaaagctat cgtggacctc ctcttcaaga cgaaccggaa agttaccgtg 1680

aaacagctca aagaagacta tttcaaaaag attgaatgtt tcgactctgt tgaaatcagc 1740

ggagtggagg atcgcttcaa cgcatccctg ggaacgtatc acgatctcct gaaaatcatt 1800

aaagacaagg acttcctgga caatgaggag aacgaggaca ttcttgagga cattgtcctc 1860

acccttacgt tgtttgaaga tagggagatg attgaagaac gcttgaaaac ttacgctcat 1920

ctcttcgacg acaaagtcat gaaacagctc aagaggcgcc gatatacagg atgggggcgg 1980

ctgtcaagaa aactgatcaa tgggatccga gacaagcaga gtggaaagac aatcctggat 2040

tttcttaagt ccgatggatt tgccaaccgg aacttcatgc agttgatcca tgatgactct 2100

ctcaccttta aggaggacat ccagaaagca caagtt 2136

<210> 24
<211> 102
<212> PRT
<213> Artificial

<220>
<223> N-Intein

<400> 24

Cys Leu Ala Gly Asp Thr Leu Ile Thr Leu Ala Asp Gly Arg Arg Val
1               5                   10                  15

Pro Ile Arg Glu Leu Val Ser Gln Gln Asn Phe Ser Val Trp Ala Leu
            20                  25                  30

Asn Pro Gln Thr Tyr Arg Leu Glu Arg Ala Arg Val Ser Arg Ala Phe
        35                  40                  45

Cys Thr Gly Ile Lys Pro Val Tyr Arg Leu Thr Thr Arg Leu Gly Arg
    50                  55                  60

Ser Ile Arg Ala Thr Ala Asn His Arg Phe Leu Thr Pro Gln Gly Trp
65                  70                  75                  80

Lys Arg Val Asp Glu Leu Gln Pro Gly Asp Tyr Leu Ala Leu Pro Arg
                85                  90                  95

Arg Ile Pro Thr Ala Ser
            100

<210>  25
<211>  306
<212>  DNA
<213>  Artificial

<220>
<223>  N-Intein encoding

<400>  25
tgtctggctg gcgatactct cattaccctg gccgatggac gacgagtgcc tattagagaa      60

ctggtgtcac agcagaattt ttccgtgtgg gctctgaatc ctcagactta ccgcctggag     120

agggctagag tgagtagagc tttctgtacc ggcatcaaac ctgtgtaccg cctcaccact     180

agactgggga gatccattag ggccactgcc aaccaccgat ttctcacacc tcagggctgg     240

aaacgagtcg atgaactcca gcctggagat tacctggctc tgcctaggag aatccctact     300

gcctcc                                                                306

<210>  26
<211>  7
<212>  PRT
<213>  Artificial

<220>
<223>  SV40-NLS

<400>  26

Pro Lys Lys Lys Arg Lys Val
1               5

<210>  27
<211>  21
<212>  DNA
<213>  Artificial

<220>
<223>  SV40-NLS encoding

<400>  27
ccaaagaaga agcggaaggt c                                                21

<210>  28
<211>  831
<212>  PRT
<213>  Artificial

<220>
<223>  SV40NLS-SpCas9 N-fragment-Intein

<400>  28

Met Ala Pro Lys Lys Lys Arg Lys Val Gly Ile His Gly Val Pro Ala
1               5                   10                  15

Ala Asp Lys Lys Tyr Ser Ile Gly Leu Asp Ile Gly Thr Asn Ser Val
            20                  25                  30

Gly Trp Ala Val Ile Thr Asp Glu Tyr Lys Val Pro Ser Lys Lys Phe
        35                  40                  45

Lys Val Leu Gly Asn Thr Asp Arg His Ser Ile Lys Lys Asn Leu Ile
    50                  55                  60

Gly Ala Leu Leu Phe Asp Ser Gly Glu Thr Ala Glu Ala Thr Arg Leu
65                  70                  75                  80

Lys Arg Thr Ala Arg Arg Arg Tyr Thr Arg Arg Lys Asn Arg Ile Cys
                85                  90                  95

Tyr Leu Gln Glu Ile Phe Ser Asn Glu Met Ala Lys Val Asp Asp Ser
            100                 105                 110

Phe Phe His Arg Leu Glu Glu Ser Phe Leu Val Glu Glu Asp Lys Lys
        115                 120                 125

His Glu Arg His Pro Ile Phe Gly Asn Ile Val Asp Glu Val Ala Tyr
    130                 135                 140

His Glu Lys Tyr Pro Thr Ile Tyr His Leu Arg Lys Lys Leu Val Asp
145                 150                 155                 160

Ser Thr Asp Lys Ala Asp Leu Arg Leu Ile Tyr Leu Ala Leu Ala His
                165                 170                 175

Met Ile Lys Phe Arg Gly His Phe Leu Ile Glu Gly Asp Leu Asn Pro
            180                 185                 190

Asp Asn Ser Asp Val Asp Lys Leu Phe Ile Gln Leu Val Gln Thr Tyr
        195                 200                 205

Asn Gln Leu Phe Glu Glu Asn Pro Ile Asn Ala Ser Gly Val Asp Ala
    210                 215                 220

Lys Ala Ile Leu Ser Ala Arg Leu Ser Lys Ser Arg Arg Leu Glu Asn
225                 230                 235                 240

Leu Ile Ala Gln Leu Pro Gly Glu Lys Lys Asn Gly Leu Phe Gly Asn
                245                 250                 255

Leu Ile Ala Leu Ser Leu Gly Leu Thr Pro Asn Phe Lys Ser Asn Phe
            260                 265                 270

Asp Leu Ala Glu Asp Ala Lys Leu Gln Leu Ser Lys Asp Thr Tyr Asp
        275                 280                 285

Asp Asp Leu Asp Asn Leu Leu Ala Gln Ile Gly Asp Gln Tyr Ala Asp
    290                 295                 300

Leu Phe Leu Ala Ala Lys Asn Leu Ser Asp Ala Ile Leu Leu Ser Asp
305                 310                 315                 320

Ile Leu Arg Val Asn Thr Glu Ile Thr Lys Ala Pro Leu Ser Ala Ser
                325                 330                 335

Met Ile Lys Arg Tyr Asp Glu His His Gln Asp Leu Thr Leu Leu Lys
            340                 345                 350

Ala Leu Val Arg Gln Gln Leu Pro Glu Lys Tyr Lys Glu Ile Phe Phe
        355                 360                 365

Asp Gln Ser Lys Asn Gly Tyr Ala Gly Tyr Ile Asp Gly Gly Ala Ser
    370                 375                 380

Gln Glu Glu Phe Tyr Lys Phe Ile Lys Pro Ile Leu Glu Lys Met Asp
385                 390                 395                 400

Gly Thr Glu Glu Leu Leu Val Lys Leu Asn Arg Glu Asp Leu Leu Arg
                405                 410                 415

Lys Gln Arg Thr Phe Asp Asn Gly Ser Ile Pro His Gln Ile His Leu
            420                 425                 430

Gly Glu Leu His Ala Ile Leu Arg Arg Gln Glu Asp Phe Tyr Pro Phe
        435                 440                 445

Leu Lys Asp Asn Arg Glu Lys Ile Glu Lys Ile Leu Thr Phe Arg Ile
    450                 455                 460

Pro Tyr Tyr Val Gly Pro Leu Ala Arg Gly Asn Ser Arg Phe Ala Trp
465                 470                 475                 480

Met Thr Arg Lys Ser Glu Glu Thr Ile Thr Pro Trp Asn Phe Glu Glu
                485                 490                 495

Val Val Asp Lys Gly Ala Ser Ala Gln Ser Phe Ile Glu Arg Met Thr

500                 505                 510

Asn Phe Asp Lys Asn Leu Pro Asn Glu Lys Val Leu Pro Lys His Ser
        515                 520                 525

Leu Leu Tyr Glu Tyr Phe Thr Val Tyr Asn Glu Leu Thr Lys Val Lys
    530                 535                 540

Tyr Val Thr Glu Gly Met Arg Lys Pro Ala Phe Leu Ser Gly Glu Gln
545                 550                 555                 560

Lys Lys Ala Ile Val Asp Leu Leu Phe Lys Thr Asn Arg Lys Val Thr
                565                 570                 575

Val Lys Gln Leu Lys Glu Asp Tyr Phe Lys Lys Ile Glu Cys Phe Asp
            580                 585                 590

Ser Val Glu Ile Ser Gly Val Glu Asp Arg Phe Asn Ala Ser Leu Gly
        595                 600                 605

Thr Tyr His Asp Leu Leu Lys Ile Ile Lys Asp Lys Asp Phe Leu Asp
    610                 615                 620

Asn Glu Glu Asn Glu Asp Ile Leu Glu Asp Ile Val Leu Thr Leu Thr
625                 630                 635                 640

Leu Phe Glu Asp Arg Glu Met Ile Glu Glu Arg Leu Lys Thr Tyr Ala
                645                 650                 655

His Leu Phe Asp Asp Lys Val Met Lys Gln Leu Lys Arg Arg Arg Tyr
            660                 665                 670

Thr Gly Trp Gly Arg Leu Ser Arg Lys Leu Ile Asn Gly Ile Arg Asp
        675                 680                 685

Lys Gln Ser Gly Lys Thr Ile Leu Asp Phe Leu Lys Ser Asp Gly Phe
    690                 695                 700

Ala Asn Arg Asn Phe Met Gln Leu Ile His Asp Asp Ser Leu Thr Phe
705                 710                 715                 720

Lys Glu Asp Ile Gln Lys Ala Gln Val Cys Leu Ala Gly Asp Thr Leu
                725                 730                 735

Ile Thr Leu Ala Asp Gly Arg Arg Val Pro Ile Arg Glu Leu Val Ser
            740                 745                 750

Gln Gln Asn Phe Ser Val Trp Ala Leu Asn Pro Gln Thr Tyr Arg Leu
      755                 760                 765

Glu Arg Ala Arg Val Ser Arg Ala Phe Cys Thr Gly Ile Lys Pro Val
    770                 775                 780

Tyr Arg Leu Thr Thr Arg Leu Gly Arg Ser Ile Arg Ala Thr Ala Asn
785                 790                 795                 800

His Arg Phe Leu Thr Pro Gln Gly Trp Lys Arg Val Asp Glu Leu Gln
                805                 810                 815

Pro Gly Asp Tyr Leu Ala Leu Pro Arg Arg Ile Pro Thr Ala Ser
            820                 825                 830

<210>  29
<211>  2493
<212>  DNA
<213>  Artificial

<220>
<223>  SV40NLS-SpCas9 N-fragment-Intein encoding

<400>  29
atggccccaa agaagaagcg gaaggtcggt atccacggag tcccagcagc cgacaagaag      60

tactccattg ggctcgatat cggcacaaac agcgtcggct gggccgtcat tacggacgag     120

tacaaggtgc cgagcaaaaa attcaaagtt ctgggcaata ccgatcgcca cagcataaag     180

aagaacctca ttggcgccct cctgttcgac tccggggaaa cggccgaagc cacgcggctc     240

aaaagaacag cacggcgcag atatacccgc agaaagaatc ggatctgcta cctgcaggag     300

atctttagta atgagatggc taaggtggat gactctttct tccataggct ggaggagtcc     360

tttttggtgg aggaggataa aaagcacgag cgccacccaa tctttggcaa tatcgtggac     420

gaggtggcgt accatgaaaa gtacccaacc atatatcatc tgaggaagaa gcttgtagac     480

agtactgata aggctgactt gcggttgatc tatctcgcgc tggcgcatat gatcaaattt     540

cggggacact tcctcatcga gggggacctg aacccagaca acagcgatgt cgacaaactc     600

tttatccaac tggttcagac ttacaatcag cttttcgaag agaacccgat caacgcatcc     660

ggagttgacg ccaaagcaat cctgagcgct aggctgtcca aatcccggcg gctcgaaaac     720

ctcatcgcac agctccctgg ggagaagaag aacggcctgt ttggtaatct tatcgccctg     780

tcactcgggc tgacccccaa ctttaaatct aacttcgacc tggccgaaga tgccaagctt     840

caactgagca aagacaccta cgatgatgat ctcgacaatc tgctggccca gatcggcgac     900

cagtacgcag accttttttt ggcggcaaag aacctgtcag acgccattct gctgagtgat     960

attctgcgag tgaacacgga gatcaccaaa gctccgctga gcgctagtat gatcaagcgc    1020

tatgatgagc accaccaaga cttgactttg ctgaaggccc ttgtcagaca gcaactgcct 1080

gagaagtaca aggaaatttt cttcgatcag tctaaaaatg gctacgccgg atacattgac 1140

ggcggagcaa gccaggagga attttacaaa tttattaagc ccatcttgga aaaaatggac 1200

ggcaccgagg agctgctggt aaagcttaac agagaagatc tgttgcgcaa acagcgcact 1260

ttcgacaatg gaagcatccc ccaccagatt cacctgggcg aactgcacgc tatcctcagg 1320

cggcaagagg atttctaccc ctttttgaaa gataacaggg aaaagattga gaaaatcctc 1380

acatttcgga taccctacta tgtaggcccc ctcgcccggg gaaattccag attcgcgtgg 1440

atgactcgca aatcagaaga gaccatcact ccctggaact tcgaggaagt cgtggataag 1500

ggggcctctg cccagtcctt catcgaaagg atgactaact ttgataaaaa tctgcctaac 1560

gaaaaggtgc ttcctaaaca ctctctgctg tacgagtact tcacagttta taacgagctc 1620

accaaggtca aatacgtcac agaagggatg agaaagccag cattcctgtc tggagagcag 1680

aagaaagcta tcgtggacct cctcttcaag acgaaccgga aagttaccgt gaaacagctc 1740

aaagaagact atttcaaaaa gattgaatgt ttcgactctg ttgaaatcag cggagtggag 1800

gatcgcttca acgcatccct gggaacgtat cacgatctcc tgaaaatcat taaagacaag 1860

gacttcctgg acaatgagga gaacgaggac attcttgagg acattgtcct cacccttacg 1920

ttgtttgaag atagggagat gattgaagaa cgcttgaaaa cttacgctca tctcttcgac 1980

gacaaagtca tgaaacagct caagaggcgc cgatatacag gatgggggcg gctgtcaaga 2040

aaactgatca atgggatccg agacaagcag agtggaaaga caatcctgga ttttcttaag 2100

tccgatggat ttgccaaccg gaacttcatg cagttgatcc atgatgactc tctcaccttt 2160

aaggaggaca tccagaaagc acaagtttgt ctggctggcg atactctcat taccctggcc 2220

gatggacgac gagtgcctat tagagaactg gtgtcacagc agaatttttc cgtgtgggct 2280

ctgaatcctc agacttaccg cctggagagg gctagagtga gtagagcttt ctgtaccggc 2340

atcaaacctg tgtaccgcct caccactaga ctggggagat ccattagggc cactgccaac 2400

caccgatttc tcacacctca gggctggaaa cgagtcgatg aactccagcc tggagattac 2460

ctggctctgc ctaggagaat ccctactgcc tcc 2493

<210> 30
<211> 655
<212> PRT
<213> Artificial

<220>
<223> C-Cas9

<400> 30

Ser Gly Gln Gly Asp Ser Leu His Glu His Ile Ala Asn Leu Ala Gly
1               5                   10                  15

Ser Pro Ala Ile Lys Lys Gly Ile Leu Gln Thr Val Lys Val Val Asp
            20                  25                  30

Glu Leu Val Lys Val Met Gly Arg His Lys Pro Glu Asn Ile Val Ile
        35                  40                  45

Glu Met Ala Arg Glu Asn Gln Thr Thr Gln Lys Gly Gln Lys Asn Ser
    50                  55                  60

Arg Glu Arg Met Lys Arg Ile Glu Glu Gly Ile Lys Glu Leu Gly Ser
65                  70                  75                  80

Gln Ile Leu Lys Glu His Pro Val Glu Asn Thr Gln Leu Gln Asn Glu
                85                  90                  95

Lys Leu Tyr Leu Tyr Tyr Leu Gln Asn Gly Arg Asp Met Tyr Val Asp
            100                 105                 110

Gln Glu Leu Asp Ile Asn Arg Leu Ser Asp Tyr Asp Val Asp His Ile
        115                 120                 125

Val Pro Gln Ser Phe Leu Lys Asp Asp Ser Ile Asp Asn Lys Val Leu
    130                 135                 140

Thr Arg Ser Asp Lys Asn Arg Gly Lys Ser Asp Asn Val Pro Ser Glu
145                 150                 155                 160

Glu Val Val Lys Lys Met Lys Asn Tyr Trp Arg Gln Leu Leu Asn Ala
                165                 170                 175

Lys Leu Ile Thr Gln Arg Lys Phe Asp Asn Leu Thr Lys Ala Glu Arg
            180                 185                 190

Gly Gly Leu Ser Glu Leu Asp Lys Ala Gly Phe Ile Lys Arg Gln Leu
        195                 200                 205

Val Glu Thr Arg Gln Ile Thr Lys His Val Ala Gln Ile Leu Asp Ser
    210                 215                 220

Arg Met Asn Thr Lys Tyr Asp Glu Asn Asp Lys Leu Ile Arg Glu Val
225                 230                 235                 240

Lys Val Ile Thr Leu Lys Ser Lys Leu Val Ser Asp Phe Arg Lys Asp
                245                 250                 255

Phe Gln Phe Tyr Lys Val Arg Glu Ile Asn Asn Tyr His His Ala His

            260                 265                 270

Asp Ala Tyr Leu Asn Ala Val Val Gly Thr Ala Leu Ile Lys Lys Tyr
        275                 280                 285

Pro Lys Leu Glu Ser Glu Phe Val Tyr Gly Asp Tyr Lys Val Tyr Asp
    290                 295                 300

Val Arg Lys Met Ile Ala Lys Ser Glu Gln Glu Ile Gly Lys Ala Thr
305                 310                 315                 320

Ala Lys Tyr Phe Phe Tyr Ser Asn Ile Met Asn Phe Phe Lys Thr Glu
                325                 330                 335

Ile Thr Leu Ala Asn Gly Glu Ile Arg Lys Arg Pro Leu Ile Glu Thr
            340                 345                 350

Asn Gly Glu Thr Gly Glu Ile Val Trp Asp Lys Gly Arg Asp Phe Ala
        355                 360                 365

Thr Val Arg Lys Val Leu Ser Met Pro Gln Val Asn Ile Val Lys Lys
    370                 375                 380

Thr Glu Val Gln Thr Gly Gly Phe Ser Lys Glu Ser Ile Leu Pro Lys
385                 390                 395                 400

Arg Asn Ser Asp Lys Leu Ile Ala Arg Lys Lys Asp Trp Asp Pro Lys
                405                 410                 415

Lys Tyr Gly Gly Phe Asp Ser Pro Thr Val Ala Tyr Ser Val Leu Val
            420                 425                 430

Val Ala Lys Val Glu Lys Gly Lys Ser Lys Lys Leu Lys Ser Val Lys
        435                 440                 445

Glu Leu Leu Gly Ile Thr Ile Met Glu Arg Ser Ser Phe Glu Lys Asn
    450                 455                 460

Pro Ile Asp Phe Leu Glu Ala Lys Gly Tyr Lys Glu Val Lys Lys Asp
465                 470                 475                 480

Leu Ile Ile Lys Leu Pro Lys Tyr Ser Leu Phe Glu Leu Glu Asn Gly
                485                 490                 495

Arg Lys Arg Met Leu Ala Ser Ala Gly Glu Leu Gln Lys Gly Asn Glu
            500                 505                 510

Leu Ala Leu Pro Ser Lys Tyr Val Asn Phe Leu Tyr Leu Ala Ser His
        515                 520                 525

Tyr Glu Lys Leu Lys Gly Ser Pro Glu Asp Asn Glu Gln Lys Gln Leu
    530                 535                 540

Phe Val Glu Gln His Lys His Tyr Leu Asp Glu Ile Ile Glu Gln Ile
545                 550                 555                 560

Ser Glu Phe Ser Lys Arg Val Ile Leu Ala Asp Ala Asn Leu Asp Lys
                565                 570                 575

Val Leu Ser Ala Tyr Asn Lys His Arg Asp Lys Pro Ile Arg Glu Gln
            580                 585                 590

Ala Glu Asn Ile Ile His Leu Phe Thr Leu Thr Asn Leu Gly Ala Pro
        595                 600                 605

Ala Ala Phe Lys Tyr Phe Asp Thr Thr Ile Asp Arg Lys Arg Tyr Thr
    610                 615                 620

Ser Thr Lys Glu Val Leu Asp Ala Thr Leu Ile His Gln Ser Ile Thr
625                 630                 635                 640

Gly Leu Tyr Glu Thr Arg Ile Asp Leu Ser Gln Leu Gly Gly Asp
                645                 650                 655

<210>  31
<211>  1965
<212>  DNA
<213>  Artificial

<220>
<223>  C-Cas9 encoding

<400>  31
tctggccagg gggacagtct tcacgagcac atcgctaatc ttgcaggtag cccagctatc      60

aaaaagggaa tactgcagac cgttaaggtc gtggatgaac tcgtcaaagt aatgggaagg     120

cataagcccg agaatatcgt tatcgagatg gcccgagaga accaaactac ccagaaggga     180

cagaagaaca gtagggaaag gatgaagagg attgaagagg gtataaaaga actggggtcc     240

caaatcctta aggaacaccc agttgaaaac acccagcttc agaatgagaa gctctacctg     300

tactacctgc agaacggcag ggacatgtac gtggatcagg aactggacat caatcggctc     360

tccgactacg acgtggatca tatcgtgccc cagtcttttc tcaaagatga ttctattgat     420

aataaagtgt tgacaagatc cgataaaaat agagggaaga gtgataacgt cccctcagaa     480

gaagttgtca agaaaatgaa aaattattgg cggcagctgc tgaacgccaa actgatcaca     540

caacggaagt tcgataatct gactaaggct gaacgaggtg gcctgtctga gttggataaa 600

gccggcttca tcaaaaggca gcttgttgag acacgccaga tcaccaagca cgtggcccaa 660

attctcgatt cacgcatgaa caccaagtac gatgaaaatg acaaactgat tcgagaggtg 720

aaagttatta ctctgaagtc taagctggtc tcagatttca gaaaggactt tcagttttat 780

aaggtgagag agatcaacaa ttaccaccat gcgcatgatg cctacctgaa tgcagtggta 840

ggcactgcac ttatcaaaaa atatcccaag cttgaatctg aatttgttta cggagactat 900

aaagtgtacg atgttaggaa aatgatcgca aagtctgagc aggaaatagg caaggccacc 960

gctaagtact tcttttacag caatattatg aattttttca agaccgagat tacactggcc 1020

aatggagaga ttcggaagcg accacttatc gaaacaaacg gagaaacagg agaaatcgtg 1080

tgggacaagg gtagggattt cgcgacagtc cggaaggtcc tgtccatgcc gcaggtgaac 1140

atcgttaaaa agaccgaagt acagaccgga ggcttctcca aggaaagtat cctcccgaaa 1200

aggaacagcg acaagctgat cgcacgcaaa aaagattggg accccaagaa atacggcgga 1260

ttcgattctc ctacagtcgc ttacagtgta ctggttgtgg ccaaagtgga gaaagggaag 1320

tctaaaaaac tcaaaagcgt caaggaactg ctgggcatca caatcatgga gcgatcaagc 1380

ttcgaaaaaa accccatcga ctttctcgag gcgaaaggat ataaagaggt caaaaaagac 1440

ctcatcatta agcttcccaa gtactctctc tttgagcttg aaaacggccg gaaacgaatg 1500

ctcgctagtg cgggcgagct gcagaaaggt aacgagctgg cactgccctc taaatacgtt 1560

aatttcttgt atctggccag ccactatgaa aagctcaaag ggtctcccga agataatgag 1620

cagaagcagc tgttcgtgga acaacacaaa cactaccttg atgagatcat cgagcaaata 1680

agcgaattct ccaaaagagt gatcctcgcc gacgctaacc tcgataaggt gctttctgct 1740

tacaataagc acagggataa gcccatcagg gagcaggcag aaaacattat ccacttgttt 1800

actctgacca acttgggcgc gcctgcagcc ttcaagtact tcgacaccac catagacaga 1860

aagcggtaca cctctacaaa ggaggtcctg gacgccacac tgattcatca gtcaattacg 1920

gggctctatg aaacaagaat cgacctctct cagctcggtg gagac 1965

<210> 32
<211> 51
<212> PRT
<213> Artificial

<220>
<223> C-Intein

<400> 32

Ala Ala Ala Cys Pro Glu Leu Arg Gln Leu Ala Gln Ser Asp Val Tyr
1               5                   10                  15

Trp Asp Pro Ile Val Ser Ile Glu Pro Asp Gly Val Glu Glu Val Phe
            20                  25                  30

Asp Leu Thr Val Pro Gly Pro His Asn Phe Val Ala Asn Asp Ile Ile
        35                  40                  45

Ala His Asn
    50

<210>  33
<211>  153
<212>  DNA
<213>  Artificial

<220>
<223>  C-Intein encoding

<400>  33
gcggcggcgt gcccggaact gcgtcagctg gcgcagagcg atgtgtattg ggatccgatt      60

gtgagcattg aaccggatgg cgtggaagaa gtgtttgatc tgaccgtgcc gggcccgcat     120

aactttgtgg cgaacgatat tattgcgcat aac                                  153

<210>  34
<211>  722
<212>  PRT
<213>  Artificial

<220>
<223>  Intein-SpCas9 C-fragment-SV40-NLS

<400>  34

Met Ala Ala Ala Cys Pro Glu Leu Arg Gln Leu Ala Gln Ser Asp Val
1               5                   10                  15

Tyr Trp Asp Pro Ile Val Ser Ile Glu Pro Asp Gly Val Glu Glu Val
            20                  25                  30

Phe Asp Leu Thr Val Pro Gly Pro His Asn Phe Val Ala Asn Asp Ile
        35                  40                  45

Ile Ala His Asn Ser Gly Gln Gly Asp Ser Leu His Glu His Ile Ala
    50                  55                  60

Asn Leu Ala Gly Ser Pro Ala Ile Lys Lys Gly Ile Leu Gln Thr Val
65                  70                  75                  80

Lys Val Val Asp Glu Leu Val Lys Val Met Gly Arg His Lys Pro Glu
                85                  90                  95

Asn Ile Val Ile Glu Met Ala Arg Glu Asn Gln Thr Thr Gln Lys Gly

100                 105                 110

Gln Lys Asn Ser Arg Glu Arg Met Lys Arg Ile Glu Glu Gly Ile Lys
        115                 120                 125

Glu Leu Gly Ser Gln Ile Leu Lys Glu His Pro Val Glu Asn Thr Gln
    130                 135                 140

Leu Gln Asn Glu Lys Leu Tyr Leu Tyr Tyr Leu Gln Asn Gly Arg Asp
145                 150                 155                 160

Met Tyr Val Asp Gln Glu Leu Asp Ile Asn Arg Leu Ser Asp Tyr Asp
                165                 170                 175

Val Asp His Ile Val Pro Gln Ser Phe Leu Lys Asp Asp Ser Ile Asp
            180                 185                 190

Asn Lys Val Leu Thr Arg Ser Asp Lys Asn Arg Gly Lys Ser Asp Asn
        195                 200                 205

Val Pro Ser Glu Glu Val Val Lys Lys Met Lys Asn Tyr Trp Arg Gln
    210                 215                 220

Leu Leu Asn Ala Lys Leu Ile Thr Gln Arg Lys Phe Asp Asn Leu Thr
225                 230                 235                 240

Lys Ala Glu Arg Gly Gly Leu Ser Glu Leu Asp Lys Ala Gly Phe Ile
                245                 250                 255

Lys Arg Gln Leu Val Glu Thr Arg Gln Ile Thr Lys His Val Ala Gln
            260                 265                 270

Ile Leu Asp Ser Arg Met Asn Thr Lys Tyr Asp Glu Asn Asp Lys Leu
        275                 280                 285

Ile Arg Glu Val Lys Val Ile Thr Leu Lys Ser Lys Leu Val Ser Asp
    290                 295                 300

Phe Arg Lys Asp Phe Gln Phe Tyr Lys Val Arg Glu Ile Asn Asn Tyr
305                 310                 315                 320

His His Ala His Asp Ala Tyr Leu Asn Ala Val Val Gly Thr Ala Leu
                325                 330                 335

Ile Lys Lys Tyr Pro Lys Leu Glu Ser Glu Phe Val Tyr Gly Asp Tyr
            340                 345                 350

Lys Val Tyr Asp Val Arg Lys Met Ile Ala Lys Ser Glu Gln Glu Ile
        355                 360                 365

Gly Lys Ala Thr Ala Lys Tyr Phe Phe Tyr Ser Asn Ile Met Asn Phe
    370                 375                 380

Phe Lys Thr Glu Ile Thr Leu Ala Asn Gly Glu Ile Arg Lys Arg Pro
385                 390                 395                 400

Leu Ile Glu Thr Asn Gly Glu Thr Gly Glu Ile Val Trp Asp Lys Gly
                405                 410                 415

Arg Asp Phe Ala Thr Val Arg Lys Val Leu Ser Met Pro Gln Val Asn
            420                 425                 430

Ile Val Lys Lys Thr Glu Val Gln Thr Gly Gly Phe Ser Lys Glu Ser
        435                 440                 445

Ile Leu Pro Lys Arg Asn Ser Asp Lys Leu Ile Ala Arg Lys Lys Asp
    450                 455                 460

Trp Asp Pro Lys Lys Tyr Gly Gly Phe Asp Ser Pro Thr Val Ala Tyr
465                 470                 475                 480

Ser Val Leu Val Val Ala Lys Val Glu Lys Gly Lys Ser Lys Lys Leu
                485                 490                 495

Lys Ser Val Lys Glu Leu Leu Gly Ile Thr Ile Met Glu Arg Ser Ser
            500                 505                 510

Phe Glu Lys Asn Pro Ile Asp Phe Leu Glu Ala Lys Gly Tyr Lys Glu
        515                 520                 525

Val Lys Lys Asp Leu Ile Ile Lys Leu Pro Lys Tyr Ser Leu Phe Glu
    530                 535                 540

Leu Glu Asn Gly Arg Lys Arg Met Leu Ala Ser Ala Gly Glu Leu Gln
545                 550                 555                 560

Lys Gly Asn Glu Leu Ala Leu Pro Ser Lys Tyr Val Asn Phe Leu Tyr
                565                 570                 575

Leu Ala Ser His Tyr Glu Lys Leu Lys Gly Ser Pro Glu Asp Asn Glu
            580                 585                 590

Gln Lys Gln Leu Phe Val Glu Gln His Lys His Tyr Leu Asp Glu Ile
        595                 600                 605

Ile Glu Gln Ile Ser Glu Phe Ser Lys Arg Val Ile Leu Ala Asp Ala
    610                 615                 620

Asn Leu Asp Lys Val Leu Ser Ala Tyr Asn Lys His Arg Asp Lys Pro
625                 630                 635                 640

Ile Arg Glu Gln Ala Glu Asn Ile Ile His Leu Phe Thr Leu Thr Asn
                645                 650                 655

Leu Gly Ala Pro Ala Ala Phe Lys Tyr Phe Asp Thr Thr Ile Asp Arg
            660                 665                 670

Lys Arg Tyr Thr Ser Thr Lys Glu Val Leu Asp Ala Thr Leu Ile His
        675                 680                 685

Gln Ser Ile Thr Gly Leu Tyr Glu Thr Arg Ile Asp Leu Ser Gln Leu
    690                 695                 700

Gly Gly Asp Ser Arg Ala Asp Pro Lys Lys Lys Arg Lys Val Ser Arg
705                 710                 715                 720

Ala Arg

<210>  35
<211>  2166
<212>  DNA
<213>  Artificial

<220>
<223>  Intein-SpCas9 C-fragment-SV40-NLS encoding

<400>  35
atggcggcgg cgtgcccgga actgcgtcag ctggcgcaga gcgatgtgta ttgggatccg      60

attgtgagca ttgaaccgga tggcgtggaa gaagtgtttg atctgaccgt gccgggcccg     120

cataactttg tggcgaacga tattattgcg cataactctg gccaggggga cagtcttcac     180

gagcacatcg ctaatcttgc aggtagccca gctatcaaaa agggaatact gcagaccgtt     240

aaggtcgtgg atgaactcgt caaagtaatg ggaaggcata agcccgagaa tatcgttatc     300

gagatggccc gagagaacca aactacccag aagggacaga agaacagtag ggaaaggatg     360

aagaggattg aagagggtat aaaagaactg gggtcccaaa tccttaagga acacccagtt     420

gaaaacaccc agcttcagaa tgagaagctc tacctgtact acctgcagaa cggcagggac     480

atgtacgtgg atcaggaact ggacatcaat cggctctccg actacgacgt ggatcatatc     540

gtgccccagt cttttctcaa agatgattct attgataata aagtgttgac aagatccgat     600

aaaaatagag ggaagagtga taacgtcccc tcagaagaag ttgtcaagaa aatgaaaaat     660

tattggcggc agctgctgaa cgccaaactg atcacacaac ggaagttcga taatctgact 720

aaggctgaac gaggtggcct gtctgagttg gataaagccg gcttcatcaa aaggcagctt 780

gttgagacac gccagatcac caagcacgtg gcccaaattc tcgattcacg catgaacacc 840

aagtacgatg aaaatgacaa actgattcga gaggtgaaag ttattactct gaagtctaag 900

ctggtctcag atttcagaaa ggactttcag ttttataagg tgagagagat caacaattac 960

caccatgcgc atgatgccta cctgaatgca gtggtaggca ctgcacttat caaaaaatat 1020

cccaagcttg aatctgaatt tgtttacgga gactataaag tgtacgatgt taggaaaatg 1080

atcgcaaagt ctgagcagga aataggcaag gccaccgcta agtacttctt ttacagcaat 1140

attatgaatt ttttcaagac cgagattaca ctggccaatg gagagattcg gaagcgacca 1200

cttatcgaaa caaacggaga aacaggagaa atcgtgtggg acaagggtag ggatttcgcg 1260

acagtccgga aggtcctgtc catgccgcag gtgaacatcg ttaaaaagac cgaagtacag 1320

accggaggct tctccaagga aagtatcctc ccgaaaagga acagcgacaa gctgatcgca 1380

cgcaaaaaag attgggaccc caagaaatac ggcggattcg attctcctac agtcgcttac 1440

agtgtactgg ttgtggccaa agtggagaaa gggaagtcta aaaaactcaa aagcgtcaag 1500

gaactgctgg gcatcacaat catggagcga tcaagcttcg aaaaaaaccc catcgacttt 1560

ctcgaggcga aaggatataa agaggtcaaa aaagacctca tcattaagct tcccaagtac 1620

tctctctttg agcttgaaaa cggccggaaa cgaatgctcg ctagtgcggg cgagctgcag 1680

aaaggtaacg agctggcact gccctctaaa tacgttaatt tcttgtatct ggccagccac 1740

tatgaaaagc tcaaagggtc tcccgaagat aatgagcaga agcagctgtt cgtggaacaa 1800

cacaaacact accttgatga gatcatcgag caaataagcg aattctccaa aagagtgatc 1860

ctcgccgacg ctaacctcga taaggtgctt tctgcttaca ataagcacag ggataagccc 1920

atcagggagc aggcagaaaa cattatccac ttgtttactc tgaccaactt gggcgcgcct 1980

gcagccttca agtacttcga caccaccata gacagaaagc ggtacacctc tacaaaggag 2040

gtcctggacg ccacactgat tcatcagtca attacggggc tctatgaaac aagaatcgac 2100

ctctctcagc tcggtggaga cagcagggct gaccccaaga agaagaggaa ggtgtctcga 2160

gctcga 2166

<210> 36
<211> 1367
<212> PRT
<213> Artificial

<220>
<223> Cas9

<400> 36

Asp Lys Lys Tyr Ser Ile Gly Leu Asp Ile Gly Thr Asn Ser Val Gly
1               5                   10                  15

Trp Ala Val Ile Thr Asp Glu Tyr Lys Val Pro Ser Lys Lys Phe Lys
            20                  25                  30

Val Leu Gly Asn Thr Asp Arg His Ser Ile Lys Lys Asn Leu Ile Gly
        35                  40                  45

Ala Leu Leu Phe Asp Ser Gly Glu Thr Ala Glu Ala Thr Arg Leu Lys
    50                  55                  60

Arg Thr Ala Arg Arg Arg Tyr Thr Arg Arg Lys Asn Arg Ile Cys Tyr
65                  70                  75                  80

Leu Gln Glu Ile Phe Ser Asn Glu Met Ala Lys Val Asp Asp Ser Phe
                85                  90                  95

Phe His Arg Leu Glu Glu Ser Phe Leu Val Glu Glu Asp Lys Lys His
            100                 105                 110

Glu Arg His Pro Ile Phe Gly Asn Ile Val Asp Glu Val Ala Tyr His
        115                 120                 125

Glu Lys Tyr Pro Thr Ile Tyr His Leu Arg Lys Lys Leu Val Asp Ser
    130                 135                 140

Thr Asp Lys Ala Asp Leu Arg Leu Ile Tyr Leu Ala Leu Ala His Met
145                 150                 155                 160

Ile Lys Phe Arg Gly His Phe Leu Ile Glu Gly Asp Leu Asn Pro Asp
                165                 170                 175

Asn Ser Asp Val Asp Lys Leu Phe Ile Gln Leu Val Gln Thr Tyr Asn
            180                 185                 190

Gln Leu Phe Glu Glu Asn Pro Ile Asn Ala Ser Gly Val Asp Ala Lys
        195                 200                 205

Ala Ile Leu Ser Ala Arg Leu Ser Lys Ser Arg Arg Leu Glu Asn Leu
    210                 215                 220

Ile Ala Gln Leu Pro Gly Glu Lys Lys Asn Gly Leu Phe Gly Asn Leu
225                 230                 235                 240

Ile Ala Leu Ser Leu Gly Leu Thr Pro Asn Phe Lys Ser Asn Phe Asp
                245                 250                 255

Leu Ala Glu Asp Ala Lys Leu Gln Leu Ser Lys Asp Thr Tyr Asp Asp
            260                 265                 270

Asp Leu Asp Asn Leu Leu Ala Gln Ile Gly Asp Gln Tyr Ala Asp Leu
        275                 280                 285

Phe Leu Ala Ala Lys Asn Leu Ser Asp Ala Ile Leu Leu Ser Asp Ile
    290                 295                 300

Leu Arg Val Asn Thr Glu Ile Thr Lys Ala Pro Leu Ser Ala Ser Met
305                 310                 315                 320

Ile Lys Arg Tyr Asp Glu His His Gln Asp Leu Thr Leu Leu Lys Ala
                325                 330                 335

Leu Val Arg Gln Gln Leu Pro Glu Lys Tyr Lys Glu Ile Phe Phe Asp
            340                 345                 350

Gln Ser Lys Asn Gly Tyr Ala Gly Tyr Ile Asp Gly Gly Ala Ser Gln
        355                 360                 365

Glu Glu Phe Tyr Lys Phe Ile Lys Pro Ile Leu Glu Lys Met Asp Gly
    370                 375                 380

Thr Glu Glu Leu Leu Val Lys Leu Asn Arg Glu Asp Leu Leu Arg Lys
385                 390                 395                 400

Gln Arg Thr Phe Asp Asn Gly Ser Ile Pro His Gln Ile His Leu Gly
                405                 410                 415

Glu Leu His Ala Ile Leu Arg Arg Gln Glu Asp Phe Tyr Pro Phe Leu
            420                 425                 430

Lys Asp Asn Arg Glu Lys Ile Glu Lys Ile Leu Thr Phe Arg Ile Pro
        435                 440                 445

Tyr Tyr Val Gly Pro Leu Ala Arg Gly Asn Ser Arg Phe Ala Trp Met
    450                 455                 460

Thr Arg Lys Ser Glu Glu Thr Ile Thr Pro Trp Asn Phe Glu Glu Val
465                 470                 475                 480

Val Asp Lys Gly Ala Ser Ala Gln Ser Phe Ile Glu Arg Met Thr Asn
                485                 490                 495

Phe Asp Lys Asn Leu Pro Asn Glu Lys Val Leu Pro Lys His Ser Leu

            500                 505                 510

Leu Tyr Glu Tyr Phe Thr Val Tyr Asn Glu Leu Thr Lys Val Lys Tyr
        515                 520                 525

Val Thr Glu Gly Met Arg Lys Pro Ala Phe Leu Ser Gly Glu Gln Lys
    530                 535                 540

Lys Ala Ile Val Asp Leu Leu Phe Lys Thr Asn Arg Lys Val Thr Val
545                 550                 555                 560

Lys Gln Leu Lys Glu Asp Tyr Phe Lys Lys Ile Glu Cys Phe Asp Ser
                565                 570                 575

Val Glu Ile Ser Gly Val Glu Asp Arg Phe Asn Ala Ser Leu Gly Thr
            580                 585                 590

Tyr His Asp Leu Leu Lys Ile Ile Lys Asp Lys Asp Phe Leu Asp Asn
        595                 600                 605

Glu Glu Asn Glu Asp Ile Leu Glu Asp Ile Val Leu Thr Leu Thr Leu
    610                 615                 620

Phe Glu Asp Arg Glu Met Ile Glu Glu Arg Leu Lys Thr Tyr Ala His
625                 630                 635                 640

Leu Phe Asp Asp Lys Val Met Lys Gln Leu Lys Arg Arg Arg Tyr Thr
                645                 650                 655

Gly Trp Gly Arg Leu Ser Arg Lys Leu Ile Asn Gly Ile Arg Asp Lys
            660                 665                 670

Gln Ser Gly Lys Thr Ile Leu Asp Phe Leu Lys Ser Asp Gly Phe Ala
        675                 680                 685

Asn Arg Asn Phe Met Gln Leu Ile His Asp Asp Ser Leu Thr Phe Lys
    690                 695                 700

Glu Asp Ile Gln Lys Ala Gln Val Ser Gly Gln Gly Asp Ser Leu His
705                 710                 715                 720

Glu His Ile Ala Asn Leu Ala Gly Ser Pro Ala Ile Lys Lys Gly Ile
                725                 730                 735

Leu Gln Thr Val Lys Val Val Asp Glu Leu Val Lys Val Met Gly Arg
            740                 745                 750

His Lys Pro Glu Asn Ile Val Ile Glu Met Ala Arg Glu Asn Gln Thr
        755                 760                 765

Thr Gln Lys Gly Gln Lys Asn Ser Arg Glu Arg Met Lys Arg Ile Glu
    770                 775                 780

Glu Gly Ile Lys Glu Leu Gly Ser Gln Ile Leu Lys Glu His Pro Val
785                 790                 795                 800

Glu Asn Thr Gln Leu Gln Asn Glu Lys Leu Tyr Leu Tyr Tyr Leu Gln
                805                 810                 815

Asn Gly Arg Asp Met Tyr Val Asp Gln Glu Leu Asp Ile Asn Arg Leu
            820                 825                 830

Ser Asp Tyr Asp Val Asp His Ile Val Pro Gln Ser Phe Leu Lys Asp
        835                 840                 845

Asp Ser Ile Asp Asn Lys Val Leu Thr Arg Ser Asp Lys Asn Arg Gly
    850                 855                 860

Lys Ser Asp Asn Val Pro Ser Glu Glu Val Val Lys Lys Met Lys Asn
865                 870                 875                 880

Tyr Trp Arg Gln Leu Leu Asn Ala Lys Leu Ile Thr Gln Arg Lys Phe
                885                 890                 895

Asp Asn Leu Thr Lys Ala Glu Arg Gly Gly Leu Ser Glu Leu Asp Lys
            900                 905                 910

Ala Gly Phe Ile Lys Arg Gln Leu Val Glu Thr Arg Gln Ile Thr Lys
        915                 920                 925

His Val Ala Gln Ile Leu Asp Ser Arg Met Asn Thr Lys Tyr Asp Glu
    930                 935                 940

Asn Asp Lys Leu Ile Arg Glu Val Lys Val Ile Thr Leu Lys Ser Lys
945                 950                 955                 960

Leu Val Ser Asp Phe Arg Lys Asp Phe Gln Phe Tyr Lys Val Arg Glu
                965                 970                 975

Ile Asn Asn Tyr His His Ala His Asp Ala Tyr Leu Asn Ala Val Val
            980                 985                 990

Gly Thr Ala Leu Ile Lys Lys Tyr  Pro Lys Leu Glu Ser  Glu Phe Val
        995                 1000                1005

Tyr Gly Asp Tyr Lys Val Tyr Asp Val Arg Lys Met Ile Ala Lys
    1010                1015                1020

Ser Glu Gln Glu Ile Gly Lys Ala Thr Ala Lys Tyr Phe Phe Tyr
    1025                1030                1035

Ser Asn Ile Met Asn Phe Phe Lys Thr Glu Ile Thr Leu Ala Asn
    1040                1045                1050

Gly Glu Ile Arg Lys Arg Pro Leu Ile Glu Thr Asn Gly Glu Thr
    1055                1060                1065

Gly Glu Ile Val Trp Asp Lys Gly Arg Asp Phe Ala Thr Val Arg
    1070                1075                1080

Lys Val Leu Ser Met Pro Gln Val Asn Ile Val Lys Lys Thr Glu
    1085                1090                1095

Val Gln Thr Gly Gly Phe Ser Lys Glu Ser Ile Leu Pro Lys Arg
    1100                1105                1110

Asn Ser Asp Lys Leu Ile Ala Arg Lys Lys Asp Trp Asp Pro Lys
    1115                1120                1125

Lys Tyr Gly Gly Phe Asp Ser Pro Thr Val Ala Tyr Ser Val Leu
    1130                1135                1140

Val Val Ala Lys Val Glu Lys Gly Lys Ser Lys Lys Leu Lys Ser
    1145                1150                1155

Val Lys Glu Leu Leu Gly Ile Thr Ile Met Glu Arg Ser Ser Phe
    1160                1165                1170

Glu Lys Asn Pro Ile Asp Phe Leu Glu Ala Lys Gly Tyr Lys Glu
    1175                1180                1185

Val Lys Lys Asp Leu Ile Ile Lys Leu Pro Lys Tyr Ser Leu Phe
    1190                1195                1200

Glu Leu Glu Asn Gly Arg Lys Arg Met Leu Ala Ser Ala Gly Glu
    1205                1210                1215

Leu Gln Lys Gly Asn Glu Leu Ala Leu Pro Ser Lys Tyr Val Asn
    1220                1225                1230

Phe Leu Tyr Leu Ala Ser His Tyr Glu Lys Leu Lys Gly Ser Pro
    1235                1240                1245

Glu Asp Asn Glu Gln Lys Gln Leu Phe Val Glu Gln His Lys His
    1250                1255                1260

Tyr Leu Asp Glu Ile Ile Glu Gln Ile Ser Glu Phe Ser Lys Arg
    1265                1270                1275

Val Ile Leu Ala Asp Ala Asn Leu Asp Lys Val Leu Ser Ala Tyr
    1280                1285                1290

Asn Lys His Arg Asp Lys Pro Ile Arg Glu Gln Ala Glu Asn Ile
    1295                1300                1305

Ile His Leu Phe Thr Leu Thr Asn Leu Gly Ala Pro Ala Ala Phe
    1310                1315                1320

Lys Tyr Phe Asp Thr Thr Ile Asp Arg Lys Arg Tyr Thr Ser Thr
    1325                1330                1335

Lys Glu Val Leu Asp Ala Thr Leu Ile His Gln Ser Ile Thr Gly
    1340                1345                1350

Leu Tyr Glu Thr Arg Ile Asp Leu Ser Gln Leu Gly Gly Asp
    1355                1360                1365

<210> 37
<211> 4101
<212> DNA
<213> Artificial

<220>
<223> Cas encoding

<400> 37
gacaagaagt acagcatcgg cctggacatc ggcaccaact ctgtgggctg ggccgtgatc 60

accgacgagt acaaggtgcc cagcaagaaa ttcaaggtgc tgggcaacac cgaccggcac 120

agcatcaaga agaacctgat cggagccctg ctgttcgaca gcggcgaaac agccgaggcc 180

acccggctga agagaaccgc cagaagaaga tacaccagac ggaagaaccg gatctgctat 240

ctgcaagaga tcttcagcaa cgagatggcc aaggtggacg acagcttctt ccacagactg 300

gaagagtcct tcctggtgga agaggataag aagcacgagc ggcaccccat cttcggcaac 360

atcgtggacg aggtggccta ccacgagaag taccccacca tctaccacct gagaaagaaa 420

ctggtggaca gcaccgacaa ggccgacctg cggctgatct atctggccct ggcccacatg 480

atcaagttcc ggggccactt cctgatcgag ggcgacctga accccgacaa cagcgacgtg 540

gacaagctgt tcatccagct ggtgcagacc tacaaccagc tgttcgagga aaaccccatc 600

aacgccagcg gcgtggacgc caaggccatc ctgtctgcca gactgagcaa gagcagacgg 660
ctggaaaatc tgatcgccca gctgcccggc gagaagaaga atggcctgtt cggaaacctg 720
attgccctga gcctgggcct gacccccaac ttcaagagca acttcgacct ggccgaggat 780
gccaaactgc agctgagcaa ggacacctac gacgacgacc tggacaacct gctggcccag 840
atcggcgacc agtacgccga cctgtttctg gccgccaaga acctgtccga cgccatcctg 900
ctgagcgaca tcctgagagt gaacaccgag atcaccaagg cccccctgag cgcctctatg 960
atcaagagat acgacgagca ccaccaggac ctgaccctgc tgaaagctct cgtgcggcag 1020
cagctgcctg agaagtacaa agagattttc ttcgaccaga gcaagaacgg ctacgccggc 1080
tacattgacg gcggagccag ccaggaagag ttctacaagt tcatcaagcc catcctggaa 1140
aagatggacg gcaccgagga actgctcgtg aagctgaaca gagaggacct gctgcggaag 1200
cagcggacct tcgacaacgg cagcatcccc caccagatcc acctgggaga gctgcacgcc 1260
attctgcggc ggcaggaaga tttttaccca ttcctgaagg acaaccggga aaagatcgag 1320
aagatcctga ccttccgcat cccctactac gtgggccctc tggccagggg aaacagcaga 1380
ttcgcctgga tgaccagaaa gagcgaggaa accatcaccc cctggaactt cgaggaagtg 1440
gtggacaagg gcgcttccgc ccagagcttc atcgagcgga tgaccaactt cgataagaac 1500
ctgcccaacg agaaggtgct gcccaagcac agcctgctgt acgagtactt caccgtgtat 1560
aacgagctga ccaaagtgaa atacgtgacc gagggaatga gaaagcccgc cttcctgagc 1620
ggcgagcaga aaaaggccat cgtggacctg ctgttcaaga ccaaccggaa agtgaccgtg 1680
aagcagctga aagaggacta cttcaagaaa atcgagtgct tcgactccgt ggaaatctcc 1740
ggcgtggaag atcggttcaa cgcctccctg ggcacatacc acgatctgct gaaaattatc 1800
aaggacaagg acttcctgga caatgaggaa aacgaggaca ttctggaaga tatcgtgctg 1860
accctgacac tgtttgagga cagagagatg atcgaggaac ggctgaaaac ctatgcccac 1920
ctgttcgacg acaaagtgat gaagcagctg aagcggcgga gatacaccgg ctggggcagg 1980
ctgagccgga agctgatcaa cggcatccgg gacaagcagt ccggcaagac aatcctggat 2040
ttcctgaagt ccgacggctt cgccaacaga aacttcatgc agctgatcca cgacgacagc 2100
ctgaccttta aagaggacat ccagaaagcc caggtgtccg gccagggcga tagcctgcac 2160
gagcacattg ccaatctggc cggcagcccc gccattaaga agggcatcct gcagacagtg 2220
aaggtggtgg acgagctcgt gaaagtgatg ggccggcaca agcccgagaa catcgtgatc 2280
gaaatggcca gagagaacca gaccacccag aagggacaga agaacagccg cgagagaatg 2340
aagcggatcg aagagggcat caaagagctg ggcagccaga tcctgaaaga acaccccgtg 2400
gaaaacaccc agctgcagaa cgagaagctg tacctgtact acctgcagaa tgggcgggat 2460
atgtacgtgg accaggaact ggacatcaac cggctgtccg actacgatgt ggaccatatc 2520

gtgcctcaga gctttctgaa ggacgactcc atcgacaaca aggtgctgac cagaagcgac 2580

aagaaccggg gcaagagcga caacgtgccc tccgaagagg tcgtgaagaa gatgaagaac 2640

tactggcggc agctgctgaa cgccaagctg attacccaga gaaagttcga caatctgacc 2700

aaggccgaga gaggcggcct gagcgaactg gataaggccg gcttcatcaa gagacagctg 2760

gtggaaaccc ggcagatcac aaagcacgtg gcacagatcc tggactcccg gatgaacact 2820

aagtacgacg agaatgacaa gctgatccgg gaagtgaaag tgatcaccct gaagtccaag 2880

ctggtgtccg atttccggaa ggatttccag ttttacaaag tgcgcgagat caacaactac 2940

caccacgccc acgacgccta cctgaacgcc gtcgtgggaa ccgccctgat caaaaagtac 3000

cctaagctgg aaagcgagtt cgtgtacggc gactacaagg tgtacgacgt gcggaagatg 3060

atcgccaaga gcgagcagga aatcggcaag gctaccgcca agtacttctt ctacagcaac 3120

atcatgaact ttttcaagac cgagattacc ctggccaacg gcgagatccg gaagcggcct 3180

ctgatcgaga caaacggcga aaccggggag atcgtgtggg ataagggccg ggattttgcc 3240

accgtgcgga aagtgctgag catgccccaa gtgaatatcg tgaaaaagac cgaggtgcag 3300

acaggcggct tcagcaaaga gtctatcctg cccaagagga acagcgataa gctgatcgcc 3360

agaaagaagg actgggaccc taagaagtac ggcggcttcg acagccccac cgtggcctat 3420

tctgtgctgg tggtggccaa agtggaaaag ggcaagtcca agaaactgaa gagtgtgaaa 3480

gagctgctgg ggatcaccat catggaaaga agcagcttcg agaagaatcc catcgacttt 3540

ctggaagcca agggctacaa agaagtgaaa aaggacctga tcatcaagct gcctaagtac 3600

tccctgttcg agctggaaaa cggccggaag agaatgctgg cctctgccgg cgaactgcag 3660

aagggaaacg aactggccct gccctccaaa tatgtgaact tcctgtacct ggccagccac 3720

tatgagaagc tgaagggctc ccccgaggat aatgagcaga aacagctgtt tgtggaacag 3780

cacaagcact acctggacga gatcatcgag cagatcagcg agttctccaa gagagtgatc 3840

ctggccgacg ctaatctgga caaagtgctg tccgcctaca acaagcaccg ggataagccc 3900

atcagagagc aggccgagaa tatcatccac ctgtttaccc tgaccaatct gggagcccct 3960

gccgccttca agtactttga caccaccatc gaccggaaga ggtacaccag caccaaagag 4020

gtgctggacg ccaccctgat ccaccagagc atcaccggcc tgtacgagac acggatcgac 4080

ctgtctcagc tgggaggcga c 4101

<210> 38
<211> 1425
<212> PRT
<213> Artificial

<220>
<223> 3xFLAG-NLS-SpCas9-NLS

<400>  38

Met Asp Tyr Lys Asp His Asp Gly Asp Tyr Lys Asp His Asp Ile Asp
1               5                   10                  15

Tyr Lys Asp Asp Asp Asp Lys Met Ala Pro Lys Lys Lys Arg Lys Val
            20                  25                  30

Gly Ile His Gly Val Pro Ala Ala Asp Lys Lys Tyr Ser Ile Gly Leu
        35                  40                  45

Asp Ile Gly Thr Asn Ser Val Gly Trp Ala Val Ile Thr Asp Glu Tyr
    50                  55                  60

Lys Val Pro Ser Lys Lys Phe Lys Val Leu Gly Asn Thr Asp Arg His
65                  70                  75                  80

Ser Ile Lys Lys Asn Leu Ile Gly Ala Leu Leu Phe Asp Ser Gly Glu
                85                  90                  95

Thr Ala Glu Ala Thr Arg Leu Lys Arg Thr Ala Arg Arg Arg Tyr Thr
            100                 105                 110

Arg Arg Lys Asn Arg Ile Cys Tyr Leu Gln Glu Ile Phe Ser Asn Glu
        115                 120                 125

Met Ala Lys Val Asp Asp Ser Phe Phe His Arg Leu Glu Glu Ser Phe
    130                 135                 140

Leu Val Glu Glu Asp Lys Lys His Glu Arg His Pro Ile Phe Gly Asn
145                 150                 155                 160

Ile Val Asp Glu Val Ala Tyr His Glu Lys Tyr Pro Thr Ile Tyr His
                165                 170                 175

Leu Arg Lys Lys Leu Val Asp Ser Thr Asp Lys Ala Asp Leu Arg Leu
            180                 185                 190

Ile Tyr Leu Ala Leu Ala His Met Ile Lys Phe Arg Gly His Phe Leu
        195                 200                 205

Ile Glu Gly Asp Leu Asn Pro Asp Asn Ser Asp Val Asp Lys Leu Phe
    210                 215                 220

Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
                245                 250                 255

Lys Ser Arg Arg Leu Glu Asn Leu Ile Ala Gln Leu Pro Gly Glu Lys
            260                 265                 270

Lys Asn Gly Leu Phe Gly Asn Leu Ile Ala Leu Ser Leu Gly Leu Thr
        275                 280                 285

Pro Asn Phe Lys Ser Asn Phe Asp Leu Ala Glu Asp Ala Lys Leu Gln
    290                 295                 300

Leu Ser Lys Asp Thr Tyr Asp Asp Asp Leu Asp Asn Leu Leu Ala Gln
305                 310                 315                 320

Ile Gly Asp Gln Tyr Ala Asp Leu Phe Leu Ala Ala Lys Asn Leu Ser
                325                 330                 335

Asp Ala Ile Leu Leu Ser Asp Ile Leu Arg Val Asn Thr Glu Ile Thr
            340                 345                 350

Lys Ala Pro Leu Ser Ala Ser Met Ile Lys Arg Tyr Asp Glu His His
        355                 360                 365

Gln Asp Leu Thr Leu Leu Lys Ala Leu Val Arg Gln Gln Leu Pro Glu
    370                 375                 380

Lys Tyr Lys Glu Ile Phe Phe Asp Gln Ser Lys Asn Gly Tyr Ala Gly
385                 390                 395                 400

Tyr Ile Asp Gly Gly Ala Ser Gln Glu Glu Phe Tyr Lys Phe Ile Lys
                405                 410                 415

Pro Ile Leu Glu Lys Met Asp Gly Thr Glu Glu Leu Leu Val Lys Leu
            420                 425                 430

Asn Arg Glu Asp Leu Leu Arg Lys Gln Arg Thr Phe Asp Asn Gly Ser
        435                 440                 445

Ile Pro His Gln Ile His Leu Gly Glu Leu His Ala Ile Leu Arg Arg
    450                 455                 460

Gln Glu Asp Phe Tyr Pro Phe Leu Lys Asp Asn Arg Glu Lys Ile Glu
465                 470                 475                 480

Lys Ile Leu Thr Phe Arg Ile Pro Tyr Tyr Val Gly Pro Leu Ala Arg
                485                 490                 495

Gly Asn Ser Arg Phe Ala Trp Met Thr Arg Lys Ser Glu Glu Thr Ile
        500                 505                 510

Thr Pro Trp Asn Phe Glu Glu Val Val Asp Lys Gly Ala Ser Ala Gln
    515                 520                 525

Ser Phe Ile Glu Arg Met Thr Asn Phe Asp Lys Asn Leu Pro Asn Glu
    530                 535                 540

Lys Val Leu Pro Lys His Ser Leu Leu Tyr Glu Tyr Phe Thr Val Tyr
545                 550                 555                 560

Asn Glu Leu Thr Lys Val Lys Tyr Val Thr Glu Gly Met Arg Lys Pro
                565                 570                 575

Ala Phe Leu Ser Gly Glu Gln Lys Lys Ala Ile Val Asp Leu Leu Phe
            580                 585                 590

Lys Thr Asn Arg Lys Val Thr Val Lys Gln Leu Lys Glu Asp Tyr Phe
        595                 600                 605

Lys Lys Ile Glu Cys Phe Asp Ser Val Glu Ile Ser Gly Val Glu Asp
    610                 615                 620

Arg Phe Asn Ala Ser Leu Gly Thr Tyr His Asp Leu Leu Lys Ile Ile
625                 630                 635                 640

Lys Asp Lys Asp Phe Leu Asp Asn Glu Glu Asn Glu Asp Ile Leu Glu
                645                 650                 655

Asp Ile Val Leu Thr Leu Thr Leu Phe Glu Asp Arg Glu Met Ile Glu
            660                 665                 670

Glu Arg Leu Lys Thr Tyr Ala His Leu Phe Asp Asp Lys Val Met Lys
        675                 680                 685

Gln Leu Lys Arg Arg Arg Tyr Thr Gly Trp Gly Arg Leu Ser Arg Lys
    690                 695                 700

Leu Ile Asn Gly Ile Arg Asp Lys Gln Ser Gly Lys Thr Ile Leu Asp
705                 710                 715                 720

Phe Leu Lys Ser Asp Gly Phe Ala Asn Arg Asn Phe Met Gln Leu Ile
                725                 730                 735

His Asp Asp Ser Leu Thr Phe Lys Glu Asp Ile Gln Lys Ala Gln Val
            740                 745                 750

Ser Gly Gln Gly Asp Ser Leu His Glu His Ile Ala Asn Leu Ala Gly
        755                 760                 765

Ser Pro Ala Ile Lys Lys Gly Ile Leu Gln Thr Val Lys Val Val Asp
    770                 775                 780

Glu Leu Val Lys Val Met Gly Arg His Lys Pro Glu Asn Ile Val Ile
785                 790                 795                 800

Glu Met Ala Arg Glu Asn Gln Thr Thr Gln Lys Gly Gln Lys Asn Ser
                805                 810                 815

Arg Glu Arg Met Lys Arg Ile Glu Glu Gly Ile Lys Glu Leu Gly Ser
            820                 825                 830

Gln Ile Leu Lys Glu His Pro Val Glu Asn Thr Gln Leu Gln Asn Glu
        835                 840                 845

Lys Leu Tyr Leu Tyr Tyr Leu Gln Asn Gly Arg Asp Met Tyr Val Asp
    850                 855                 860

Gln Glu Leu Asp Ile Asn Arg Leu Ser Asp Tyr Asp Val Asp His Ile
865                 870                 875                 880

Val Pro Gln Ser Phe Leu Lys Asp Asp Ser Ile Asp Asn Lys Val Leu
                885                 890                 895

Thr Arg Ser Asp Lys Asn Arg Gly Lys Ser Asp Asn Val Pro Ser Glu
            900                 905                 910

Glu Val Val Lys Lys Met Lys Asn Tyr Trp Arg Gln Leu Leu Asn Ala
        915                 920                 925

Lys Leu Ile Thr Gln Arg Lys Phe Asp Asn Leu Thr Lys Ala Glu Arg
    930                 935                 940

Gly Gly Leu Ser Glu Leu Asp Lys Ala Gly Phe Ile Lys Arg Gln Leu
945                 950                 955                 960

Val Glu Thr Arg Gln Ile Thr Lys His Val Ala Gln Ile Leu Asp Ser
                965                 970                 975

Arg Met Asn Thr Lys Tyr Asp Glu Asn Asp Lys Leu Ile Arg Glu Val
            980                 985                 990

Lys Val Ile Thr Leu Lys Ser Lys  Leu Val Ser Asp Phe  Arg Lys Asp

995                 1000                1005

Phe Gln Phe Tyr Lys Val Arg Glu Ile Asn Asn Tyr His His Ala
    1010                1015                1020

His Asp Ala Tyr Leu Asn Ala Val Val Gly Thr Ala Leu Ile Lys
    1025                1030                1035

Lys Tyr Pro Lys Leu Glu Ser Glu Phe Val Tyr Gly Asp Tyr Lys
    1040                1045                1050

Val Tyr Asp Val Arg Lys Met Ile Ala Lys Ser Glu Gln Glu Ile
    1055                1060                1065

Gly Lys Ala Thr Ala Lys Tyr Phe Phe Tyr Ser Asn Ile Met Asn
    1070                1075                1080

Phe Phe Lys Thr Glu Ile Thr Leu Ala Asn Gly Glu Ile Arg Lys
    1085                1090                1095

Arg Pro Leu Ile Glu Thr Asn Gly Glu Thr Gly Glu Ile Val Trp
    1100                1105                1110

Asp Lys Gly Arg Asp Phe Ala Thr Val Arg Lys Val Leu Ser Met
    1115                1120                1125

Pro Gln Val Asn Ile Val Lys Lys Thr Glu Val Gln Thr Gly Gly
    1130                1135                1140

Phe Ser Lys Glu Ser Ile Leu Pro Lys Arg Asn Ser Asp Lys Leu
    1145                1150                1155

Ile Ala Arg Lys Lys Asp Trp Asp Pro Lys Lys Tyr Gly Gly Phe
    1160                1165                1170

Asp Ser Pro Thr Val Ala Tyr Ser Val Leu Val Val Ala Lys Val
    1175                1180                1185

Glu Lys Gly Lys Ser Lys Lys Leu Lys Ser Val Lys Glu Leu Leu
    1190                1195                1200

Gly Ile Thr Ile Met Glu Arg Ser Ser Phe Glu Lys Asn Pro Ile
    1205                1210                1215

Asp Phe Leu Glu Ala Lys Gly Tyr Lys Glu Val Lys Lys Asp Leu
    1220                1225                1230

Ile Ile Lys Leu Pro Lys Tyr Ser Leu Phe Glu Leu Glu Asn Gly
    1235                1240                1245

Arg Lys Arg Met Leu Ala Ser Ala Gly Glu Leu Gln Lys Gly Asn
    1250                1255                1260

Glu Leu Ala Leu Pro Ser Lys Tyr Val Asn Phe Leu Tyr Leu Ala
    1265                1270                1275

Ser His Tyr Glu Lys Leu Lys Gly Ser Pro Glu Asp Asn Glu Gln
    1280                1285                1290

Lys Gln Leu Phe Val Glu Gln His Lys His Tyr Leu Asp Glu Ile
    1295                1300                1305

Ile Glu Gln Ile Ser Glu Phe Ser Lys Arg Val Ile Leu Ala Asp
    1310                1315                1320

Ala Asn Leu Asp Lys Val Leu Ser Ala Tyr Asn Lys His Arg Asp
    1325                1330                1335

Lys Pro Ile Arg Glu Gln Ala Glu Asn Ile Ile His Leu Phe Thr
    1340                1345                1350

Leu Thr Asn Leu Gly Ala Pro Ala Ala Phe Lys Tyr Phe Asp Thr
    1355                1360                1365

Thr Ile Asp Arg Lys Arg Tyr Thr Ser Thr Lys Glu Val Leu Asp
    1370                1375                1380

Ala Thr Leu Ile His Gln Ser Ile Thr Gly Leu Tyr Glu Thr Arg
    1385                1390                1395

Ile Asp Leu Ser Gln Leu Gly Gly Asp Lys Arg Pro Ala Ala Thr
    1400                1405                1410

Lys Lys Ala Gly Gln Ala Lys Lys Lys Lys Glu Phe
    1415                1420                1425

<210> 39
<211> 4275
<212> DNA
<213> Artificial

<220>
<223> 3xFLAG-NLS-SpCas9-NLS encoding

<400> 39
atggactata aggaccacga cggagactac aaggatcatg atattgatta caaagacgat 60

gacgataaga tggccccaaa gaagaagcgg aaggtcggta tccacggagt cccagcagcc 120

gacaagaagt acagcatcgg cctggacatc ggcaccaact ctgtgggctg ggccgtgatc 180

accgacgagt acaaggtgcc cagcaagaaa ttcaaggtgc tgggcaacac cgaccggcac 240

agcatcaaga agaacctgat cggagccctg ctgttcgaca gcggcgaaac agccgaggcc 300

acccggctga agagaaccgc cagaagaaga tacaccagac ggaagaaccg gatctgctat 360

ctgcaagaga tcttcagcaa cgagatggcc aaggtggacg acagcttctt ccacagactg 420

gaagagtcct tcctggtgga agaggataag aagcacgagc ggcaccccat cttcggcaac 480

atcgtggacg aggtggccta ccacgagaag taccccacca tctaccacct gagaaagaaa 540

ctggtggaca gcaccgacaa ggccgacctg cggctgatct atctggccct ggcccacatg 600

atcaagttcc ggggccactt cctgatcgag ggcgacctga accccgacaa cagcgacgtg 660

gacaagctgt tcatccagct ggtgcagacc tacaaccagc tgttcgagga aaaccccatc 720

aacgccagcg gcgtggacgc caaggccatc ctgtctgcca gactgagcaa gagcagacgg 780

ctggaaaatc tgatcgccca gctgcccggc gagaagaaga atggcctgtt cggaaacctg 840

attgccctga gcctgggcct gacccccaac ttcaagagca acttcgacct ggccgaggat 900

gccaaactgc agctgagcaa ggacacctac gacgacgacc tggacaacct gctggcccag 960

atcggcgacc agtacgccga cctgtttctg gccgccaaga acctgtccga cgccatcctg 1020

ctgagcgaca tcctgagagt gaacaccgag atcaccaagg cccccctgag cgcctctatg 1080

atcaagagat acgacgagca ccaccaggac ctgaccctgc tgaaagctct cgtgcggcag 1140

cagctgcctg agaagtacaa agagattttc ttcgaccaga gcaagaacgg ctacgccggc 1200

tacattgacg gcggagccag ccaggaagag ttctacaagt tcatcaagcc catcctggaa 1260

aagatggacg gcaccgagga actgctcgtg aagctgaaca gagaggacct gctgcggaag 1320

cagcggacct tcgacaacgg cagcatcccc caccagatcc acctgggaga gctgcacgcc 1380

attctgcggc ggcaggaaga tttttaccca ttcctgaagg acaaccggga aaagatcgag 1440

aagatcctga ccttccgcat cccctactac gtgggccctc tggccagggg aaacagcaga 1500

ttcgcctgga tgaccagaaa gagcgaggaa accatcaccc cctggaactt cgaggaagtg 1560

gtggacaagg gcgcttccgc ccagagcttc atcgagcgga tgaccaactt cgataagaac 1620

ctgcccaacg agaaggtgct gcccaagcac agcctgctgt acgagtactt caccgtgtat 1680

aacgagctga ccaaagtgaa atacgtgacc gagggaatga gaaagcccgc cttcctgagc 1740

ggcgagcaga aaaaggccat cgtggacctg ctgttcaaga ccaaccggaa agtgaccgtg 1800

aagcagctga aagaggacta cttcaagaaa atcgagtgct tcgactccgt ggaaatctcc 1860

ggcgtggaag atcggttcaa cgcctccctg ggcacatacc acgatctgct gaaaattatc 1920

aaggacaagg acttcctgga caatgaggaa aacgaggaca ttctggaaga tatcgtgctg 1980

accctgacac tgtttgagga cagagagatg atcgaggaac ggctgaaaac ctatgcccac 2040
ctgttcgacg acaaagtgat gaagcagctg aagcggcgga gatacaccgg ctggggcagg 2100
ctgagccgga agctgatcaa cggcatccgg gacaagcagt ccggcaagac aatcctggat 2160
ttcctgaagt ccgacggctt cgccaacaga aacttcatgc agctgatcca cgacgacagc 2220
ctgaccttta aagaggacat ccagaaagcc caggtgtccg gccagggcga tagcctgcac 2280
gagcacattg ccaatctggc cggcagcccc gccattaaga agggcatcct gcagacagtg 2340
aaggtggtgg acgagctcgt gaaagtgatg ggccggcaca agcccgagaa catcgtgatc 2400
gaaatggcca gagagaacca gaccacccag aagggacaga agaacagccg cgagagaatg 2460
aagcggatcg aagagggcat caaagagctg ggcagccaga tcctgaaaga acaccccgtg 2520
gaaaacaccc agctgcagaa cgagaagctg tacctgtact acctgcagaa tgggcgggat 2580
atgtacgtgg accaggaact ggacatcaac cggctgtccg actacgatgt ggaccatatc 2640
gtgcctcaga gctttctgaa ggacgactcc atcgacaaca aggtgctgac cagaagcgac 2700
aagaaccggg gcaagagcga caacgtgccc tccgaagagg tcgtgaagaa gatgaagaac 2760
tactggcggc agctgctgaa cgccaagctg attacccaga gaaagttcga caatctgacc 2820
aaggccgaga gaggcggcct gagcgaactg gataaggccg gcttcatcaa gagacagctg 2880
gtggaaaccc ggcagatcac aaagcacgtg gcacagatcc tggactcccg gatgaacact 2940
aagtacgacg agaatgacaa gctgatccgg gaagtgaaag tgatcaccct gaagtccaag 3000
ctggtgtccg atttccggaa ggatttccag ttttacaaag tgcgcgagat caacaactac 3060
caccacgccc acgacgccta cctgaacgcc gtcgtgggaa ccgccctgat caaaaagtac 3120
cctaagctgg aaagcgagtt cgtgtacggc gactacaagg tgtacgacgt gcggaagatg 3180
atcgccaaga gcgagcagga aatcggcaag gctaccgcca agtacttctt ctacagcaac 3240
atcatgaact ttttcaagac cgagattacc ctggccaacg gcgagatccg gaagcggcct 3300
ctgatcgaga caaacggcga aaccggggag atcgtgtggg ataagggccg ggattttgcc 3360
accgtgcgga aagtgctgag catgccccaa gtgaatatcg tgaaaaagac cgaggtgcag 3420
acaggcggct tcagcaaaga gtctatcctg cccaagagga acagcgataa gctgatcgcc 3480
agaaagaagg actgggaccc taagaagtac ggcggcttcg acagccccac cgtggcctat 3540
tctgtgctgg tggtggccaa agtggaaaag ggcaagtcca agaaactgaa gagtgtgaaa 3600
gagctgctgg ggatcaccat catggaaaga agcagcttcg agaagaatcc catcgacttt 3660
ctggaagcca agggctacaa agaagtgaaa aaggacctga tcatcaagct gcctaagtac 3720
tccctgttcg agctggaaaa cggccggaag agaatgctgg cctctgccgg cgaactgcag 3780
aagggaaacg aactggccct gccctccaaa tatgtgaact tcctgtacct ggccagccac 3840

tatgagaagc tgaagggctc ccccgaggat aatgagcaga aacagctgtt tgtggaacag    3900

cacaagcact acctggacga gatcatcgag cagatcagcg agttctccaa gagagtgatc    3960

ctggccgacg ctaatctgga caaagtgctg tccgcctaca acaagcaccg ggataagccc    4020

atcagagagc aggccgagaa tatcatccac ctgtttaccc tgaccaatct gggagcccct    4080

gccgccttca agtactttga caccaccatc gaccggaaga ggtacaccag caccaaagag    4140

gtgctggacg ccaccctgat ccaccagagc atcaccggcc tgtacgagac acggatcgac    4200

ctgtctcagc tgggaggcga caaaaggccg gcggccacga aaaaggccgg ccaggcaaaa    4260

aagaaaaagg aattc                                                     4275

<210>  40
<211>  22
<212>  DNA
<213>  Artificial

<220>
<223>  target sequence for miR122 (human)

<400>  40
caaacaccat tgtcacactc ca                                             22

<210>  41
<211>  22
<212>  DNA
<213>  Artificial

<220>
<223>  miR1 target sequence

<400>  41
atacatactt ctttacattc ca                                             22

<210>  42
<211>  21
<212>  DNA
<213>  Artificial

<220>
<223>  miR142-5p target sequence

<400>  42
agtagtgctt tctactttat g                                              21

<210>  43
<211>  23
<212>  DNA
<213>  Artificial

<220>
<223>  miR142-3p target sequence

<400>  43
tccataaagt aggaaacact aca                                            23

<210> 44
<211> 71
<212> RNA
<213> Homo sapiens

<400> 44
ugggaaacau acuucuuuau augcccauau ggaccugcua agcuauggaa uguaaagaag 60

uauguaucuc a 71


<210> 45
<211> 86
<212> RNA
<213> Homo sapiens

<400> 45
ugcuucccga ggccacaugc uucuuuauau ccccauaugg auuacuuugc uauggaaugu 60

aaggaagugu gugguuucgg caagug 86


<210> 46
<211> 71
<212> RNA
<213> Homo sapiens

<400> 46
ugacgggcga gcuuuuggcc cggguuauac cugaugcuca cguauaagac gagcaaaaag 60

cuuguugguc a 71


<210> 47
<211> 85
<212> RNA
<213> Homo sapiens

<400> 47
ccuuagcaga gcuguggagu gugacaaugg uguuuguguc uaaacuauca aacgccauua 60

ucacacuaaa uagcuacugc uaggc 85


<210> 48
<211> 87
<212> RNA
<213> Homo sapiens

<400> 48
gacagugcag ucacccauaa aguagaaagc acuacuaaca gcacuggagg guguaguguu 60

uccuacuuua uggaugagug uacugug 87